(19) **Europäisches Patentamt European Patent Office Office européen des brevets**

(11) **EP 4 472 498 B1**

(12) **EUROPEAN PATENT SPECIFICATION**

(45) Date of publication and mention
of the grant of the patent:
**01.04.2026 Bulletin 2026/14**

(21) Application number: **23703019.2**

(22) Date of filing: **01.02.2023**

(51) International Patent Classification (IPC):
***A61B 5/021*** (2006.01)   ***A61B 5/022*** (2006.01)
***A61B 5/08*** (2006.01)   ***A61B 5/024*** (2006.01)
***A61B 5/00*** (2006.01)

(52) Cooperative Patent Classification (CPC):
**A61B 5/02116; A61B 5/02241; A61B 5/02422;
A61B 5/4818; A61B 5/6826**

(86) International application number:
**PCT/EP2023/052380**

(87) International publication number:
**WO 2023/148190 (10.08.2023 Gazette 2023/32)**

(54) **SYSTEMS FOR SCREENING, DIAGNOSIS, DETECTION, MONITORING AND/OR THERAPY**

SYSTEME FÜR SCREENING, DIAGNOSE, NACHWEIS, ÜBERWACHUNG UND/ODER THERAPIE

SYSTÈMES DE CRIBLAGE, DE DIAGNOSTIC, DE DÉTECTION, DE SURVEILLANCE ET/OU DE
THÉRAPIE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **03.02.2022 US 202263306413 P**

(43) Date of publication of application:
**11.12.2024 Bulletin 2024/50**

(73) Proprietor: **Ectosense NV
3000 Leuven (BE)**

(72) Inventors:
• **MASSIE, Frederik Roger Anne
3012 Wilsele (BE)**
• **KHACHATRYAN, Ani
Yerevan, 0035 (AM)**
• **VITS, Steven Elsa Louis
1060 Sint-Gillis (BE)**

(74) Representative: **Ipsilon Belgium
Bellevue 5/501
9050 Gent-Ledeberg (BE)**

(56) References cited:
**WO-A1-2021/260192     US-A1- 2020 015 737
US-A1- 2021 275 090**

• **PILLAR GIORA ET AL: "Detecting central sleep
apnea in adult patients using WatchPAT-a
multicenter validation study", SLEEP AND
BREATHING - SCHLAF UND ATMUNG,
DRUCKBILD, TITISEE-NEUSTADT, DE, vol. 24,
no. 1, 11 August 2019 (2019-08-11), pages 387 -
398, XP037082176, ISSN: 1520-9512, [retrieved
on 20190811], DOI: 10.1007/S11325-019-01904-5**
• **IOACHIMESCU OCTAVIAN C. ET AL:
"Performance of peripheral arterial tonometry-
based testing for the diagnosis of obstructive
sleep apnea in a large sleep clinic cohort",
JOURNAL OF CLINICAL SLEEP MEDICINE, vol.
16, no. 10, 15 October 2020 (2020-10-15), US,
pages 1663 - 1674, XP093033623, ISSN:
1550-9389, DOI: 10.5664/jcsm.8620**

**Description**

1 CROSS REFERENCE TO RELATED APPLICATIONS

This application claims the benefit of United States Provisional Application No. 63/306,413, filed 3 February 2022.

2 BACKGROUND OF THE TECHNOLOGY

2.1 FIELD OF THE TECHNOLOGY

The present technology relates to one or more of the screening, detection, diagnosis, monitoring, treatment, prevention and amelioration of respiratory-related disorders. The present technology also relates to medical devices or apparatus, and their use. In some implementations, technology involves the screening, detection diagnosis, monitoring, treatment, prevention and/or amelioration of sleep disordered breathing, such as central or obstructive apnea.

2.2 DESCRIPTION OF THE RELATED ART

Sleep Apnea (SA) is a common sleep disorder affecting millions of people worldwide [1], [2]. An apnea event is defined as a cessation of airflow during sleep lasting 10 seconds or more, whereas a hypopnea event is characterized by an airflow reduction rather than a full cessation [3], [4]. A patient's SA severity can be expressed by their apnea-hypopnea index (AHI), which is simply the number of apnea and hypopnea events per hour of sleep [5], [6], [7].

SA can be obstructive (OSA) or central (CSA) in nature. OSA is the more common form of SA, and some of its symptoms and consequences are fatigue, daytime sleepiness, cardiac arrhythmia, and systemic hypertension [3]. During OSA, breathing effort continues but the upper airway is mechanically obstructed, resulting in interruptions of airflow. CSA, on the other hand, is characterized by a lack of neural drive to breathe during sleep [8], [9]. Both types of respiratory events can coexist in one patient. A respiratory event can also be of mixed origin, characterized by a lack of respiratory drive followed by obstructive breathing. Typically, CSA is the primary diagnosis when at least 50% of respiratory events are scored as central in origin. CSA can be characterized by the cAHI (central AHI), which is calculated as the number of central respiratory events per hour of sleep.

While the symptoms of CSA are often similar to those of OSA, the choice of therapy depends on the type of SA. Treatment of OSA includes life style measures (f.i. weight loss), mandibular advancement devices, surgical procedures and, most commonly, continuous positive airway pressure (CPAP) [10], [11]. Comparably, a wide variety of therapies for CSA exist of which some are distinctly different from therapies for OSA. These methods include drug intervention, oxygen, nocturnal mechanical ventilation (via nasal mask or tracheostomy and tracheal tube), and diaphragm pacing [3], [13]. As such, for clinicians to make optimal therapy decisions, it is important to differentiate between CSA and OSA in SA patients.

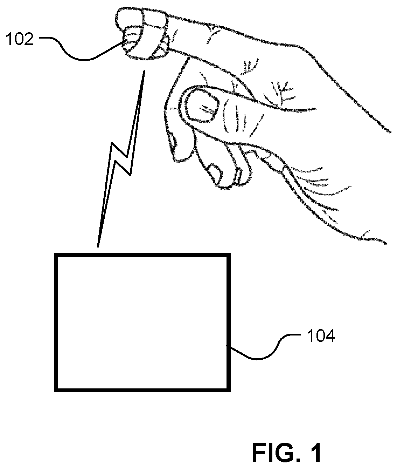

The gold standard for diagnosing SA is polysomnography (PSG) [14], [15]. PSG utilizes multiple sensing channels including electroencephalography, electro-oculography, electromyography, electrocardiography, and pulse oximetry, as well as airflow and respiratory effort to assess underlying causes of sleep disturbances [16]. However, due to the inconvenience of performing an in-lab PSG and in part due to the global COVID pandemic, Home Sleep Apnea Testing (HSAT) based on peripheral arterial tonometry (PAT) have rapidly gained popularity and currently comprise the most widely deployed category of HSAT [17]. PAT HSAT allows for minimally invasive multi-night testing and is available in a fully disposable format [18], [19]. An example of such a system, is called NightOwl PAT HSAT, which was described by Massie et al [20]. It includes a finger probe of the size of a fingertip that works with analysis software that is cloud-based. (Fig. 1). The analysis determines respiratory events from a peripheral arterial tone signal determined from a photoplethysmography signal generated by the sensor.

Compared to PSG, PAT HSATs have a major drawback, namely a reduced channel design (i.e., fewer sensors and signals). Indeed, a PAT-based HSAT obtains most of its modalities from finger photoplethysmography (PPG), from which it derives the blood oxygen saturation ($SpO_2$), pulse rate (PR), and PAT. Inherently, this means that PAT HSATs do not have access to information sources such as airflow reduction and cortical arousals and must instead rely on alternative sources of information to infer the presence of respiratory events [18].

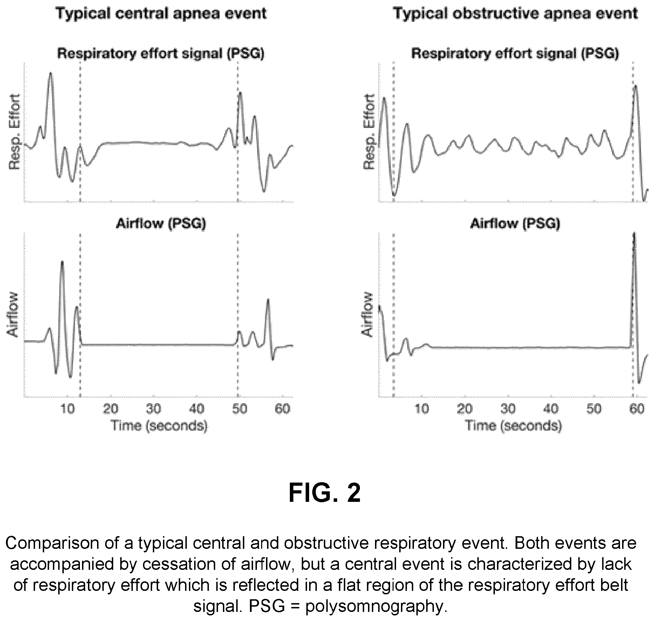

One more challenge for PAT HSATs is the differentiation between the types of respiratory events. Both types of events are characterized by airflow reduction. However, central respiratory events are accompanied by a cessation of respiratory effort as traditionally inferred from the abdominal and thoracic respiratory effort belts of the PSG (which monitor respiratory-related fluctuations in the abdominal and thoracic circumference) [5]. Fig. 2 shows a comparison of the respiratory effort during a central and obstructive respiratory event.

A PAT HSATs device, utilizing only a finger PPG probe for respiratory event detection, does not have airflow and respiratory effort channels (signals) that can be utilized for respiratory event type labeling.

It would be desirable to have an automated method to detect CSA in SA patients using only the PPG data acquired at the finger by a PAT HSAT. A validated PPG-based CSA detection method could be used to flag CSA in patients which enables referral to an in-lab PSG for further confirmation.

I. Other works

Recently, Pillar et al. published a validation study of their novel CSA detection method using the WatchPAT™ PAT HSAT[21] with extra sensors placed on the chest. In addition to finger PPG, the system comprises an optional snoring and body position (SBP) sensor positioned under the sternal notch. The SBP sensor is used to derive upper chest wall respiratory movements that are diminished during central respiratory events. According to Pillar et al. [21], the PPG's systolic upstroke shows more variability during obstructive respiratory events compared to central ones. The method uses both the upstroke variability together with the respiratory effort data acquired by the SBP sensor to identify central events.

Lazazzera et al. also published a method to detect and classify respiratory events into apnea and hypopnea and their origin (central or obstructive) using PPG and $SpO_2$ signals [22]. Their method uses PPG features, $SpO_2$ features, pulse rate time domain features, and pulse rate frequency domain features to classify respiratory events. However, they do not report on any patient-wise performance analysis, nor do they report on the cAHI estimation accuracy.

### 2.2.1 Screening / Diagnosis / Monitoring Systems

Screening and diagnosis generally describe the identification of a disorder from its signs and symptoms. Screening typically gives a true / false result such as indicating whether or not a patient's disorder is severe enough to warrant further investigation, while diagnosis may result in clinically actionable information. Screening and diagnosis tend to be one-off processes, whereas monitoring the progress of a disorder can continue indefinitely. Some screening / diagnosis systems are suitable only for screening / diagnosis, whereas some may also be used for monitoring.

As previously noted, Polysomnography (PSG) is a conventional system for diagnosis / monitoring of cardio-pulmonary disorders, and typically involves expert clinical staff to apply the system. PSG typically involves the placement of 15 to 20 contact sensors on a person in order to record various biosignals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. Clinical experts may be able to diagnose or monitor patients adequately based on visual observation of PSG signals. However, there are circumstances where a clinical expert may not be available, or a clinical expert may not be affordable. PSG is therefore expensive and inconvenient. In particular it is unsuitable for in-home diagnosis / monitoring.

Another convenient screening / diagnosis / monitoring system for home use comprises a nasal cannula, a pressure sensor, a processing device, and recording means. A nasal cannula is a device comprising two hollow open-ended projections that are configured to be inserted non-invasively a little way into a patient's nares so as to interfere as little as possible with the patient's respiration. The hollow projections are in fluid communication with a pressure transducer via a Y-shaped tube. The pressure transducer provides a data signal representative of the pressure at the entrance to the patient's nares (the nasal pressure). It has been shown that a nasal pressure signal is a satisfactory proxy for the nasal flow rate signal generated by a flow rate transducer in-line with a sealed nasal mask, in that the nasal pressure signal is comparable in shape to the nasal flow rate signal. The processing device may be configured to analyse the nasal pressure signal from the pressure transducer in real time or near real time to detect and classify SDB events in order to monitor the patient's condition. Screening or diagnosis may require similar analysis but not necessarily in real time or near real time. The recording means is therefore configured to record the nasal pressure signal from the pressure transducer for later off-line or "batch" analysis by the processing device for screening / diagnosis purposes.

### 2.2.2 Therapies

A range of therapies have been used to treat or ameliorate respiratory conditions. Furthermore, otherwise healthy individuals may take advantage of such therapies to prevent respiratory disorders from arising.

Nasal Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion by pushing the soft palate and tongue forward and away from the posterior oropharyngeal wall.

High flow therapy (HFT) does not rely on positive airway pressure within a sealed patient interface, but rather on delivery of air at a therapeutic flow rate to the vicinity of the entrance(s) to the patient's airway via an unsealed patient interface that may be significantly open to atmosphere. High flow therapy has been used to treat OSA, CSR, and COPD. The air being delivered to the airway at a high flow rate, relative to typical respiratory flow rates, flushes out the patient's anatomical deadspace and decreases the amount of rebreathed $CO_2$, thereby increasing the efficiency of gas exchange.

High flow therapy may be used in conjunction with respiratory pressure therapy.

Non-invasive ventilation (NIV) therapy provides ventilatory support to a patient through the upper airways to assist the patient in taking a full breath and/or maintaining adequate oxygen levels in the body by doing some or all of the work of breathing. NIV is provided via a non-invasive patient interface. NIV has been used to treat CSR, OHS, COPD, NMD, and Chest Wall disorders.

### 2.2.3 Diagnosis and Treatment Systems

These therapies may be provided by a treatment system or device. Such systems and devices may also be used to diagnose a condition without treating it.

A treatment system may comprise a Respiratory Therapy Device (RT device) such as Respiratory Pressure Therapy Device (RPT device) or a Respiratory Flow Therapy Device such as a high flow therapy device (HFT device), an air circuit, a humidifier, and a patient interface.

### 2.2.3.1 Patient Interface

A patient interface may be used to interface respiratory equipment to its user, for example by providing a flow of breathable gas. The flow of breathable gas may be provided via a mask to the nose and/or mouth, a tube to the mouth or a tracheostomy tube to the trachea of the user. Depending upon the therapy to be applied, the patient interface may form a seal, e.g., with a face region of the patient, to facilitate the delivery of gas at a pressure at sufficient variance with ambient pressure to effect therapy, e.g., a positive pressure of about 10 cmH$_2$O. For other forms of therapy, such as the delivery of oxygen, the patient interface may not include a seal sufficient to facilitate delivery to the airways of a supply of air at a positive pressure of about 10 cmH$_2$O.

### 2.2.3.2 Respiratory Therapy (RT) devices

Air pressure generators are known in a range of applications, e.g., industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, including one or more of comfort, noise, ease of use, efficacy, size, weight, manufacturability, cost, and reliability.

One known RPT device used for treating sleep disordered breathing is the S9 Sleep Therapy System, manufactured by ResMed, which proves CPAP therapy. Another example of an RPT device is a ventilator. Ventilators such as the ResMed Stellar™ Series of Adult and Paediatric Ventilators may provide invasive and non-invasive non-dependent ventilation therapy for a range of patients for treating a number of conditions such as but not limited to NMD, OHS and COPD.

RPT devices typically comprise a pressure generator, such as a motor-driven blower or a compressed gas reservoir, and are configured to supply pressurised air to the airways of a patient. The outlet of the RPT device is connected via an air circuit to a patient interface such as those described above.

There may still be a need for a system that can not only monitor the health of the user, but can also help those users understand how to improve their own quality of life, such as by understanding their symptoms and triggers, and how they respond to medication.

Additionally, it is desirable for the system to have access to, and be capable of processing, as much data relevant to the user's condition as possible. In this respect, it is also desirable that the system be capable of monitoring the patient for as much of the day and night as possible, not just when the patient is in bed.

U.S. 2021/275090 A1 relates to a system for detecting sleep apnea that uses a PPG sensor for generating a PPG signal. An estimate of respiratory effort is derived from the PPG signal, and from this a respiratory effort signal is derived. Characteristic features are extracted from the respiratory effort signal, and sleep disordered breathing events are detected from the extracted characteristic features. The analysis is for distinguishing between sleep disordered breathing events and normal breathing during sleep.

U.S. 2020/015737 A1 relates to a system configured to diagnose sleep with an apparatus configured to be attached to a patient. The apparatus includes: i) an optical sensor configured to measure a blood volume pulse of the patient; and ii) a wireless communication interface configured to wirelessly transmit the measured blood volume pulse. The system further includes means for performing: i) obtaining the wirelessly transmitted measured blood volume pulse; and ii) deriving from the blood volume pulse the peripheral arterial tone; iii) determining occurrences of sleep events from changes in the peripheral arterial tone.

WO2021/260192 A1 relates to a computer-implemented method for assessing peripheral arterial tone, PAT, of an individual monitored by optical plethysmography and for detecting therefrom occurrence of a sleep-related event. The method includes - obtaining: an optical plethysmography signal measured at an investigated volume; and light intensities acquired at two or more points in time along said optical plethysmography signal; determining changes in arterial blood volume in said investigated volume between said two or more points in time by determining a logarithm or a function approximation thereof of a function of said light intensities, thereby assessing PAT of said individual; and determining a drop in said PAT indicative for a vasoconstriction of arteries and arterioles in said investigated volume, thereby detecting occurrence of a sleep-related event.

3 BRIEF SUMMARY OF THE TECHNOLOGY

The invention is set out in the appended the claims.

The present technology is directed towards providing medical devices used in the screening, monitoring, diagnosis, amelioration, treatment, or prevention of respiratory disorders having one or more of improved comfort, cost, efficacy, ease of use and manufacturability.

The technology may include a system, apparatus and/or method that detects CSA in SA patients using, such as only, the PPG data acquired at the finger by a PPG sensor such as a PAT HSAT. For example, the technology may be implemented to discriminate/differentiate/distinguish CSA from OSA events. In this regard, the technology may detect such events by discrimination. That is, such discrimination may differentiate one type of event from another type of event with similar characteristics (e.g., different types of apnea events) so as to distinguish between them.

In some implementations, the method of the system/apparatus may involve extraction and analysis of respiratory-effort related information from the finger PPG data, as such by deriving a signal that resembles PSG-based respiratory effort as closely as possible. This extracted breathing signal may then be used to extract features to for a machine process or machine learning process or classifier, such as an ensemble of trees classifier, that is designed/trained to differentiate between the two types of respiratory events that may otherwise have similar characteristics. Such differentiation may be part of apparatus of a screening, monitoring and/or therapy system.

Some implementations of the present technology may include a processor-readable medium, having stored thereon processor-executable instructions which, when executed by one or more processors, cause the one or more processors to distinguish sleep disordered breathing events from a signal generated by a photoplethysmography (PPG) sensor. The processor-executable instructions may include instructions to receive data representing the signal from the PPG sensor. The processor-executable instructions may include instructions to derive a PPG-based respiratory signal from the data representing the signal from the PPG sensor. The processor-executable instructions may include instructions to compute first level features from the PPG-based respiratory signal. The processor-executable instructions may include instructions to compute second level features from the first level features. The processor-executable instructions may include instructions to evaluate, such as in a classifier, the first level features and the second level features. The processor-executable instructions may include instructions to generate, based on the evaluation, an output indication that identifies a sleep disordered breathing type.

In some implementations, the output indication that identifies the sleep disordered breathing type may characterize a plurality of events of a sleep session. The processor-executable instructions may further include instructions to derive a peripheral arterial tonometry (PAT) signal from the data representing the signal from the PPG sensor. The processor-executable instructions may further include instructions to detect one or more respiratory events based on the PAT signal. The output indication that identifies a sleep disordered breathing type may characterize the one or more respiratory events. The PPG sensor may be a finger sensor. The output indication may identify a central sleep apnea type. The central sleep apnea type may include a mixed central and obstructive sleep apnea type. The output indication may identify an obstructive sleep apnea type.

In some implementations, the instructions to compute the first level features may include instructions to compute values from a plurality of windows of the PPG-based respiratory signal. The computed values may include, for each of the plurality of windows, any one or more of a range, an interquartile range, a variance, and a number of local maxima. The plurality of windows may include windows of a first uniform size moving along the PPG-based respiratory signal. The plurality of windows may further include windows of a second uniform size moving along the PPG-based respiratory signal, the second uniform size being shorter than the first uniform size. The instructions to compute the second level features from the first level features may include instructions to select a subset of values for each of the first level features wherein the subset of values may be associated with a subset of windows, of the plurality of windows, that correspond with occurrence of the detected respiratory event. The instructions to compute the second level features from the first level features may include instructions to compute, from the selected subset of values, one or more values of the second level features. The one or more computed values of the second level features may include any one or more of: a minimum value, an average of lowest three values, and one or more percentile values.

In some implementations, the instructions to derive a PPG-based respiratory signal from the data representing the signal from the PPG sensor may include instructions to filter baseline modulations of the data representing the signal from the PPG sensor to generate a filtered signal. The instructions to derive a PPG-based respiratory signal from the data representing the signal from the PPG sensor may include instructions to generate an envelope signal from peaks of the filtered signal. The instructions to derive a PPG-based respiratory signal from the data representing the signal from the PPG sensor further may include instructions to normalize the envelope signal by subtracting a moving average window of the envelope signal and dividing the envelope signal by a moving interquartile range window of the envelope signal. In some implementations, the evaluation may be in a classifier or the evaluation may be in a classifier that may include an ensemble of trees classifier.

In some implementations, the processor-executable instructions may further include instructions to detect an event of vasoconstriction from the data representing the signal from the PPG sensor. The output indication may be based on the detected event of vasoconstriction. The instructions to detect an event of vasoconstriction may include instructions to filter the PPG signal data representing the signal from the PPG sensor to produce a filtered PPG signal. The instructions to detect an event of vasoconstriction may include instructions to process the filtered PPG signal to detect a trough attributable to a vasoconstriction. The processor-executable instructions further may include instructions to omit, from the evaluation (e.g., of the classifier), one or more values of one or both of the first level features and second level features that are associated with the detected event of vasoconstriction.

In some implementations, the processor-executable instructions may further include instructions to detect an event of arrythmia from the data representing the signal from the PPG sensor. The output indication may be based on the detected event of arrythmia. The instructions to detect an event of arrythmia may include instructions to detect one or more irregular peak-to-peak interval zones in the PPG signal that are associated with arrythmia. The processor-executable instructions may further include instructions omit, from the evaluation (e.g., of the classifier), data corresponding to at least one respiratory event, of the one or more respiratory events, that may be associated with the detected one or more irregular peak-to-peak interval zones. The instructions to detect the one or more irregular peak-to-peak interval zones may include instructions to compute an absolute value of a difference of two neighboring peak-to-peak intervals. The instructions to detect the one or more irregular peak-to-peak interval zones may include instructions to compute an average value of absolute values of differences of neighboring peak-to-peak intervals of the PPG signal. The instructions to detect the one or more irregular peak-to-peak interval zones may include instructions to compare the absolute value and the average value. The processor-executable instructions further may include instructions to generate a signal for controlling operation of a respiratory therapy apparatus based on the output indication. The controlling operation may include controlling a pressure or flow therapy of a blower of the respiratory therapy apparatus. The instructions to receive the data representing the signal from the PPG sensor may include instructions for receiving the data at a server.

Some implementations of the present technology may include server with access to the processor-readable medium as described therein, wherein the server may be configured to receive requests for downloading the processor-executable instructions of the processor-readable medium to a processing device over a network.

Some implementations of the present technology may include processing device that may include one or more processors and a processor-readable medium as described herein, or the processing device may be configured to access the processor-executable instructions with a server described herein. The processing device may be a respiratory therapy apparatus. The processing device may be configured to generate a pressure therapy or a flow therapy.

Some implementations of the present technology may include method of a server having access to the processor-readable medium as described herein, the method may include receiving, at the server, a request for downloading the processor-executable instructions of the processor-readable medium to an electronic processing device over a network. The method may include transmitting the processor-executable instructions to the electronic processing device in response to the request.

Some implementations of the present technology may include method of one or more processors for distinguishing sleep disordered breathing events from a signal generated by a photoplethysmography (PPG) sensor. The method may include accessing, with the one or more processors, any processor-readable medium described herein. The method may include executing, in the one or more processors, the processor-executable instructions of the processor-readable medium.

Some implementations of the present technology may include method of one or more processors for distinguishing sleep disordered breathing events from a signal generated by a photoplethysmography (PPG) sensor. The method may include receiving data representing the signal from the PPG sensor. The method may include deriving a PPG-based respiratory signal from the data representing the signal from the PPG sensor. The method may include computing first level features from the PPG-based respiratory signal. The method may include computing second level features from the first level features. The method may include evaluating, such as in a classifier, the first level features and the second level features. The method may include generating, based on the evaluation such as of the classifier, an output indication that identifies a sleep disordered breathing type.

In some implementations, the output indication that identifies the sleep disordered breathing type characterizes a plurality of events of a sleep session. The method may include may further include deriving a peripheral arterial tonometry (PAT) signal from the data representing the signal from the PPG sensor. The method may further include detecting one or more respiratory events based on the PAT signal. The output indication that identifies a sleep disordered breathing type characterizes the one or more respiratory events. The PPG sensor may be a finger sensor. The output indication may identify a central sleep apnea type. The central sleep apnea type may include a mixed central and obstructive sleep apnea type. The output indication may identify an obstructive sleep apnea type.

The computing the first level features may include computing values from a plurality of windows of the PPG-based respiratory signal. The computed values may include, for each of the plurality of windows, any one or more of a range, an interquartile range, a variance, and a number of local maxima. The plurality of windows may include windows of a first uniform size moving along the PPG-based respiratory signal. The plurality of windows may further include windows of a second uniform size moving along the PPG-based respiratory signal, the second uniform size being shorter than the first uniform size. Computing the second level features from the first level features may include selecting a subset of values for each of the first level features wherein the subset of values may be associated with subset of windows, of the plurality of windows, that correspond with occurrence of the detected respiratory event. Computing the second level features from the first level features may include computing, from the selected subset of values, one or more values of the second level features. The one or more computed values of the second level features may include any one or more of: a minimum value, an average of lowest three values, and one or more percentile values. Deriving a PPG-based respiratory signal from the data representing the signal from the PPG sensor may include filtering baseline modulations of the data representing the signal from the PPG sensor to generate a filtered signal. Deriving a PPG-based respiratory signal from the data representing the signal from the PPG sensor may include generating an envelope signal from peaks of the filtered signal. Optionally, deriving a PPG-based respiratory signal from the data representing the signal from the PPG sensor further may include normalizing the envelope signal by subtracting a moving average window of the envelope signal and dividing the envelope signal by a moving interquartile range window of the envelope signal. The evaluation may be in a classifier, or the evaluation may be in a classifier that may include an ensemble of trees classifier.

In some implementations, the method may further include detecting an event of vasoconstriction from the data representing the signal from the PPG sensor. The output indication may be based on the detected event of vasoconstriction. Detecting an event of vasoconstriction may include filtering the PPG signal data representing the signal from the PPG sensor to produce a filtered PPG signal. The method may include processing the filtered PPG signal to detect a trough attributable to a vasoconstriction. The method may include omitting, from the evaluation (e.g., of the classifier), one or more values of one or both of the first level features and second level features that are associated with the detected event of vasoconstriction. The method may include detecting an event of arrythmia from the data representing the signal from the PPG sensor. The output indication may be based on the detected event of arrythmia. Optionally, the detecting an event of arrythmia may include detecting one or more irregular peak-to-peak interval zones in the PPG signal that are associated with arrythmia. Optionally, the method may include omitting, from the evaluation (e.g. of the classifier), data corresponding to at least one respiratory event, of the one or more respiratory events, that may be associated with the detected one or more irregular peak-to-peak interval zones. Optionally, the detecting the one or more irregular peak-to-peak interval zones may include computing an absolute value of a difference of two neighboring peak-to-peak intervals. The detecting the one or more irregular peak-to-peak interval zones may include computing an average value of absolute values of differences of neighboring peak-to-peak intervals of the PPG signal. The detecting the one or more irregular peak-to-peak interval zones may include comparing the absolute value and the average value.

In some implementations, the method may further include generating a signal for controlling operation of a respiratory therapy apparatus based on the output indication. The controlling operation may include controlling a pressure or flow therapy of a blower of the respiratory therapy apparatus. Optionally, receiving the data representing the signal from the PPG sensor may include receiving the data at a server.

Some implementations of the present technology may include processor-readable medium, having stored thereon processor-executable instructions which, when executed by one or more processors, cause the one or more processors to detect a sleep disordered breathing event from a signal generated by a photoplethysmography (PPG) sensor. The processor-executable instructions may include instructions to receive data representing the signal from the PPG sensor. The processor-executable instructions may include instructions to detect an event of vasoconstriction and/or an event of arrythmia from the data representing the signal from the PPG sensor. The processor-executable instructions may include instructions to evaluate the data representing the signal from the PPG sensor to generate, based on the evaluation and the detected event of vasoconstriction and/or the detected event of arrythmia, an output indication that identifies a sleep disordered breathing event.

In some implementations, the processor-readable medium may include the instructions to detect the event of vasoconstriction, wherein the instructions to detect the event of vasoconstriction may include instructions to detect signal depression attributable to vasoconstriction. Optionally, the instructions to detect signal depression may include instructions to filter the PPG signal data representing the signal from the PPG sensor to produce a filtered PPG signal. The instructions to detect signal depression may include instructions to process the filtered PPG signal to detect a trough attributable to vasoconstriction. The processor-readable medium may include instructions to evaluate the data representing the signal from the PPG sensor that generate the output indication based on the detected event of vasoconstriction. To generate the output indication based on the detected event of vasoconstriction, the instructions to evaluate the data representing the signal from the PPG sensor may include instructions to disregard one or more values of one or more features computed from the data that are associated with the detected event of vasoconstriction.

In some implementations, the processor-readable medium may include the instructions to detect the event of arrythmia, wherein the instructions to detect the event of arrythmia may include instructions to detect irregular zones attributable to arrythmia. Optionally, the instructions to detect the irregular zones attributable to arrythmia may include instructions to detect one or more irregular peak-to-peak interval zones in the PPG signal that are associated with arrythmia. The instructions to detect the one or more irregular peak-to-peak interval zones may include instructions to compute an absolute value of a difference of two neighboring peak-to-peak intervals. The instructions to detect the one or more irregular peak-to-peak interval zones may include instructions to compute an average value of absolute values of differences of neighboring peak-to-peak intervals of the PPG signal. The instructions to detect the one or more irregular peak-to-peak interval zones may include instructions to compare the absolute value and the average value. The processor-readable medium may include the instructions to evaluate the data representing the signal from the PPG sensor that generate the output indication based on the detected event of arrythmia. To generate the output indication based on the detected event of arrythmia, the instructions to evaluate the data representing the signal from the PPG sensor may include instructions to disregard at least one respiratory event detected from the data representing the signal from the PPG sensor that may be associated with the detected irregular zones. The processor-executable instructions may further include instructions to generate a signal for controlling operation of a respiratory therapy apparatus based on the output indication. The controlling operation may include controlling a pressure or flow therapy of a blower of the respiratory therapy apparatus. The instructions to receive the data representing the signal from the PPG sensor may include instructions for receiving the data at a server.

Some implementations of the present technology may include server with access to any processor-readable medium as described herein, wherein the server may be configured to receive requests for downloading the processor-executable instructions of the processor-readable medium to a processing device over a network.

Some implementations of the present technology may include processing device that may include: one or more processors; and a processor-readable medium described herein, or the processing device may be configured to access the processor-executable instructions with a server described herein. The processing device may be a respiratory therapy apparatus. The processing device may be configured to generate a pressure therapy or a flow therapy.

Some implementations of the present technology may include method of a server having access to any processor-readable medium described herein. The method of the server may include receiving, at the server, a request for downloading the processor-executable instructions of the processor-readable medium to an electronic processing device over a network. The method of the server may include transmitting the processor-executable instructions to the electronic processing device in response to the request.

Some implementations of the present technology may include a method of one or more processors for detecting a sleep disordered breathing event from a signal generated by a photoplethysmography (PPG) sensor. The method may include accessing, with the one or more processors, any processor-readable medium described herein. The method may include executing, in the one or more processors, the processor-executable instructions of the processor-readable medium.

Some implementations of the present technology may include method for detecting a sleep disordered breathing event from a signal generated by a photoplethysmography (PPG) sensor. The method may include receiving data representing the signal from the PPG sensor. The method may include detecting an event of vasoconstriction and/or an event of arrythmia from the data representing the signal from the PPG sensor. The method may include evaluating the data representing the signal from the PPG sensor to generate, based on the evaluation and the detected event of vasoconstriction and/or the detected event of arrythmia, an output indication that identifies a sleep disordered breathing event.

In some implementations, the method may include the detecting the event of vasoconstriction, wherein detecting the event of vasoconstriction may include detecting signal depression attributable to vasoconstriction. The detecting signal depression may include filtering the PPG signal data representing the signal from the PPG sensor to produce a filtered PPG signal. The detecting signal depression may include processing the filtered PPG signal to detect a trough attributable to vasoconstriction. The method may include evaluating the data representing the signal from the PPG sensor to generate the output indication based on the detected event of vasoconstriction. To generate the output indication based on the detected event of vasoconstriction, the evaluating the data representing the signal from the PPG sensor may include disregarding one or more values of one or more features computed from the data that may be associated with the detected event of vasoconstriction.

In some implementations, the method may include detecting the event of arrythmia, wherein the detecting the event of arrythmia may include detecting irregular zones attributable to arrythmia. Optionally, the detecting the irregular zones attributable to arrythmia may include detecting one or more irregular peak-to-peak interval zones in the PPG signal that are associated with arrythmia. The detecting the one or more irregular peak-to-peak interval zones may include computing an absolute value of a difference of two neighboring peak-to-peak intervals. The detecting the one or more irregular peak-to-peak interval zones may include computing an average value of absolute values of differences of neighboring peak-to-peak intervals of the PPG signal. The detecting the one or more irregular peak-to-peak interval zones may include comparing the absolute value and the average value. The method may include the evaluating the data representing the signal from the PPG sensor to generate the output indication based on the detected event of arrythmia. To generate the output indication based on the detected event of arrythmia, the evaluating the data representing the signal from the PPG sensor may include disregarding at least one respiratory event detected from the data representing the signal from the PPG sensor that may be associated with the detected irregular zones. The method may further include generating a signal for controlling operation of a respiratory therapy apparatus based on the output indication. The controlling operation may include controlling a pressure or flow therapy of a blower of the respiratory therapy apparatus. The receiving the data representing the signal from the PPG sensor may include receiving the data at a server.

Other features of the technology will be apparent from consideration of the information contained in the following detailed description, abstract, drawings and claims.

## 4 BRIEF DESCRIPTION OF THE DRAWINGS

The present technology is illustrated by way of example, and not by way of limitation, in the figures of the accompanying drawings, in which like reference numerals refer to similar elements including:

FIG. 1 shows a peripheral arterial tonometry (PAT) device 102 that may be implemented for SA detection as described herein, in accordance with examples of the present technology.

FIG. 2 includes graphs of airflow and effort signals determined from PSG (polysomnography) that illustrate, by comparison, a typical central event and typical obstructive respiratory event. Both events are accompanied by cessation of airflow, but a central event is characterized by lack of respiratory effort which is reflected in a flat region of the respiratory effort belt signal.

FIG. 3 is a graph of a segment of a PPG signal and an envelope illustrating the respiratory effort modulations.

FIG. 4 includes two graphs showing, by comparison, a respiratory effort signal that is extracted from the finger PPG and a respiratory effort channel of the PSG.

FIG. 5 illustrates an event labelling procedure;

FIG. 6 includes two graphs showing a PPG signal (top) and respiratory effort signal derived from the PPG signal (bottom);

FIG. 7 includes three graphs showing a PPG signal (top), a respiratory effort signal derived from the PPG signal (middle), and a respiratory effort signal from a PSG (bottom);

FIG. 8 is a graph with sensitivity and specificity data for an example implementation of the present technology.

FIG. 9 includes graph scatterplots of the central sleep apnea predicted by an example implementation of the present technology along with expert labelling of such event by two diffeent expert groups.

FIG. 10 shows the Negative Predictive Values (NPV) and Positive Predictive Values (PPV), and their sum for various central apnea hypopnea index (cAHI) cutoff thresholds for screening of patients.

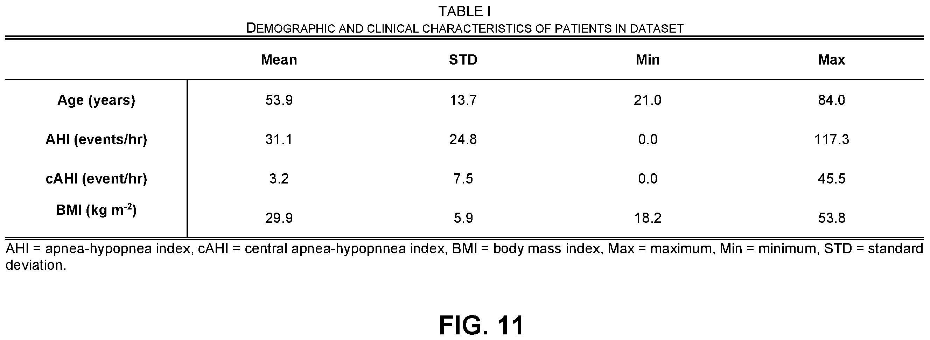

FIG. 11 is a table of demographic and clinical characteristics of a dataset involved in training an example implementation of the technology;

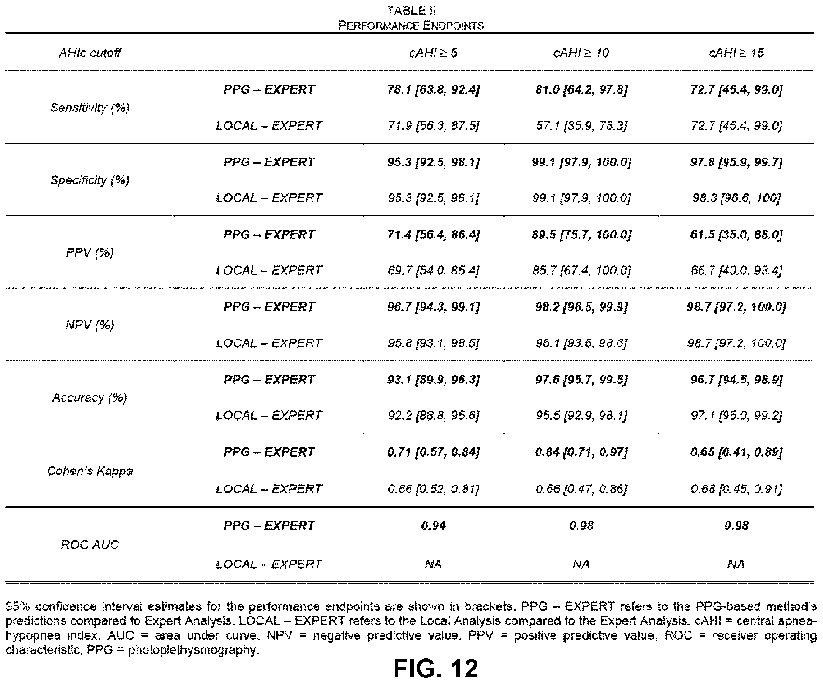

FIG. 12 is a table of performance endpoints of an example implementation of the technology;

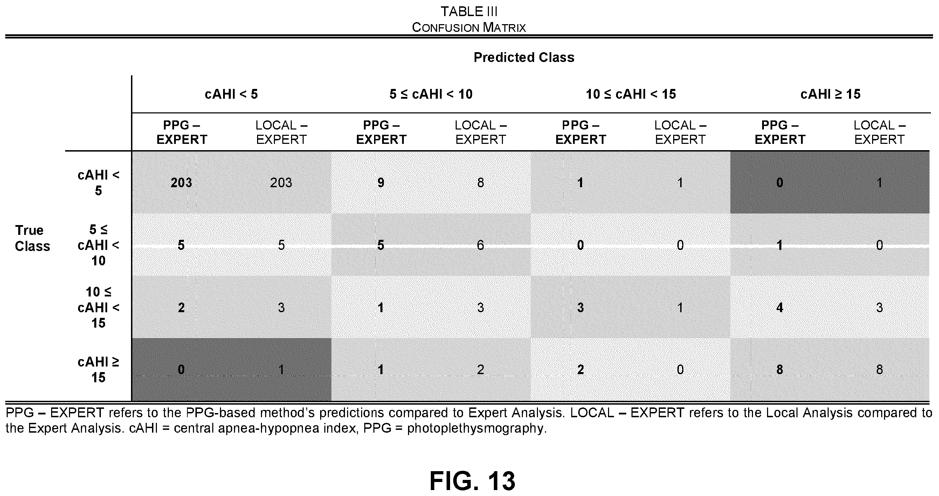

FIG. 13 is a confusion matrix illustrating classification performance of an example of the present technology.

FIG. 14A is an example methodology of one or more processors, such as in a system of the present technology, for detecting or distinguishing between different types of sleep disordered breathing events.

FIG. 14B is another example methodology of one or more processors, such as in a system of the present technology, for distinguishing between different types of sleep disordered breathing events.

FIG. 14C is an example methodology of one or more processors, such as in a system of the present technology, for detecting (such as by distinguishing between different types) of sleep disordered breathing events, based on a detection of arrythmia events and/or detection of vasoconstriction events.

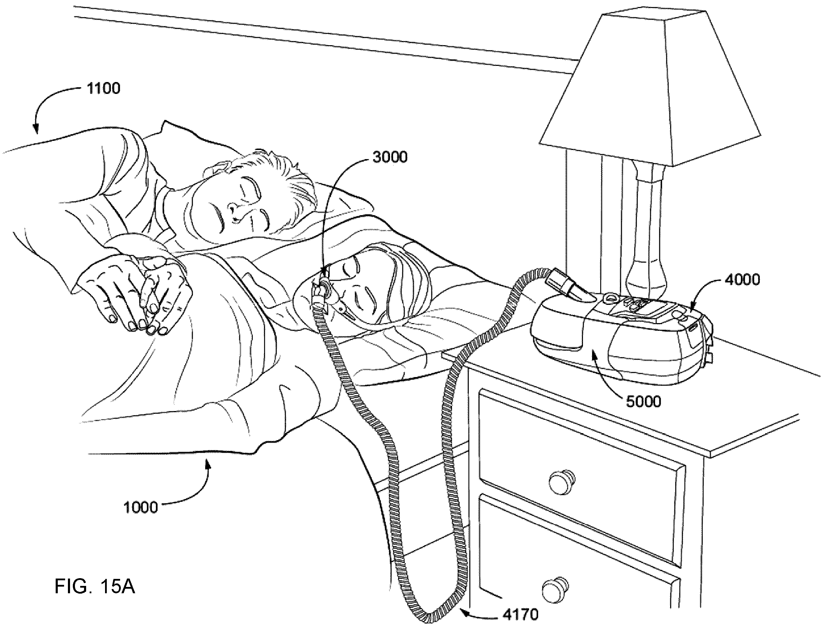

Fig. 15A shows a system in accordance with the present technology. A patient 1000 wearing a patient interface 3000 receives a supply of pressurised air from an RPT device 4000. Air from the RPT device 4000 is humidified in a humidifier 5000, and passes along an air circuit 4170 to the patient 1000. A bed partner 1100 is also shown.

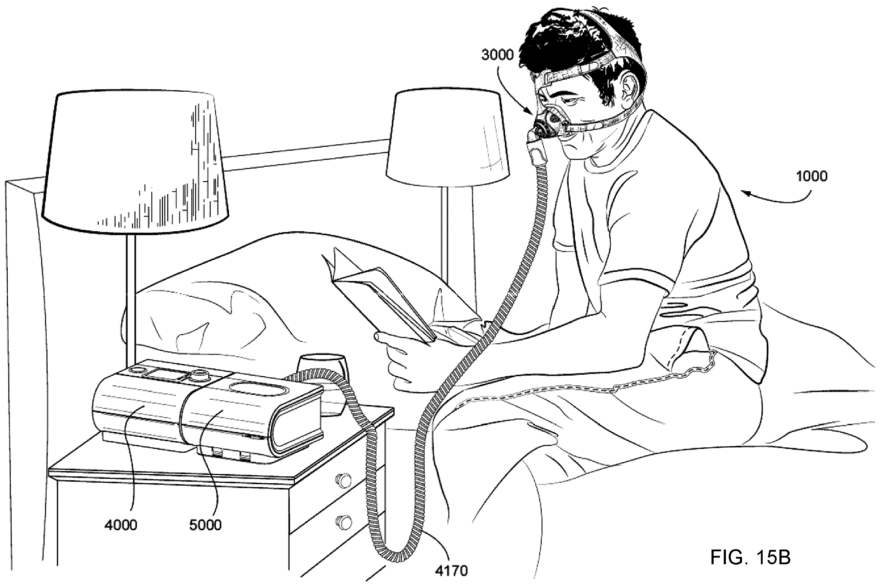

Fig. 15B shows an RPT device 4000 in use on a patient 1000 with a nasal mask 3000.

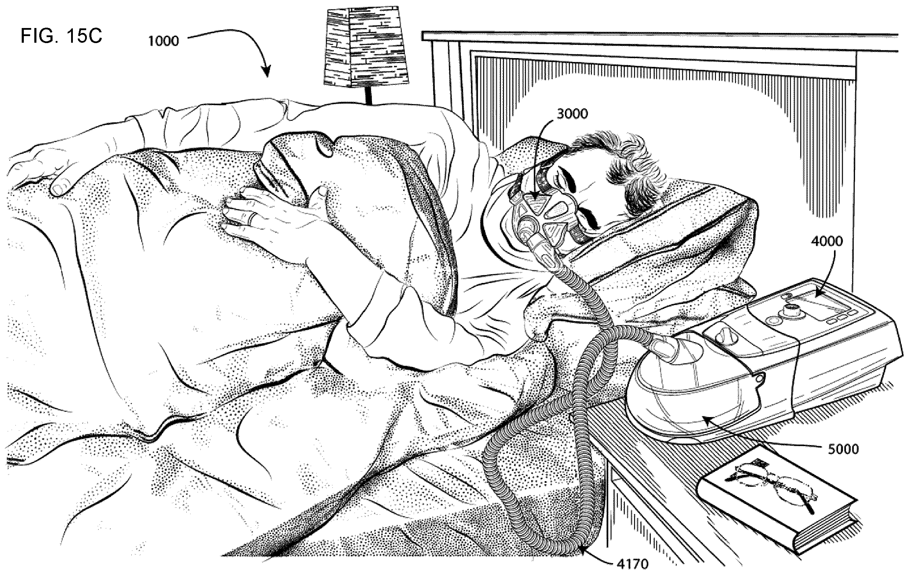

Fig. 15C shows an RPT device 4000 in use on a patient 1000 with a full-face mask 3000.

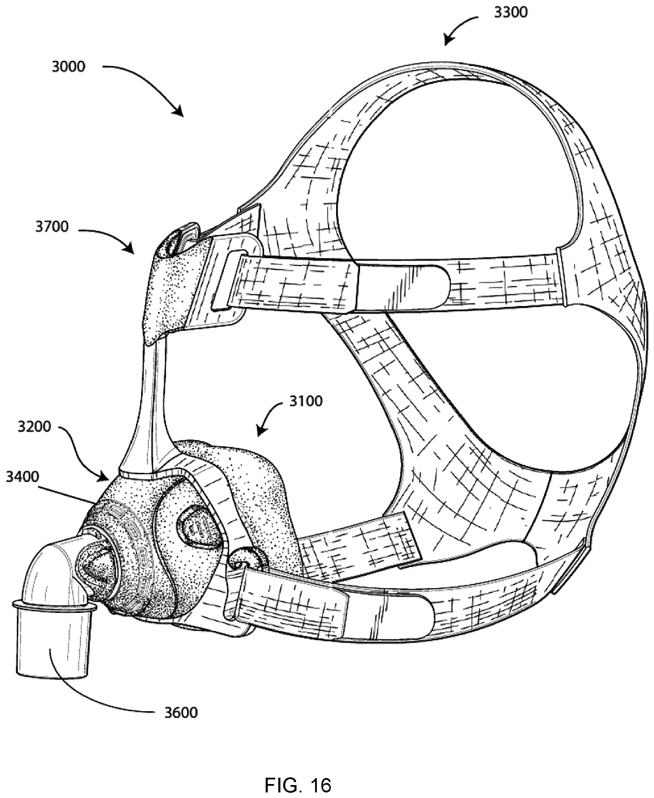

Fig. 16 shows a non-invasive patient interface 3000 in the form of a nasal mask.

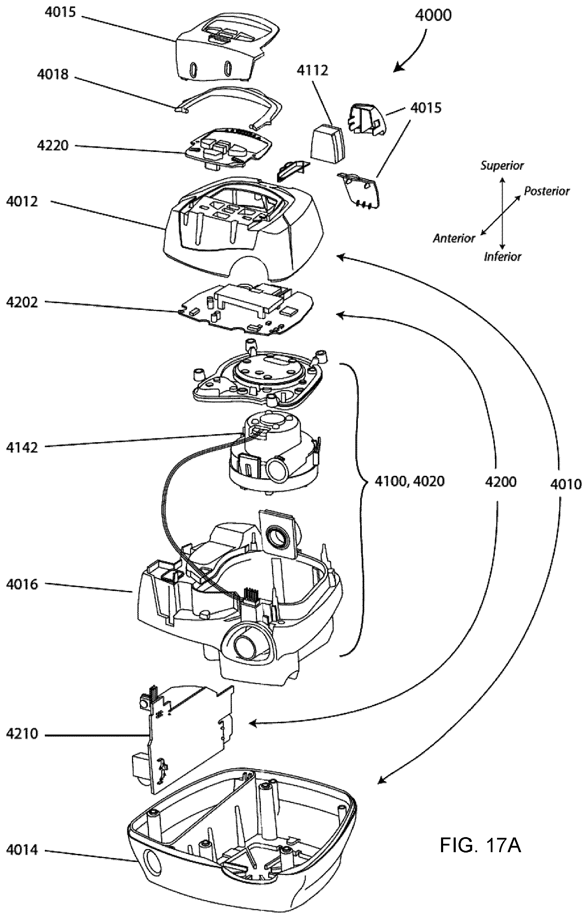

Fig. 17A shows an RPT device 4000 in accordance with one form of the present technology.

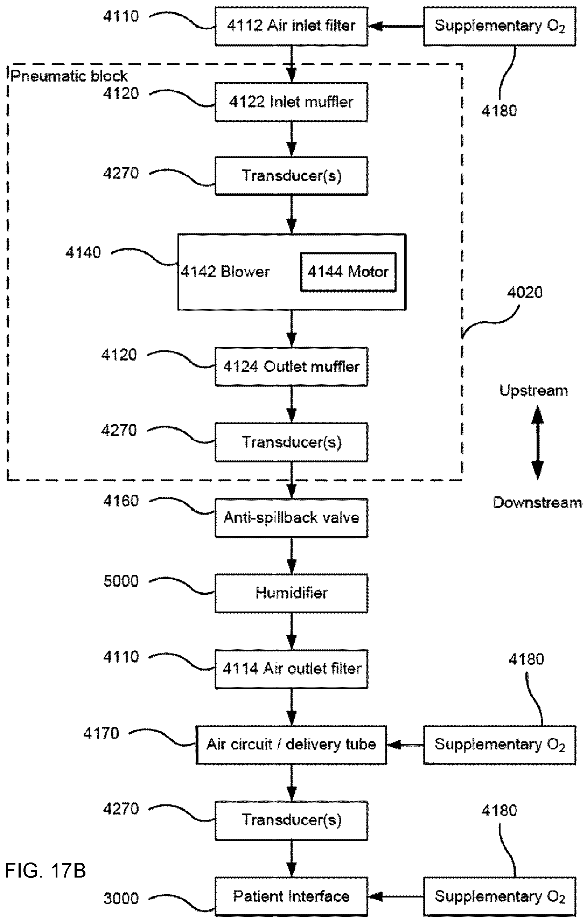

Fig. 17B shows a schematic diagram of the pneumatic circuit of an RPT device 4000 in accordance with one form of the present technology. The directions of upstream and downstream are indicated.

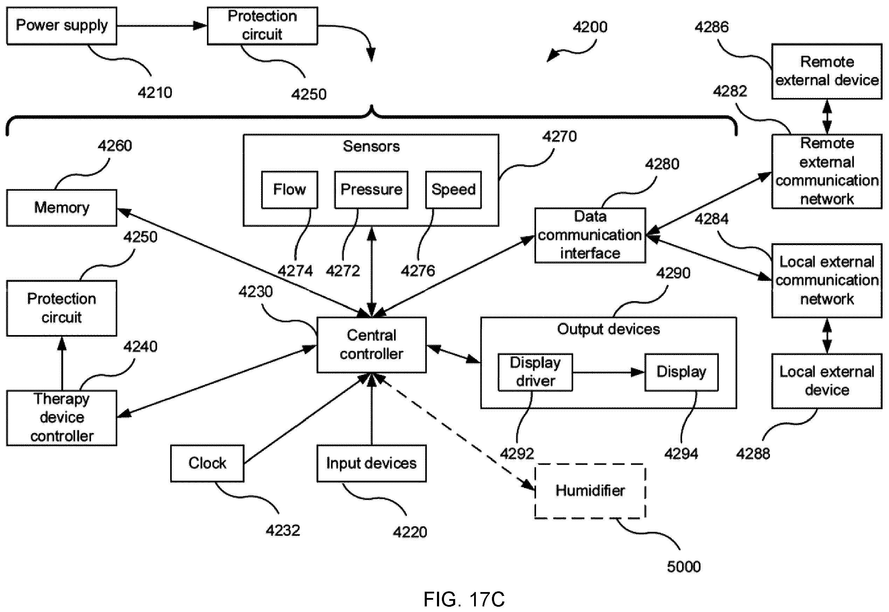

Fig. 17C shows a schematic diagram of the electrical components of an RPT device 4000 in accordance with one aspect of the present technology.

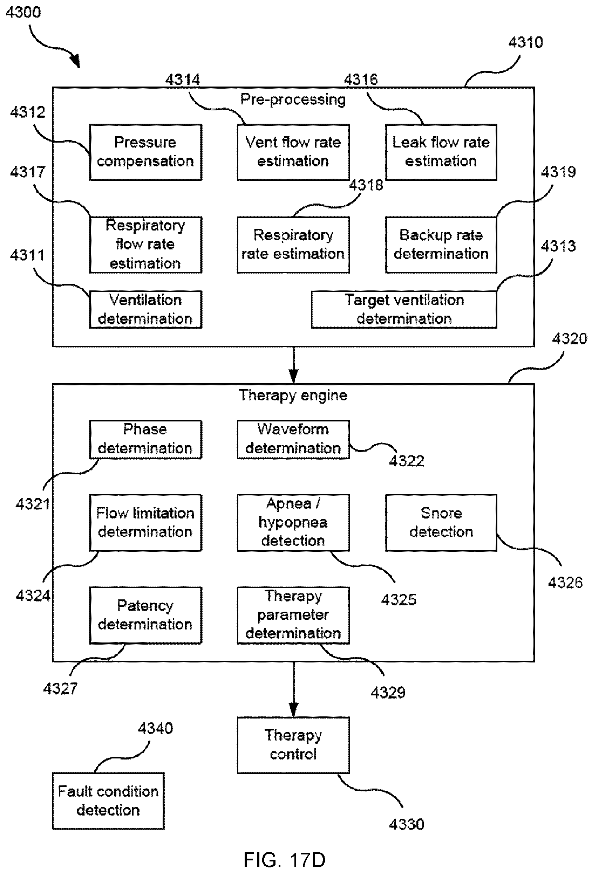

Fig. 17D shows a schematic diagram of the algorithms 4300 implemented in an RPT device 4000 in accordance with an aspect of the present technology. In Fig. 9D, arrows with solid lines indicate an actual flow of information, for example via an electronic signal.

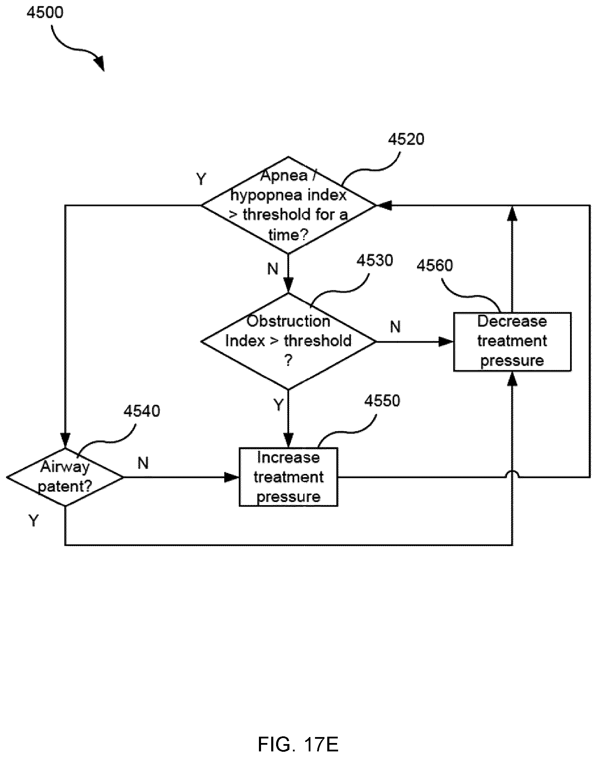

Fig. 17E is a flow chart illustrating a method 4500 carried out by the therapy engine module 4320 of Fig. 9D in accordance with one aspect of the present technology.

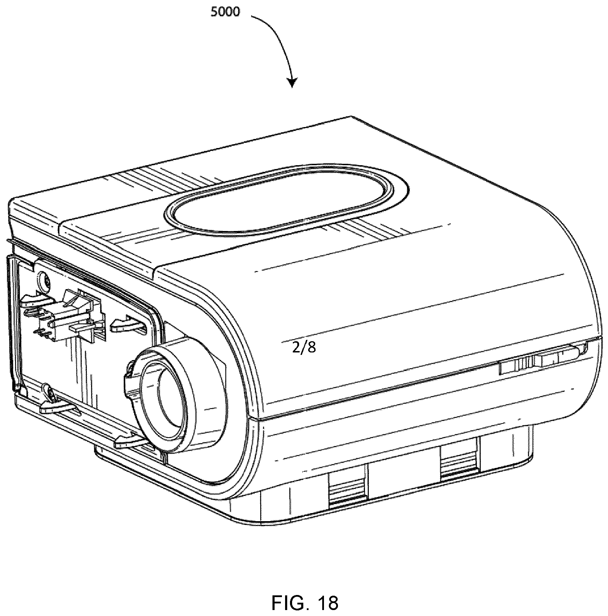

Fig. 18 shows a humidifier 5000.

## 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

The present technology concerns processes for detection of or distinguishing between central and obstructive respiratory events from data representing a signal from a finger photoplethysmography sensor. The described method(s) can be incorporated into a system such as for minimally invasive central sleep apnea screening and provide improved apparatus such as for detection, screening, monitoring, diagnosis or therapy for such sleep related respiratory issues

A PAT sensing device 102 makes use of a fingertip mounted PPG probe [20]. PPG operates with an optical technology that detects blood volume changes in the tissue's microvascular bed [23], [24]. PPG measurements are used to derive the arterial blood oxygen saturation ($SpO_2$), pulse rate (PR), and changes in peripheral arterial tone, which are then used to detect respiratory events [20]. Peripheral arterial tone (PAT) refers to the tone of the peripheral arterial smooth muscle tissue. When the muscle tone of peripheral arteries increases, the arteries' diameter decreases, resulting in a reduction of perfusion and thus a decrease in pulsatile blood volume in the peripheral tissue. The latter is picked up as a drop in the PPG signal swing between systole and diastole. The PAT signal may be derived from the PPG signal from the PPG sensor, such as by the method described in PCT/EP2021/067532. The PPG-derived signal, which may be derived by trending such pulsatile blood volume reductions, is referred to as the PAT signal. A PAT sensing device analyzes the concurrence of drops in $SpO_2$, surges in pulse rate, and increases in peripheral arterial tone. As airflow is reduced, the oxygen supply to the lungs decreases from baseline, resulting in distinct $SpO_2$ drops. Near the end of a respiratory event, the sympathetic nervous system activity spikes to arouse the patient, resulting in resumption of ventilation, a surge in pulse rate and a release of norepinephrine in the blood stream. Consequently, norepinephrine binds to alpha-adrenergic receptors innervating the arterial smooth muscle tissue in the finger, causing sudden vasoconstrictions, or synchronously increases in PAT, picked up by the PPG sensor [20].

Such a PPG sensor may be implemented for detection of respiratory events, such as by detecting signal characteristics indicative of apnea and/or hypopnea events within a peripheral arterial tonometry (PAT) signal that can be derived from the sensor's PPG signal. As discussed in more detail herein, the PPG signal may also be processed to extract respiratory effort-related information from which signal processing, computing and/or classification techniques may be applied for detection of or discriminating between central type and obstructive type for the respiratory events.

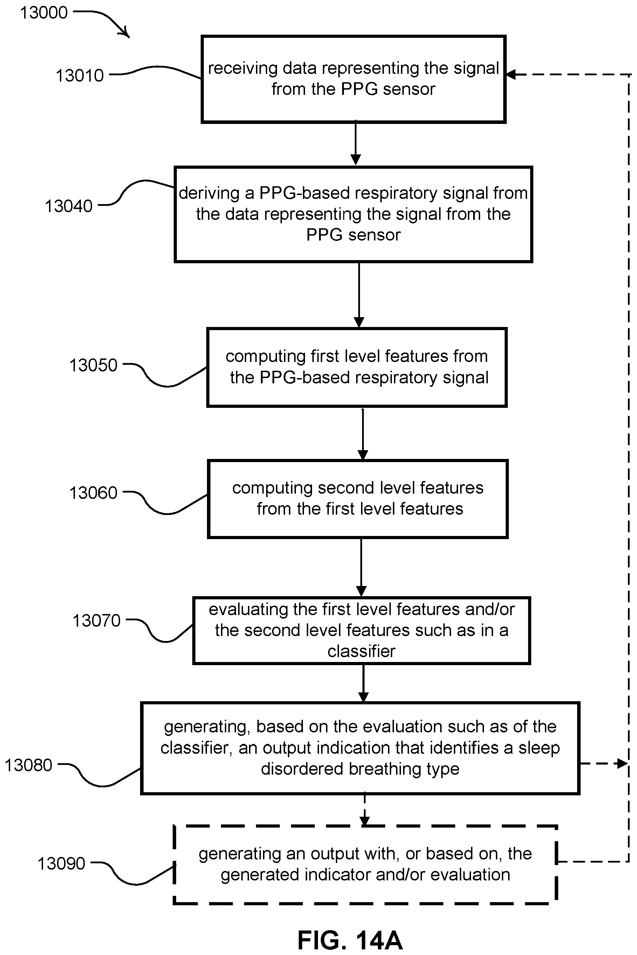

Thus, as described in more detail in the context of examples herein, any one or more of the following techniques may be applied to a signal from the PPG sensing device. For example, as illustrated in Fig. 14A, some implementations may include a method 13000 of one or more processors for distinguishing sleep disordered breathing events from a signal generated by a photoplethysmography (PPG) sensor. Such a method may include receiving data representing the signal from the PPG sensor in process 13010. The method may include deriving a PPG-based respiratory signal (e.g., respiratory effort signal) from the data representing the signal from the PPG sensor at process 13040. The method may include computing first level features from the PPG-based respiratory signal at process 13050. The method may include computing second level features from the first level features at process 13060. The method may include evaluating the first level features and/or the second level features, such as in a classifier, at process 13070. The method may include generating, based on the evaluation, such as of the classifier, an output indication that identifies a sleep disordered breathing type at process 13080. Such a type identification may be on a respiratory event basis or on a sleep session basis. For example, the type indication may be for an apnea event that is central or obstruction or it may be for some interval such as a session (e.g., a night of sleep or several nights of sleep such as with a screening device) that can be characterized as a session influenced by central type sleep apnea. The method may also include, at 13090, generating output with, or based on, the generated indicator and/or evaluation, such as by comparing the indicator to a threshold. Such output may include an electronic communication or transmission of the indicator and/or a control instruction for operating a therapy device, such as a setting for the device.

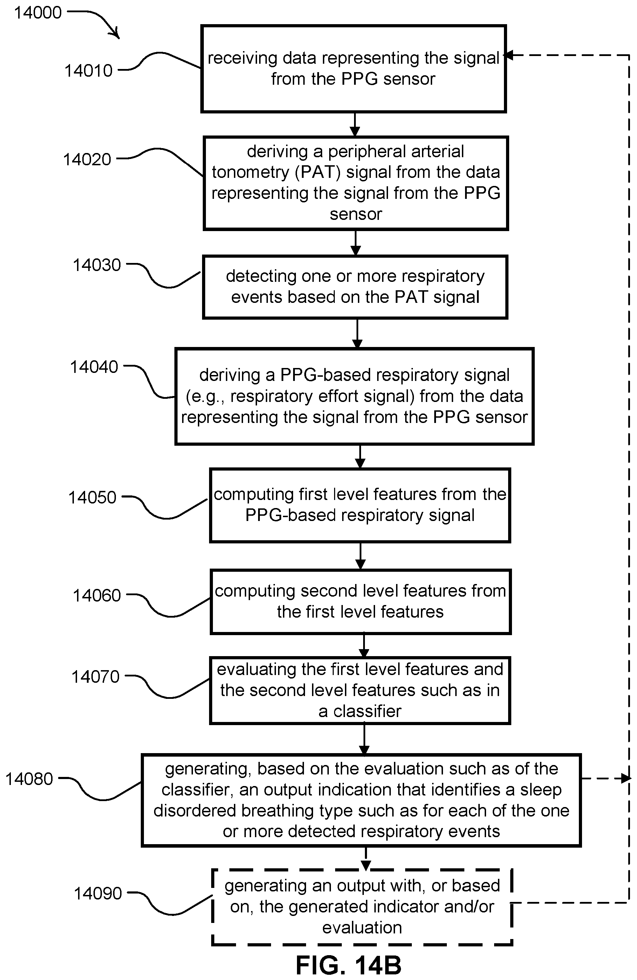

By way of further example, as illustrated in Fig. 14B, some implementations may include a method 14000 of one or more processors for detecting, such as by distinguishing, sleep disordered breathing events from a signal generated by a photoplethysmography (PPG) sensor. Such a method may include receiving data representing the signal from the PPG sensor in process 14010. The method may include deriving a peripheral arterial tonometry (PAT) signal from the data representing the signal from the PPG sensor at process 14020. The method may include detecting one or more respiratory events based on the PAT signal at process 14030. The method may include deriving a PPG-based respiratory signal from the data representing the signal from the PPG sensor at process 14040. The method may include computing first level features from the PPG-based respiratory signal at process 14050. The method may include computing second level features from the first level features at process 14060. The method may include evaluating the first level features and/or the second level features, such as in a classifier, at process 14070. The method may include generating, based on the evaluation, such as of the classifier, an output indication that identifies a sleep disordered breathing type for each of the one or more detected respiratory events at process 14080. The method may also include, at 14090, generating output with, or based on, the generated indicator and/or evaluation, such as by comparing the indicator to a threshold. Such output may include an electronic communication or transmission of the indicator and/or a control instruction for operating a therapy device, such as a setting for the device.

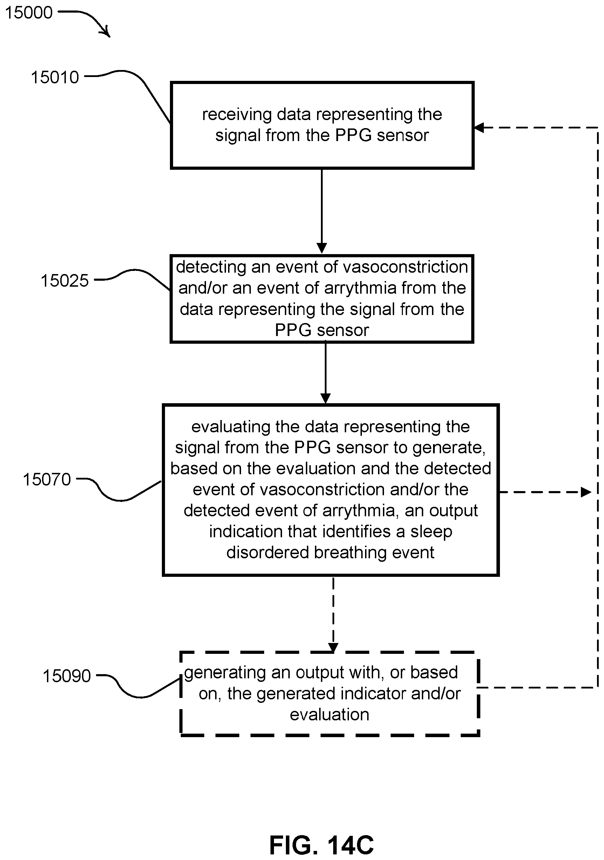

By way of further example, as illustrated in Fig. 14C, the processing of the present technology may include a method 15000 for detecting a sleep disordered breathing event (e.g., one or more) from a signal generated by a photoplethysmography (PPG) sensor. The method, at 15010 receiving data representing the signal from the PPG sensor. The method may include, at 15025, detecting an event of vasoconstriction and/or an event of arrythmia from the data representing the signal from the PPG sensor. The method may include, at 15070, evaluating the data representing the signal from the PPG sensor to generate, based on the evaluation and the detected event of vasoconstriction and/or the detected event of arrythmia, an output indication that identifies a sleep disordered breathing event based on the detected event of vasoconstriction. The method may also include, at 15090, generating output with, or based on, the generated indicator and/or evaluation, such as by comparing the indicator to a threshold. Such output may include an electronic communication or transmission of the indicator and/or a control instruction for operating a therapy device, such as a setting for the device.

Although such processes are illustrated in an order, such ordering is not strictly required, such as in the case where some of the processes may be performed in parallel or in a different ordering or where certain aspects are performed without other aspects that are not required.

Such techniques/processes may be performed by one or more processors or other processing apparatus 104 or computer, such as a cloud-based processing system (e.g., one or more servers) or other processing device in communication with or receiving signals or data from (directly or indirectly) the PAT sensing device 102, so as to serve as a system 100. Such processing apparatus 104 or device may include, for example, a controller, central controller or other processor described herein, such as a processor of an RT or RPT or a remote processing device or server in communication with such a controller. Thus, the PPG based determination with the sensing device may serve as a basis for (locally or remotely) controlling or setting operations, such as making therapy changes (e.g., control of a pressure or flow therapy), of an RT device as discussed in more detail herein. Thus, the techniques of the one or more methodologies described herein may be implemented as computer programs or processor control instructions stored in a non-transitory computer readable storage medium, such as memory that is accessible to the processing apparatus described herein. Moreover, as noted herein, the features of the signals may be evaluated in a machine algorithm or process that may be a machine learning process, such as a classifier, of the one or more processors. However, such a processor-based evaluation may also or alternatively be implemented as a rules-based logistic decision algorithm or other computer evaluation involving a logic inference engine configured to evaluate sensor data, or features calculated therefrom, with suitable thresholds. Such thresholds may be determined by empirical analysis or study.

Details of example aspects of such methodologies may be considered further herein.

*i. Respiratory signal extraction from raw PPG*

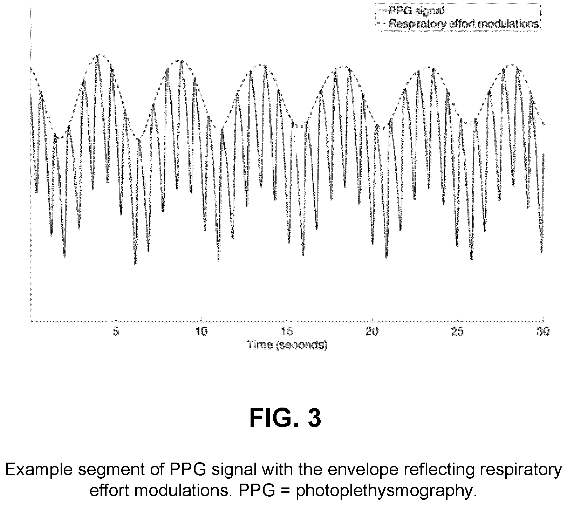

As previously noted, the PPG signal measured at the fingertip contains respiratory information. Such information is present since the blood flow to body extremities gets affected by alterations in thoracic pressure throughout the respiratory cycle [20]. Therefore, the PPG signal amplitude oscillates in synchrony with the respiratory cycle. This amplitude modulation can be isolated to retain a signal representing respiratory effort. Fig. 3 shows the PPG signal along with the respiratory modulations present in the signal. The present technology may utilize the presence or absence of these breathing modulations in the finger PPG to infer the type of a respiratory event.

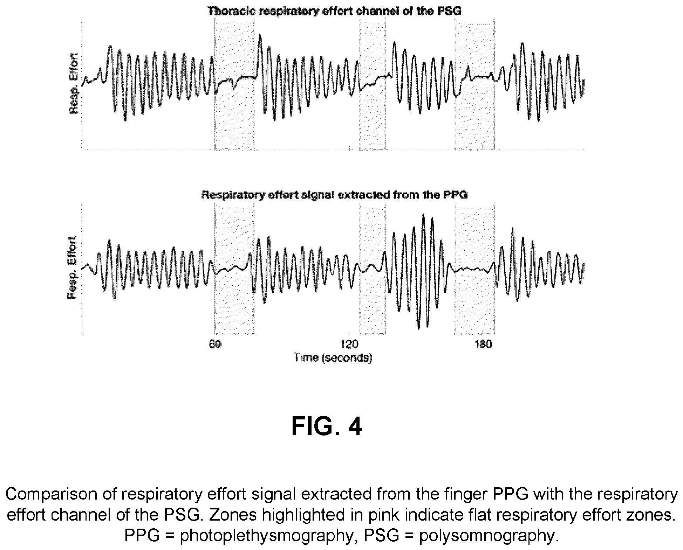

To this end, the respiratory modulation may be extracted from the raw PPG signal by obtaining or generating an envelope of the signal. For example, the raw PPG signal may be filtered, such as by a high-pass-filter at a suitable frequency (e.g., 0.15 Hz) to remove slowly varying baseline modulations. Breathing frequency at rest is usually 0.2 Hz or higher (the normal breathing frequency range for an adult is 0.2-0.33 Hz) and, therefore, the breathing modulations remain substantially unaffected by such low-end filtering. Subsequently, a peak envelope of the filtered PPG signal may be calculated, such as with a peak detector or other envelope detection methodology. The envelop signal may optionally be normalized by subtracting a moving average window (e.g., 6 second average) of this envelope and dividing it by a moving window interquartile range window (e.g., a 30-second moving window interquartile range window). The initial moving average window (e.g., 6-second window) can help to remove signal baseline fluctuations while retaining breathing modulations. The latter moving window (e.g., the 30-second window) can help to normalize any steady-state changes in the signal amplitude. The resulting signal, which may be referred to as a PPG-derived respiratory effort (PPGDR) signal, may then be analysed or processed further such as for classification by a classifier such as by determining or calculating classification features from the PPGDR) signal. Fig. 4 shows a comparison of a segment of the PPGDR to the corresponding respiratory effort segment from the thoracic effort belt of the PSG.

*ii. Feature calculation*

Thus, after PPGDR signal extraction, features may be extracted from the PPGDR signal such as by using one or more moving window or sliding-window operation(s). Such a "window" in signal processing generally concerns a subset of samples of a time based signal and different windows can include different subsets of samples taken from the time based signal. For example, two window sizes may be implemented of shorter and longer duration (e.g., 11 and 21 seconds). The window(s) may be shifted over or along the PPGDR signal in steps (such as a stride of 1 second). First level feature values may then be calculated for each window's samples of the signal so as to be ascribed to the one-second interval at the center of each window. This results in a set of first level feature values for each first level feature derived from the shorter windows and a set of first level feature values for each first level feature for the longer windows.

The first level features computed for each window may include any one or more of, for example, the range, the interquartile range, the variance, and the number of PPGDR local maxima (peaks). Optionally, the range of the signal may additionally be computed for other window sizes such as 5 and 7 seconds.

For local maxima-related features, local maxima detection may be performed on the PPGDR signal (a minimum peak-to-peak distance may, for example, be set to approximately 250 milliseconds), where the peak-to-peak interval represents the duration of a breathing cycle. A lack of local maxima suggests a lack of breathing effort.

Second level features may then also be computed, such as by deriving values for them from the first level features, or a chosen subset of the values from the first level features. For example, second level features may be determined such that they correlate with particular respiratory events that are otherwise detected in the signal from PAT sensing device. In this regard, feature design may account for the variability in duration and location of the flat zones in the PPGDR signal during a respiratory event. As such, for each respiratory event detected by evaluation of the PAT sensing device signal, such as by a detection methodology that determines a flat zone in the PAT signal, the moving-window feature values, or first level features values, for which the center-second overlapped in time with a detected respiratory event period, can be selected. That is, the second level features may be determined from first level feature values that had been derived from windows that overlap with the respiratory event. From these selected first level features values, second level features are calculated/determined. Such second level features may include, any one or more of, for example, the minimum value of the selected first level feature values, the average of the lowest three values of the selected first level feature values, and one or more percentiles such as the 10th and/or 25th percentiles of the selected first level feature values. This results in a second level feature value for each first level feature.

For local maxima-related features, the aggregation operations comprised computing the minimum value and the numbers and proportions of windows with zero, one, and two peaks. The resulting aggregated feature sets for each respiratory event may be used to support subsequent steps described herein.

*iii. Signal Rejection*

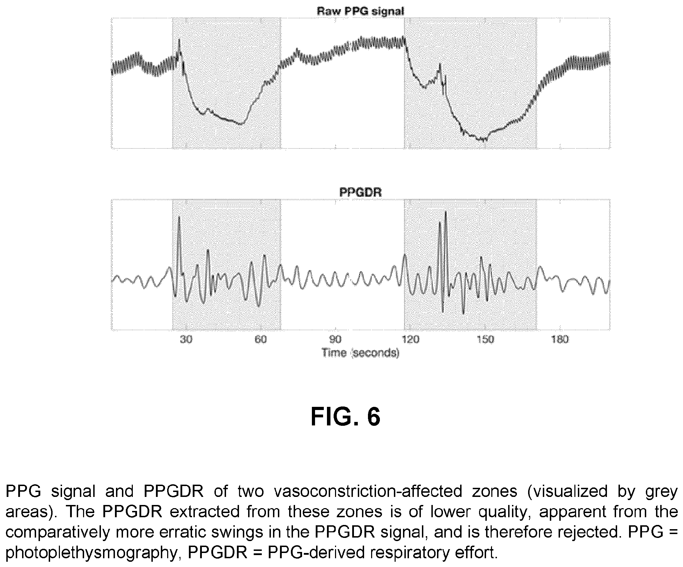

In implementations, such as where only a PPG signal is used to extract the PPGDR signal, the quality of type determination (e.g., classification) is heavily dependent on the underlying quality of the PPG data. Strong vasoconstrictions reflected in sudden drops in PPG pulse amplitude frequently contain low signal-to-noise ratio episodes and do not get sufficiently canceled out by normalization operations. This significantly reduces PPGDR quality. Thus, the system may be configured to recognize vasoconstrictions to improve quality of the sleep disordered breathing event detection and/or type distinction, such that the sleep disordered breathing event detection and/or type distinction is based on the vasoconstriction event detection. For example, a vasoconstriction events may be detected from a signal from the sensor by detection of a signal depression in PPG and/or PAT signal, such as by detecting/identifying troughs (vasoconstriction-affected periods or zones) of significant prominence in a filtered version of the PPG signal. This filtering of the PPG signal may be performed to remove baseline shifts and shifts in perfusion that might otherwise impact trough detection. Fig. 6 shows an example of such a vasoconstriction-affected zone. Alternatively, such periods or zones may be detected by detection of a period of signal drop in a PAT signal that is derived from the PPG signal.

Other methodologies for detections of such signal depressions may also, or alternatively, be implemented for such vasoconstriction event detection. For example, the PAT or PPG signal may be processed to detect a "V" shape, which may be characterized by detection of a declining and increasing slope in the signal, or a baseline thereof, with a central minimum value. Such characterizations may, for example, be implemented with a curve fitting or best fitting technique(s), such as with least squares and/or linear regression methodology and/or correlation for finding a curve in the signal that correlates to a predetermined curve function of the desired shape (e.g., the "V" shape). Other methodologies may include filtering such as to detect peak-to-trough trends or amplitude trends, which may be indicative of sudden signal drop. In some implementations, the signal may be processed to track peaks and/or track troughs in an envelope of the signal for detection of such sudden depressions.

Such detected vasoconstriction-affected periods, in the PAT signal or in the PPG signal, such as when identified by the trough detection methodology, may be ignored by the system. Thus, corresponding periods of the PPGDR extracted from such vasoconstriction-affected zones may be omitted from the aforementioned sleep disordered breathing detection or differentiation processes (e.g., classification). For example, if a respiratory event included a vasoconstriction-affected zone, features (or values thereof) derived from that zone portion of the PPGDR signal may be omitted for the compilation of the aggregated feature set for the classification of the respiratory event.

*iv. Event rejection*

Another issue with PPGDR signal quality arises when motion or other artifacts impact the PPG quality or when severe cardiac arrhythmia are present. Thus, the system may be configured to recognize arrhythmia to improve quality of the sleep disordered breathing event detection and/or type distinction, such that the sleep disordered breathing event detection and/or type distinction is based on the arrhythmia event detection. For example, a arrhythmia events may be detected from a signal from the sensor by detection of a irregular periods in PPG and/or PAT signal that are indicative of a signal fluctuation of arrhythmia. For example, when the peak-to-peak intervals of the PPG are irregular, either due to artifacts or severe cardiac arrhythmia, the breathing modulations present in the PPG get overpowered by such irregularities and PPGDR extracted from these regions would show spurious oscillations unrelated to the true respiratory modulations.

To make sure that the PPGDR can be utilized for respiratory event type prediction, irregular PPG peak-to-peak interval zones may be detected, and any respiratory event(s) that has more than some percentage (e.g., 20%) of irregular PPG peak-to-peak intervals with regard to the total number of PPG peak-to-peak intervals of the respiratory event(s) may be rejected.

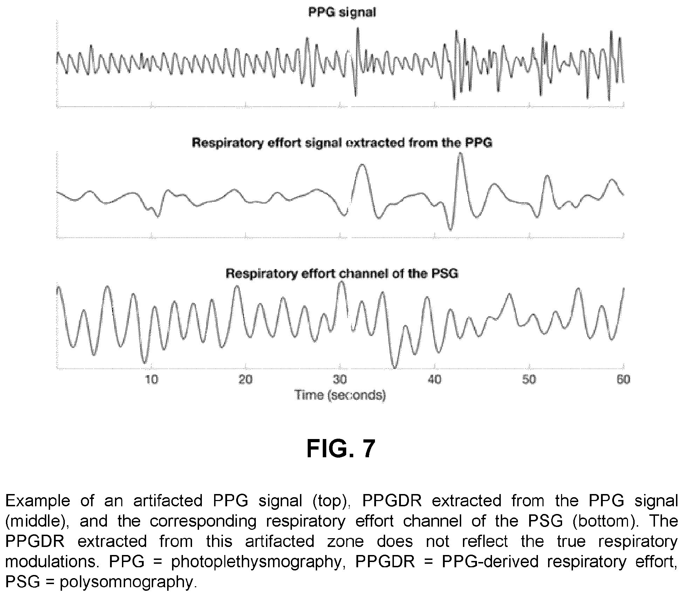

In some implementations, the system may identify irregular peak-to-peak intervals when the absolute value of the difference of two neighboring peak-to-peak intervals is more than some percentage (e.g., 20%) of the average of those values for other intervals of the PPG signal. Thus, in an example, a respiratory event may be rejected if more than 20% of its PPG peak-to-peak intervals of the respiratory event are irregular. Fig. 7 shows a segment of an example of such a rejected PPG signal and the corresponding PPGDR compared against the respiratory effort channel of the PSG. The proportion of rejected respiratory events for each recording may be understood to be an Event Rejection Proportion (ERP).

Other methodologies for detection of such irregular periods of the PPG signal depressions may also, or alternatively, be implemented for such arrythmia event detection. For example, rather than rely upon peaks (i.e., peak-to-peak) of the signal for the interval detection, the PPG signal may be processed to detect other characteristic points of the cardiac information in PPG signal such as on a point-to-corresponding-point basis for determining such intervals. For example, such a characteristic point may be a trough such that trough-to-trough intervals may be determined and applied for the irregular interval detection methodology previously described. Another example characteristic point may be the Dicrotic notch such that notch-to-notch intervals may be implemented in the methodology. In some versions, the detection of such intervals may be improved by pre-processing of the PPG signal for the detections. For example, the PPG signal may be filtered, such as by a band pass filtering or the like and the intervals, based on the characteristic points, may be detected from the preprocessed signal or filtered signal.

v. *Machine Respiratory Event Type Identification*

The aggregated set of features may then be applied to a machine algorithm as previously described, such as a machine learning algorithm or a trained classifier. An example of such an implementation may include an ensemble of trees classifier, neural network, support vector machine or other machine learning algorithm/model. The output of the classifier may then include type identifications that distinguish between central respiratory events and obstructive respiratory events, such as by generating suitable representative labels that designate the respiratory events as either central or obstructive. Such event labels may be applied by a system, such as for screening, monitoring, diagnosis and/or therapy. For example, the system may estimate or predict a central apnea hypopnea index (cAHI) by counting the number of central events as predicted by the computer model/classifier and dividing the count by a total sleep time (TST) estimate which may also be determined with the PAT sensing device. In some versions, if predictions are made only with non-rejected respiratory events, the estimated or predicted cAHI may be extrapolated by dividing it by one minus the ERP to account for the total number of respiratory events (rejected and non-rejected) of the patient. Thus, in the case of a screening application, the estimated or predicted cAHI may then be compared to a cutoff threshold to provide an indication of whether the user should receive further care (e.g., monitoring, further screening, or therapy) when the cAHI suggest that the user is experiencing significant central sleep apnea, which may require other intervention.

In the case of the training a computer model or classifier with patient data, the respiratory events, which may be inferred by detection of a flat zone in the PAT sensing device signal for the patients, may be labeled using expert analysis event type labels (central vs. obstructive). Such respiratory events identified by the expert review, or otherwise identified with a high level of certainty, can be applied as labeled events (Ground Truth Events) for training of any suitable classifier.

*vi.* Statistical Performance Analysis

A. The Dataset - An Example Verification

Aspects of the he aforementioned technology was implemented, and evaluated and validated in a study. The respiratory information embedded in the PPG data of a dataset was extracted and used to train an ensemble of trees classifiers that predicts the central or obstructive nature of each respiratory event. The classifier performance was evaluated using patient-wise leave-one-out cross-validation where an expert analysis of the PSG served as ground truth. A second, independent analysis of the PSG was also evaluated against the ground truth to allow benchmarking of the PPG-based method.

The dataset used in this study comprised 266 patients with suspicion of SA which were prospectively recruited across four different centers of which three were located in the USA (where all centers were part of the United Health Services Group in Miami, Florida) and one in Belgium (Ziekenhuis Oost Limburg, ZOL, Genk). The USA branch of the study was approved by Aspire IRB, part of the WIRB-Copernicus Group. The Belgian branch of the study was approved by the Ethics Committee of ZOL.

All patients were scheduled for one overnight in-lab PSG. The patient cohort was not stratified for CSA prevalence to retain the normal cohort prevalence of CSA. As the cohort matches the intended target population of the Study Device, this leads to the most representative prevalence-dependent performance endpoints such as Negative Predictive Values (NPV) and Positive Predictive Values (PPV). Qualified lab technicians at each participating study center were responsible for setting up the equipment and capturing PSG data. During setup of PSG, the PAT HSAT was attached to the middle finger of the hand to which the pulse oximeter of PSG was applied.

Patient Rejection

Predictions made for patients that have a substantial number of rejected events would be unreliable due to the small proportion of retained events used for cAHI estimation. Therefore, patients with an ERP higher than 0.3 were rejected and removed from further analysis.

All PSG data was double-scored by two independent centers which were blinded from one-another's analysis. The first scoring was performed by the team of sleep technicians of the center where the patient was admitted (Local Analysis). Another independent scoring was performed by scorers of Cerebra Medical (CM, Canada). The studies were first analyzed by their Michele Sleep Scoring System (MSSS) and were subsequently complemented with complete manual rescoring by an expert technologist. Malhotra et. al [25] confirmed in a multi-centric trial that the MSSS, complemented with manual editing by an expert scorer, is more robust than the results of a single scorer. Because of this conclusion, CM's analysis served as the expert benchmark (Expert Analysis) to which the Local Analysis and our PPG-based method were compared. All PSG data were scored according to the latest AASM scoring rules using the recommended 1A (3%) rule for hypopnea scoring [26].

In the model training of the study, each respiratory event inferred by the PAT HSAT was labeled using the Expert Analysis' event type labels (central vs. obstructive). Since for model training we only wanted to retain respiratory events that were identified with a comparatively high level of certainty, respiratory events identified by the Expert Analysis that were not also identified by Local Analysis were removed. The respiratory events for which there was consensus by the Expert and Local Analysis on their presence (but not necessarily on their type) were used to label PAT HSAT events (Ground Truth Events). The PAT HSAT events that did not overlap with or were within ten seconds after any Ground Truth Event were removed from the training data. This removal of Expert Analysis or PAT HSAT events was only performed for the training sets and not for model validation. Each Expert Analysis' annotation was used at most once for PAT HSAT event labeling.

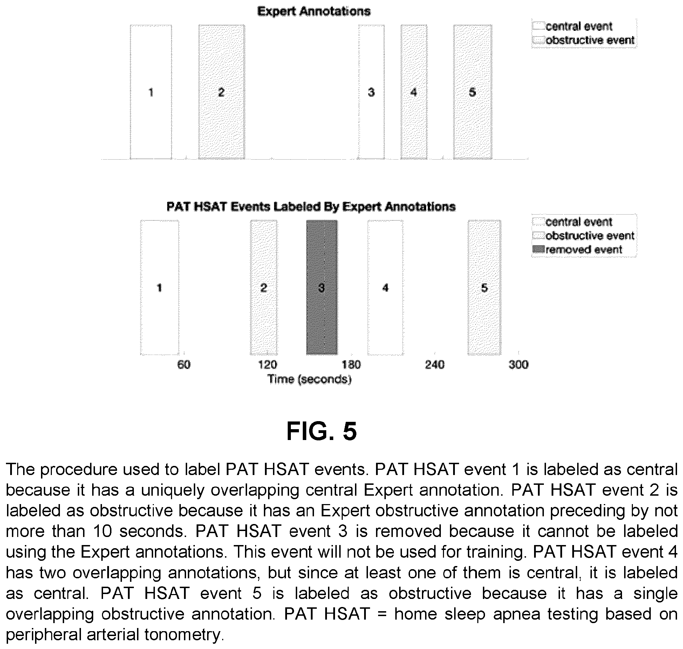

Fig. 5 illustrates the labeling procedure by means of examples. If a PAT HSAT event had a uniquely overlapping Expert Analysis annotation, the PAT HSAT event was labeled according to the corresponding Expert Analysis' event type. If there was more than one annotated event overlapping with a PAT HSAT event, the PAT event was labeled as central if any of the overlapping Expert Analysis' annotations were central events, otherwise the respiratory event was labeled as obstructive. Since the PAT HSAT respiratory event locations may be slightly shifted with regards to Expert Analysis' annotations, any preceding Expert Analysis' annotations of up to ten seconds were matched to a PAT HSAT event if there was no other annotation overlapping with the PAT HSAT event. Each Expert Analysis' annotation was used at most once for PAT HSAT event labeling. Out of a total of 44,420 PAT HSAT events, 26,208 had a corresponding Expert Analysis annotation and were labeled according to the above-described procedure. Out of the 26,208 labeled PAT HSAT events, 2,881 were central (or mixed) and 23,327 were obstructive.

Since an airflow channel is necessary to mark the exact start and end of a respiratory event, a PAT HSAT has an imperfect delimitation of respiratory events. As such, it is not readily possible to infer via a PAT HSAT whether an episode without breathing effort followed by an episode with breathing effort corresponds to a central event, that may encompass a mixed event. For that reason, the Expert Analysis labels were binarized into central and obstructive, considering mixed events as central. Therefore, the reported cAHI of the implemented method is an estimate of the sum of central and mixed respiratory events per hour of sleep.

*vii. cAHI prediction and evaluation*

Unlike for training, for evaluation purposes all 44,420 PAT HSAT events were retained regardless of the existence of a corresponding PSG annotation. To maximize utilization of the strongly imbalanced dataset caused by the low prevalence rate of CSA, training and evaluation were performed via a patient-wise leave-one-out cross-validation. For each patient, an ensemble of trees classifier (ensemble method: bagging, false positive cost: 2.5, number of trees: 30, max. number of splits: 150) was trained on the labeled data of the remaining patients. The false positive cost parameter of the classifier was used to tune the event-wise sensitivity and specificity of the predictions, used in this case to prevent over-detection of CSA. Using this classifier, predictions were made on the non-rejected PAT HSAT events of the considered patient. The predicted cAHI was calculated as the number of central events as predicted by the model, divided by the PAT HSAT Total Sleep Time (TST) estimate. Since predictions were made only with non-rejected events, the predicted cAHI was extrapolated by dividing it by one minus the ERP to account for the total number of respiratory events (rejected and non-rejected) of the patient.

Performance of the method was evaluated for cAHI cutoffs of 5, 10, and 15. The evaluation was performed on a patient-wise base by flagging a patient as central if the cAHI was above the given cutoff value.

*i. General*

Statistical analysis was performed using MATLAB (version 2020a, MathWorks, USA). Patient demographic information was obtained during the clinical study and is reported in Results. For the cAHI predictions, the Pearson correlation with the ground truth was computed. For each of the cAHI cutoffs, cohort-wise sensitivity, specificity, NPV, and PPV, accuracy, and Cohen's Kappa values were computed, and a Receiver Operating Characteristic (ROC) curve was generated. The ROC curve was generated by obtaining classifier probability scores for each respiratory event and varying the probability cutoff threshold for binary classification. For all sensitivity, specificity, PPV, and NPV, accuracy and Cohen's Kappa endpoint parameters, and 95% confidence intervals were computed. The classifier confusion matrix was constructed. The confusion matrix was constructed for four cAHI categories: cAHI < 5, $5 \leq$ cAHI < 10, $10 \leq$ cAHI < 15, and cAHI $\geq$ 15. Significance levels were determined for an alpha (p-value) of 0.05.

Where possible, the same analysis was performed for the comparison between the Local Analysis and the Expert Analysis.

*ii. Endpoint analysis*

Since there are no standardized performance targets established for cAHI prediction, the Local Analysis's performance against the Expert Analysis serves as a performance benchmark. In line with Pillar et al. [21], cAHI cutoffs of 10, and 15 were used for performance analysis. AHI cutoffs of 5 and 30 are also standard in literature, therefore performance analysis was also reported for the cAHI cutoff of 5.

As there were only 6 patients with a cAHI $\geq$ 30, no analysis was performed for this cutoff since the performance measures would be hard to generalize due to the small sample size.

The endpoints used for performance assessment were the sensitivity, specificity, NPV, PPV, accuracy, and Cohen's Kappa of the PPG-based method at the examined cAHI cutoff values.

II. Results

*A.* Patient Rejection

After removing patients with an ERP above the 0.3 cutoff from further analysis, 245 of the original 266 patients were retained. All statistical analyses and evaluations were performed on this final set of accepted patients.

*B.* Demographic Information

As listed in Table I, patients were predominantly male (60%), of middle age (mean 53.9 years, STD 13.7), and overweight (mean BMI 29.9 kg/m2 and STD 5.9). The mean AHI was 31.1 (STD 24.8).

24 patients had no clinical sleep apnea (AHI < 5), 56 patients had mild sleep apnea ($5 \leq$ AHI < 15), 64 patients had moderate sleep apnea ($15 \leq$ AHI < 30) and 101 patients had severe sleep apnea (AHI $\geq$ 30).

213 patients had cAHI < 5, 11 patients had $5 \leq$ cAHI < 10, 10 patients had $10 \leq$ cAHI < 15, 5 patients had $15 \leq$ cAHI < 30, and 6 patients had cAHI $\geq$ 30.

*C.* Cohort-wise Performance

Table II shows the sensitivity, specificity, NPV, PPV, accuracy, and Cohen's Kappa for three cAHI cutoffs for the PPG-based method against the Expert Analysis. The same is displayed for the Local Analysis against the Expert Analysis. For a cAHI cutoff of 10, the method achieved a sensitivity of 81%, a specificity of 99%, a PPV of 90%, an NPV of 98%, an accuracy of 97.6%, and a Cohen's Kappa of 0.84.

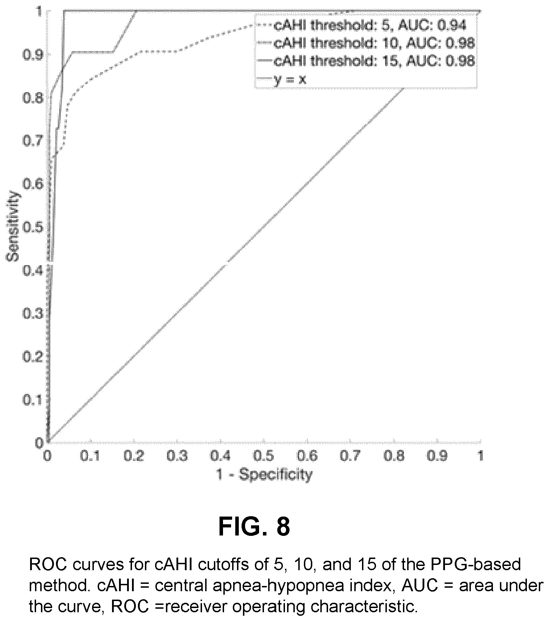

Fig. 8 shows the ROC curves for each of the cAHI cutoffs. The largest AUC of our method of 0.98 was found at a cAHI cutoff of 15.

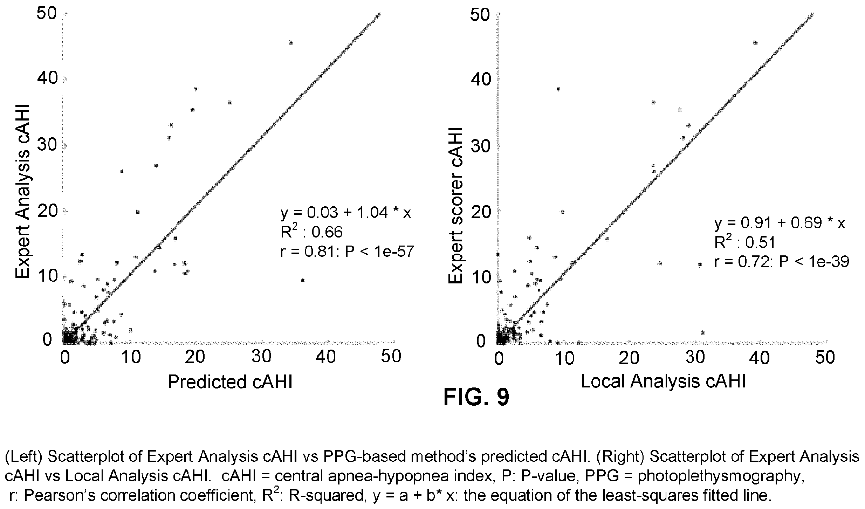

Fig. 9 shows scatterplots of the cAHI predicted by the PPG-based method and Local Analysis' cAHI against the Expert Analysis' cAHI. The Pearson correlation was 0.81 for PPG-based predictions (p-value < 0.001) and 0.72 for the Local Analysis (p-value < 0.001).

Table III shows the confusion matrix of PPG-based predictions for four cAHI intervals as well as the same analysis for the Local Analysis.

The 4-class accuracy for cAHI prediction derived from this matrix was 89.4% for PPG-based predictions and 89.0% for the Local Analysis. Cohen's Kappa was 0.573 for PPG-based predictions and 0.544 for the Local Analysis.

III. Discussion

*A.* General

The results indicate that the aforementioned example methodology allows for accurate cAHI prediction based on PPG data obtained by a finger probe and does so without the need for any additional sensor modalities such as a chest probe as described by Pillar et al. [21].

*B.* PAT HSAT Performance

The performance of the implementation was found to be comparable to that of the Local Analysis of the concurrently acquired PSG.

Another comparison can be made between the automated methodology and the PAT HSAT cAHI detection performance described by Pillar et al. [21] (note that as stated earlier this method combines PPG and chest accelerometry modalities). The study population described by Pillar et al. [21] was preselected for CSA. As such, PPV and NPV endpoints could not be compared due the large prevalence discrepancies between their study and ours.

The method described by Pillar et al. [21] achieved a sensitivity of 71.4% and specificity of 98.6% for cAHI cutoff 10. For cAHI cutoff 15, it achieved a sensitivity of 66.7% and a specificity of 100%. In comparison, the automated methodology's performance endpoints listed in Table II show an outperformance in the sum of sensitivity and specificity for both cAHI cutoffs.

*C.* Selecting the cAHI cutoff for CSA screening; clinical implications and tradeoffs

The choice of the most desirable cAHI cutoff value for CSA screening should take into consideration the tradeoff between NPV and PPV. Since patients flagged by the automated methodology can be referred to a full in-lab PSG, having a high PPV is important to avoid unnecessary follow-up examinations, whereas a high NPV is required to avoid false negative tests.

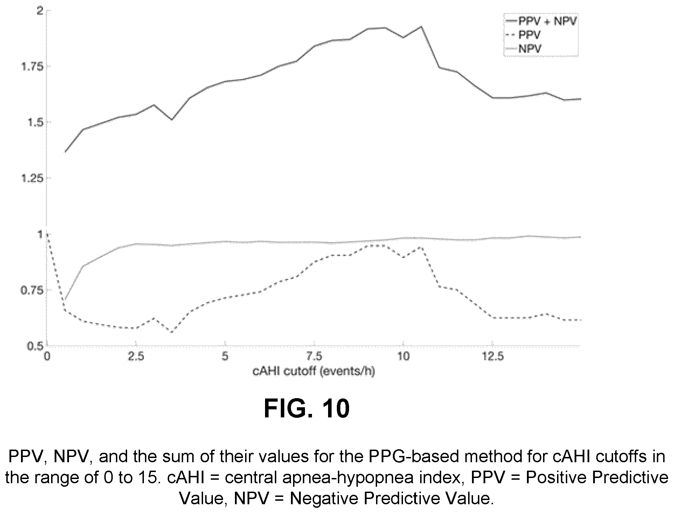

Fig. 10 shows the PPV, NPV, and their sum for varying cAHI cutoffs. For cAHI cutoffs between 2.5 and 15, the NPV remains relatively stable at around 95%, reaching a maximum of 99.1% at cAHI cutoff 13.5. The PPV increases from 57.8% at cAHI cutoff 2.5 to 94.7% at cAHI cutoff 9.5. At cutoff 10.5, it starts to decrease approximately monotonically. The cAHI cutoff corresponding to the highest sum of NPV and PPV of 19.3 was located at cAHI cutoff 10.5.

Another consideration when selecting the cAHI besides maximizing NPV and PPV, is the selection of the desired sensitivity for detecting plausible CSA predominance. Predominant CSA occurs when 50% or more of the events are central in nature. As such, if we wish to detect patients with predominant CSA with an AHI as low as 5, we need to place the cAHI cutoff at 2.5. If we only wish to detect patients with predominant CSA with an AHI of 15 or higher, we need to place the cAHI cutoff at 7.5. The most performant cAHI cutoff of approximately 10 is optimized to flag predominant CSA in patients with an AHI of 20 or higher. Considering the above, graph in Fig. 10 reveals the flexibility available to clinicians to select the cAHI cutoff based on this desired minimum AHI for which predominant CSA can be flagged. In the range of cAHI cutoffs between 2.5 and 10, a lower cAHI cutoff is generally associated with a lower PPV, and as such a higher rate of unnecessary follow-up examinations.

C. Conclusion

In sum, the study validated example aspects of the disclosed method of detection of CSA in SA patients using only the PPG data acquired at the finger. The method's performance endpoints at the examined Central Apnea-Hypopnea Index (cAHI) cutoff levels of 5 and 10 were higher or equal to the PSG benchmark and not significantly lower than the PSG benchmark for cAHI cutoff level 15. The method achieved a sensitivity of 81%, a specificity of 99%, a positive predictive value of 90%, and a negative predictive value of 98% at the cAHI cutoff of 10 events per hour of sleep. Thus, the PPG-based CSA detection method can, for example, be used to flag CSA in patients, which can enable referral to an in-lab PSG for further confirmation. The method can accurately flag a significant presence of CSA which in turn may aid in more optimal therapy decision making.

5.1 OPTIONAL EXAMPLE TREATMENT SYSTEMS

As previously mentioned, in one form, the present technology may include an apparatus or device for treating and/or monitoring a respiratory disorder, which may be involved in and/or configured for any of the aforementioned processes. The apparatus or device may be an RT or RPT device 4000 for supplying a flow of pressurised air to the patient 1000 via an air circuit 4170 leading to a patient interface 3000. The flow of air may be pressure-controlled (for respiratory pressure therapies) or flow-controlled (for flow therapies such as high flow therapy HFT). Thus, RPT devices may also be configured to act as flow therapy devices. In the following description, the RT or RPT device may be considered in reference to Figs. 7-10.

## 5.2 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, a connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to an airway of the patient so as to facilitate the supply of pressurised air to the airway.

## 5.3 RPT DEVICE

An RPT device 4000 in accordance with one aspect of the present technology comprises mechanical and pneumatic components 4100, electrical components 4200 and is programmed to execute one or more algorithms 4300. The RPT device 4000 may have an external housing 4010 formed in two parts, an upper portion 4012 and a lower portion 4014. In one form, the external housing 4010 may include one or more panel(s) 4015. The RPT device 4000 may comprise a chassis 4016 that supports one or more internal components of the RPT device 4000. The RPT device 4000 may include a handle 4018.

The pneumatic path of the RPT device 4000 may comprise one or more air path items, *e.g.*, an inlet air filter 4112, an inlet muffler 4122, a pressure generator 4140 capable of supplying pressurised air (*e.g.*, a blower 4142), an outlet muffler 4124, and one or more transducers 4270, such as pressure sensors 4272 and flow rate sensors 4274.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

### 5.3.1 RPT device mechanical & pneumatic components

An RPT device 4000 may comprise one or more of the following components in an integral unit. In an alternative form, one or more of the following components may be located as respective separate units.

#### 5.3.1.1 Air filter(s)

An RPT device 4000 in accordance with one form of the present technology may include an air filter 4110, or a plurality of air filters 4110.

In one form, an air inlet filter 4112 is located at the beginning of the pneumatic path upstream of a pressure generator 4140.

In one form, an air outlet filter 4114, for example an antibacterial filter, is located between an outlet of the pneumatic block 4020 and a patient interface 3000.

#### 5.3.1.2 Muffler(s)

An RPT device 4000 in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000.

#### 5.3.1.3 Pressure generator

In one form of the present technology, a pressure generator 4140 for supplying pressurised air is a controllable blower 4142. For example, the blower 4142 may include a brushless DC motor 4144 with one or more impellers housed in a volute. The pressure generator 4140 may be capable of generating a supply or flow of air, for example at about 120 litres/minute, at a positive pressure in a range from about 4 cmH$_2$O to about 20 cmH$_2$O, or in other forms up to about 30 cmH$_2$O.

The pressure generator 4140 is under the control of the therapy device controller 4240.

In other forms, a pressure generator 4140 may be a piston-driven pump, a pressure regulator connected to a high pressure source (e.g., compressed air reservoir), or a bellows.

### 5.3.1.4 Transducer(s)

Transducers may be internal of the RPT device, or external of the RPT device, such as a finger PPG sensor or PAT sensing device previously described. External transducers may be located for example on or form part of the air circuit, e.g., the patient interface. External transducers may be in the form of non-contact sensors such as a Doppler radar movement sensor that transmit or transfer data to the RPT device.

In one form of the present technology, one or more transducers 4270 are located upstream and / or downstream of the pressure generator 4140. The one or more transducers 4270 are constructed and arranged to generate data representing respective properties of the air flow, such as a flow rate, a pressure or a temperature, at that point in the pneumatic path.

In one form of the present technology, one or more transducers 4270 are located proximate to the patient interface 3000.

In one form, a signal from a transducer 4270 may be filtered, such as by low-pass, high-pass or band-pass filtering.

### 5.3.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.3.1.6 Air circuit

An air circuit 4170 in accordance with one aspect of the present technology is a conduit or tube constructed and arranged to allow, in use, a flow of air to travel between two components such as the pneumatic block 4020 and the patient interface 3000.

### 5.3.1.7 Oxygen delivery

In one form of the present technology, supplemental oxygen 4180 is delivered to one or more points in the pneumatic path, such as upstream of the pneumatic block 4020, to the air circuit 4170 and/or to the patient interface 3000.

### 5.3.2 RPT device electrical components

### 5.3.2.1 Power supply

In one form of the present technology power supply 4210 is internal of the external housing 4010 of the RPT device 4000. In another form of the present technology, power supply 4210 is external of the external housing 4010 of the RPT device 4000.

In one form of the present technology power supply 4210 provides electrical power to the RPT device 4000 only. In another form of the present technology, power supply 4210 provides electrical power to both RPT device 4000 and humidifier 5000.

### 5.3.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form the input device 4220 may be constructed and arranged to allow a person to select a value and/or a menu option.

### 5.3.2.3 Central controller

In one form of the present technology, the central controller 4230 is a processor suitable to control an RPT device 4000 such as an x86 INTEL processor.

A central controller 4230 suitable to control an RPT device 4000 in accordance with another form of the present technology includes a processor based on ARM Cortex-M processor from ARM Holdings. For example, an STM32 series microcontroller from ST MICROELECTRONICS may be used.

Another central controller 4230 suitable to control an RPT device 4000 in accordance with a further alternative form of the present technology includes a member selected from the family ARM9-based 32-bit RISC CPUs. For example, an STR9 series microcontroller from ST MICROELECTRONICS may be used.

In certain alternative forms of the present technology, a 16-bit RISC CPU may be used as the central controller 4230 for the RPT device 4000. For example a processor from the MSP430 family of microcontrollers, manufactured by TEXAS INSTRUMENTS, may be used.

In another form of the present technology, the central controller 4230 is a dedicated electronic circuit. In another form, the central controller 4230 is an application-specific integrated circuit (ASIC). In another form, the central controller 4230 comprises discrete electronic components.

The central controller 4230 is configured to receive input signal(s) from one or more transducers 4270, one or more input devices 4220, and the humidifier 5000.

The central controller 4230 is configured to provide output signal(s) to one or more of an output device 4290, a therapy device controller 4240, a data communication interface 4280, and the humidifier 5000.

In some forms of the present technology, the central controller 4230 is configured to implement the one or more methodologies described herein, such as the one or more algorithms 4300, expressed as computer programs stored in a non-transitory computer readable storage medium, such as memory 4260 or other memory described herein. In some forms of the present technology, as previously discussed, the central controller 4230 may be integrated with an RPT device 4000. However, in some forms of the present technology, some methodologies may be performed by a remotely located device or server such as the server previously mentioned. For example, the remotely located device or server may determine control settings for transfer to a ventilator or other RT device such as by detecting respiratory related events and distinguishing them by type by an analysis of stored data such as from any of the sensors described herein.

While the central controller 4230 may comprise a single controller interacting with various sensors 4270, data communications interface 4280, memory 4260, as well as other devices, the functions of controller 4230 may be distributed among more than one controller. Thus, the term "central" as used herein is not meant to limit the architecture to a single controller or processor that controls the other devices. For example, alternative architectures may include a distributed controller architecture involving more than one controller or processor, which may optionally be directly or indirectly in electronic (wired or wireless) communications with the previously described finger sensor or a server in communication with the finger sensor, such as for implementing any of the methodologies described herein. This may include, for example, a separate local (*i.e.*, within RPT device 4000) or remotely located controller that perform some of the algorithms 4300, or even more than one local or remote memory that stores some of the algorithms. In addition, the algorithms when expressed as computer programs may comprise high level human readable code (*e.g.*, C++, Visual Basic, other object oriented languages, etc.) or low/machine level instructions (Assembler, Verilog, etc.). Depending on the functionality of an algorithm(s), such code or instructions may be burnt in the controller, e.g., an ASIC or DSP, or be a run time executable ported to a DSP or general purpose processor that then becomes specifically programmed to perform the tasks required by the algorithm(s).

### 5.3.2.4 Clock

The RPT device 4000 may include a clock 4232 that is connected to the central controller 4230.

### 5.3.2.5 Therapy device controller

In one form of the present technology, therapy device controller 4240 is a therapy control module 4330 that forms part of the algorithms 4300 executed by the central controller 4230.

In one form of the present technology, therapy device controller 4240 is a dedicated motor control integrated circuit. For example, in one form a MC33035 brushless DC motor controller, manufactured by ONSEMI is used.

### 5.3.2.6 Protection circuits

An RPT device 4000 in accordance with the present technology may comprise one or more protection circuits 4250.

One form of protection circuit 4250 in accordance with the present technology is an electrical protection circuit.

One form of protection circuit 4250 in accordance with the present technology is a temperature or pressure safety circuit.

### 5.3.2.7 Memory

In accordance with one form of the present technology the RPT device 4000 includes memory 4260, for example non-volatile memory. In some forms, memory 4260 may include battery powered static RAM. In some forms, memory 4260 may include volatile RAM.

Memory 4260 may be located on PCBA 4202. Memory 4260 may be in the form of EEPROM, or NAND flash.

Additionally or alternatively, RPT device 4000 includes a removable form of memory 4260, for example a memory card made in accordance with the Secure Digital (SD) standard.

In one form of the present technology, the memory 4260, such as an of the memories previously described, acts as a non-transitory computer readable storage medium on which is stored computer program instructions expressing the one or more methodologies described herein, such as the one or more algorithms 4300.

### 5.3.2.8 Transducers

Transducers may be internal of the device 4000, or external of the RPT device 4000. External transducers may be located for example on or form part of the air delivery circuit 4170, *e.g.*, at the patient interface 3000. External transducers may be in the form of non-contact sensors such as a Doppler radar movement sensor that transmit or transfer data to the RPT device 4000.

#### 5.3.2.8.1 Flow rate

A flow rate transducer 4274 in accordance with the present technology may be based on a differential pressure transducer, for example, an SDP600 Series differential pressure transducer from SENSIRION. The differential pressure transducer is in fluid communication with the pneumatic circuit, with one of each of the pressure transducers connected to respective first and second points in a flow restricting element.

In one example, a signal representing total flow rate $Qt$ from the flow transducer 4274 is received by the central controller 4230.

#### 5.3.2.8.2 Pressure

A pressure transducer 4272 in accordance with the present technology is located in fluid communication with the pneumatic path. An example of a suitable pressure transducer 4272 is a sensor from the HONEYWELL ASDX series. An alternative suitable pressure transducer is a sensor from the NPA Series from GENERAL ELECTRIC.

In use, a signal from the pressure transducer 4272 is received by the central controller 4230. In one form, the signal from the pressure transducer 4272 is filtered prior to being received by the central controller 4230.

#### 5.3.2.8.3 Motor speed

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

### 5.3.2.9 Data communication systems

In one form of the present technology, a data communication interface 4280 is provided, and is connected to the central controller 4230. Data communication interface 4280 may be connectable to a remote external communication network 4282 and / or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor.

In one form, remote external communication network 4282 is the Internet. The data communication interface 4280 may use wired communication (*e.g.*, via Ethernet, or optical fibre) or a wireless protocol (*e.g.*, CDMA, GSM, LTE) to connect to the Internet.

In one form, local external communication network 4284 utilises one or more communication standards, such as Bluetooth, or a consumer infrared protocol and may optionally communicate with any of the sensors described herein.

In one form, remote external device 4286 is one or more computers, for example a cluster of networked computers and/or server as described herein. In one form, remote external device 4286 may be virtual computers, rather than physical computers. In either case, such a remote external device 4286 may be accessible to an appropriately authorised person such as a clinician.

The local external device 4288 may be a personal computer, mobile phone, tablet or remote control.

### 5.3.2.10 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

### *5.3.2.10.1 Display driver*

A display driver 4292 receives as an input the characters, symbols, or images intended for display on the display 4294, and converts them to commands that cause the display 4294 to display those characters, symbols, or images.

### *5.3.2.10.2 Display*

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

### 5.3.3 RPT device algorithms

### 5.3.3.1 Pre-processing module

A pre-processing module 4310 in accordance with the present technology receives, as an input, raw data from a transducer 4270, for example a flow rate sensor 4274 or a pressure sensor 4272, and performs one or more process steps to calculate one or more output values that will be used as an input to another module, for example a therapy engine module 4320.

In one form of the present technology, the output values include the interface or mask pressure *Pm,* the respiratory flow rate *Qr,* and the leak flow rate *Ql.*

In various forms of the present technology, the pre-processing module 4310 comprises one or more of the following algorithms: pressure compensation 4312, vent flow rate estimation 4314, leak flow rate estimation 4316, respiratory flow rate estimation 4317, ventilation determination 4311, target ventilation determination 4313, respiratory rate estimation 4318, and backup rate determination 4319.

### *5.3.3.1.1 Pressure compensation*

In one form of the present technology, a pressure compensation algorithm 4312 receives as an input a signal indicative of the pressure in the pneumatic path proximal to an outlet of the pneumatic block 4020. The pressure compensation algorithm 4312 estimates the pressure drop in the air circuit 4170 and provides as an output an estimated pressure, *Pm,* in the patient interface 3000.

### *5.3.3.1.2 Vent flow rate estimation*

In one form of the present technology, a vent flow rate estimation algorithm 4314 receives as an input an estimated pressure, *Pm,* in the patient interface 3000 and estimates a vent flow rate of air, *Qv,* from a vent 3400 in a patient interface 3000.

### 5.3.3.1.3 Leak flow rate estimation

In one form of the present technology, a leak flow rate estimation algorithm 4316 receives as an input a total flow rate $Qt$ and a vent flow rate $Qv$, and estimates a leak flow rate $Ql$. In one form, the leak flow rate estimation algorithm 4316 estimates the leak flow rate $Ql$ by calculating an average of the difference between the total flow rate and the vent flow rate $Qv$ over a period sufficiently long to include several breathing cycles, *e.g.*, about 10 seconds.

In one form, the leak flow estimation algorithm 4316 receives as an input a total flow rate $Qt$, a vent flow rate $Qv$, and an estimated pressure, $Pm$, in the patient interface 3000, and estimates a leak flow rate $Ql$ by calculating a leak conductance, and determining a leak flow rate $Ql$ to be a function of leak conductance and the pressure $Pm$. Leak conductance may be calculated as the quotient of low-pass filtered non-vent flow rate equal to the difference between total flow rate $Qt$ and vent flow rate $Qv$, and low-pass filtered square root of pressure $Pm$, where the low-pass filter time constant has a value sufficiently long to include several breathing cycles, *e.g.*, about 10 seconds. The leak flow rate $Ql$ may be estimated as the product of leak conductance and a function of pressure, $Pm$.

### 5.3.3.1.4 Respiratory flow rate estimation

In one form of the present technology, a respiratory flow rate estimation algorithm 4317 receives as an input a total flow rate, $Qt$, a vent flow rate, $Qv$, and a leak flow rate, $Ql$, and estimates a respiratory flow rate of air, $Qr$, to the patient, by subtracting the vent flow rate $Qv$ and the leak flow rate $Ql$ from the total flow rate $Qt$.

In other forms of the present technology, the respiratory flow estimation algorithm 4317 provides a value that acts as a proxy for the respiratory flow rate $Qr$. Possible proxies for respiratory flow rate include:

- Respiratory movement of the chest of the patient 1000
- Current drawn by the pressure generator 4140
- Motor speed of the pressure generator 4140
- Trans-thoracic impedance of the patient 1000

The respiratory flow rate proxy value may be provided by a transducer 4270 in the RPT device 4000, *e.g.*, the motor speed sensor 4276, or a sensor external to the RPT device 4000, such a respiratory movement sensor or a trans-thoracic impedance sensor.

### 5.3.3.1.5 Ventilation determination

In one form of the present technology, a ventilation determination algorithm 4311 receives an input a respiratory flow rate $Qr$, and determines a measure *Vent* indicative of current patient ventilation.

In some implementations, the ventilation determination algorithm 4311 determines a measure of ventilation *Vent* that is an estimate of actual patient ventilation.

In one such implementation, the measure of ventilation *Vent* is half the absolute value of respiratory flow, $Qr$, optionally filtered by low-pass filter such as a second order Bessel low-pass filter with a corner frequency of 0.11 Hz.

In one such implementation, the measure of ventilation *Vent* is an estimate of gross alveolar ventilation (*i.e.* non-anatomical-deadspace ventilation). This requires an estimate of anatomical deadspace. One can use the patient's height (or arm-span in cases of severe skeletal deformity) as a good predictor of anatomical deadspace. Gross alveolar ventilation is then equal to a measure of actual patient ventilation, *e.g.*, determined as above, less the product of the estimated anatomical deadspace and the estimated spontaneous respiratory rate $Rs$.

In other implementations, the ventilation determination algorithm 4311 determines a measure of ventilation *Vent* that is broadly proportional to actual patient ventilation. One such implementation estimates peak respiratory flow rate *Qpeak* over the inspiratory portion of the cycle. This and many other procedures involving sampling the respiratory flow rate *Or* produce measures which are broadly proportional to ventilation, provided the flow rate waveform shape does not vary very much (here, the shape of two breaths is taken to be similar when the flow rate waveforms of the breaths normalised in time and amplitude are similar). Some simple examples include the median positive respiratory flow rate, the median of the absolute value of respiratory flow rate, and the standard deviation of flow rate. Arbitrary linear combinations of arbitrary order statistics of the absolute value of respiratory flow rate using positive coefficients, and even some using both positive and negative coefficients, are approximately proportional to ventilation. Another example is the mean of the respiratory flow rate in the middle $K$ proportion (by time) of the inspiratory portion, where $0 < K < 1$. There is an arbitrarily large number of measures that are exactly proportional to ventilation if the flow rate waveform shape is constant.

In other forms, the ventilation determination algorithm 4311 determines a measure *Vent* of ventilation that is not based on respiratory flow rate $Qr$, but is a proxy for the current patient ventilation, such as oxygen saturation ($SaO_2$), or partial pressure of carbon dioxide ($PCO_2$), obtained from suitable sensors attached to the patient 1000.

### 5.3.3.1.6 Target ventilation determination

In one form of the present technology, a central controller 4230 takes as input the measure of current ventilation, *Vent,* and executes one or more target ventilation determination algorithms 4313 for the determination of a target value *Vtgt* for the measure of ventilation.

In some forms of the present technology, there is no target ventilation determination algorithm 4313, and the target ventilation *Vtgt* is predetermined, for example by hard-coding during configuration of the RPT device 4000 or by manual entry through the input device 4220.

In other forms of the present technology, such as adaptive servo-ventilation (ASV) therapy (described below), the target ventilation determination algorithm 4313 computes the target ventilation *Vtgt* from a value *Vtyp* indicative of the typical recent ventilation of the patient 1000.

In some forms of adaptive servo-ventilation therapy, the target ventilation *Vtgt* is computed as a high proportion of, but less than, the typical recent ventilation *Vtyp.* The high proportion in such forms may be in the range (80%, 100%), or (85%, 95%), or (87%, 92%).

In other forms of adaptive servo-ventilation therapy, the target ventilation *Vtgt* is computed as a slightly greater than unity multiple of the typical recent ventilation *Vtyp.*

The typical recent ventilation *Vtyp* is the value around which the distribution of the measure of current ventilation *Vent* over multiple time instants over some predetermined timescale tends to cluster, that is, a measure of the central tendency of the measure of current ventilation over recent history. In one implementation of the target ventilation determination algorithm 4313, the recent history is of the order of several minutes, but in any case should be longer than the timescale of Cheyne-Stokes waxing and waning cycles. The target ventilation determination algorithm 4313 may use any of the variety of well-known measures of central tendency to determine the typical recent ventilation *Vtyp* from the measure of current ventilation, *Vent.* One such measure is the output of a low-pass filter on the measure of current ventilation *Vent,* with time constant equal to one hundred seconds.

### 5.3.3.1.7 Respiratory rate estimation

In one form of the present technology, a respiratory rate estimation algorithm 4318 receives as an input a respiratory flow rate, *Qr,* to the patient 1000, and produces an estimate of the spontaneous respiratory rate *Rs* of the patient.

The respiratory rate estimation algorithm 4318 may estimate the spontaneous respiratory rate *Rs* over periods when the patient 1000 is breathing spontaneously, *i.e.,* when the RPT device 4000 is not delivering "backup breaths" (described below). In some forms of the present technology, the respiratory rate estimation algorithm 4318 estimates the respiratory rate over periods when servo-assistance (defined as pressure support minus minimum pressure support) is low, in one implementation less than 4 $cmH_2O$, as such periods are more likely to reflect spontaneous respiratory effort.

In some forms of the present technology, the respiratory rate estimation algorithm 4318 estimates the respiratory rate over periods of asleep breathing, since the respiratory rate during these periods may be substantially different from the respiratory rate during wake. Anxiety typically results in a higher respiratory rate than that prevailing during sleep. When patients focus on their own breathing process, their respiratory rates are typically lower than those during normal wakefulness or during sleep. Techniques such as described in Patent Application no. PCT/AU2010/000894, published as WO 2011/006199, may be used to identify periods of awake breathing from the respiratory flow rate, *Qr.*

In some forms of the present technology, the respiratory rate estimation algorithm 4318 estimates the spontaneous respiratory rate *Rs* as the reciprocal of one of a variety of well-known statistical measures of central tendency of breath duration *Ttot* during the period of interest. In such measures it is desirable to reject, or at least be robust to, outliers. One such measure, trimmed mean, in which the lower and upper K proportions of the sorted breath durations are discarded and the mean calculated on the remaining breath durations, is robust to outliers. For example, when K is 0.25, this amounts to discarding the upper and lower quartiles of breath duration *Ttot.* The median is another robust measure of central tendency, though this can occasionally give unsatisfactory results when the distribution is strongly bimodal. A simple mean may also be employed as a measure of central tendency, though it is sensitive to outliers. An initial interval filtering stage, in which contiguous time intervals corresponding to implausible respiratory rates (e.g., greater than 45 breaths/minute or less than 6 breaths/minute) are excluded as outliers from the mean calculation, may be employed. Other filtering mechanisms which may be used alone or in combination with interval filtering are to exclude any breaths that are not part of a sequence of N successive spontaneous breaths, where *N* is some small integer (*e.g.,* 3), and to exclude the early and late breaths of a sequence of successive spontaneous breaths, e.g., to exclude the first and last breaths of a sequence of four breaths. The rationale for the latter mechanism is that the first and the last breaths in particular, and the early and late breaths in general, of a sequence of spontaneous breaths may be atypical; for example, the first spontaneous breath may occur as a result of an arousal, and the last spontaneous breath may be longer because of the decreasing respiratory drive which results in the backup breath which ends the sequence of spontaneous breaths.

In some forms of the present technology, the respiratory rate estimation algorithm 4318 makes an initial estimate of the spontaneous respiratory rate $Rs$ using an initial period of estimation, to enable the subsequent processing in the therapy engine module 4320 to begin, and then continuously updates the estimate of the spontaneous respiratory rate $Rs$ using a period of estimation that is longer than the initial period of estimation, to improve statistical robustness. For example, the initial period of estimation may be 20 minutes of suitable spontaneous breaths, but the period of estimation may then progressively increase up to some maximum duration, for example 8 hours. Rather than a rolling window of this duration being used for this estimation, low-pass filters on breath duration may be used, with progressively longer response times (more precisely, progressively lower corner frequencies) as the session proceeds.

In some forms, a suitably processed short-term (e.g., 10-minute) measure of central tendency, such as trimmed mean, may be input to a suitable low-pass filter to give an estimate $Rs$ which changes on the time scale of hours or longer. This has the advantage that potentially large amounts of breath duration data do not need to be stored and processed, as might occur if a trimmed mean needs to be calculated on a moving window of breath duration data lasting hours or days.

In some forms of the present technology, respiratory rates measured over short periods of time, and in particular over one breath, may also be used instead of breath duration in the above-described measures of central tendency, giving generally similar but not identical results.

### *5.3.3.1.8 Backup rate determination*

In one form of the present technology, a backup rate determination algorithm 4319 receives as input a spontaneous respiratory rate estimate $Rs$ provided by the respiratory rate estimation algorithm 4318 and returns a "backup rate" $Rb$. The backup rate $Rb$ is the rate at which the RPT device 4000 will deliver backup breaths, *i.e.,* continue to provide ventilatory support, to a patient 1000 in the absence of significant spontaneous respiratory effort.

In one form of the pre-processing module 4310, there is no backup rate determination algorithm 4319, and the backup rate $Rb$ is instead provided manually to the RPT device 4000, *e.g.,* via the input device 4220, or hard-coded at the time of configuration of the RPT device 4000.

In one form, known as adaptive backup rate, the backup rate determination algorithm 4319 determines the backup rate $Rb$ as a function of the spontaneous respiratory rate $Rs$. In one implementation, the function determines the backup rate $Rb$ as the spontaneous respiratory rate $Rs$ minus a constant such as 2 breaths per minute. In another implementation, the function determines the backup rate $Rb$ as the spontaneous respiratory rate $Rs$ multiplied by a constant that is slightly less than unity.

In one form, known as variable backup rate, the backup rate determination algorithm 4319 determines the backup rate $Rb$ as a function of time. The backup rate $Rb$ is initialised to a value known as the spontaneous backup rate (SBR) that is some fraction of a final target backup rate, known as the sustained timed backup rate (STBR). The fraction may be two thirds, or three quarters, or other positive values less than one. The SBR is the reciprocal of the timeout period to a backup breath when the most recent inspiration was a spontaneous (*i.e.,* patent-triggered) breath. The STBR may be predetermined (e.g., by manual entry or hard-coding as described above) or set to some typical respiratory rate such as 15 bpm. Over time elapsed since the previous spontaneous breath, the backup rate $Rb$ is increased from the SBR towards the STBR. The increase may be according to a predetermined profile, such as a series of steps, or a continuous linear profile. The profile is chosen such that the backup rate $Rb$ reaches the STBR after a predetermined interval. The interval may be measured in units of time, such as 30 seconds, or relative to the patient's respiration, such as 5 breaths.

In some forms of variable backup rate, the predetermined interval over which the backup rate $Rb$ increases from the SBR towards the STBR may be a function of the adequacy of current ventilation. In one implementation, suitable for servo-ventilation in which a target value $Vtgt$ exists for the measure of ventilation, the backup rate approaches the STBR faster to the extent that current measure of ventilation $Vent$ is less than the target ventilation $Vtgt$.

In one form of variable backup rate, known as adaptive variable backup rate, the backup rate determination algorithm 4319 determines the backup rate $Rb$ as a function of the current estimated spontaneous respiratory rate $Rs$ provided by the respiratory rate estimation algorithm 4318, as well as a function of time. As in variable backup rate determination, adaptive variable backup rate determination increases the backup rate $Rb$ from the SBR towards the STBR over a predetermined interval that may be a function of the adequacy of current ventilation. The STBR may be initialised to a standard respiratory rate, such as 15 bpm. Once a reliable estimate of spontaneous respiratory rate $Rs$ is available from the respiratory rate estimation algorithm 4318, the STBR may be set to the current estimated spontaneous respiratory rate $Rs$ multiplied by some constant. The SBR may be set to some fraction of the STBR, as in variable backup rate. In one form, the fraction, for example two thirds, can be set to a lower value, such as 0.55, during the initial period of estimation of the spontaneous respiratory rate $Rs$, to accommodate occasional long breath durations in patients with relatively low respiratory rates, such as 12 breaths per minute.

In some forms, the constant by which the current estimated spontaneous respiratory rate $Rs$ is multiplied to obtain the STBR may be slightly higher than 1, *e.g.,* 1.1, to provide more aggressive ventilation during apneas, which may be desirable in short apneas. The constant may be somewhat lower than 1, *e.g.,* 0.8, particularly if difficulty in resynchronisation with the patient on the return of patient effort turns out to be a problem in a particular patient. Lower backup rates make resynchronisation easier, by lengthening the expiratory pause, during which resynchronisation commonly occurs.

### 5.3.3.2 Therapy Engine Module

In one form of the present technology, a therapy engine module 4320 receives as inputs one or more of a pressure, $Pm$, in a patient interface 3000, a respiratory flow rate of air to a patient, $Qr$, and an estimate $Rs$ of the spontaneous respiratory rate, and provides as an output one or more therapy parameters. In various forms, the therapy engine module 4320 comprises one or more of the following algorithms: phase determination 4321, waveform determination 4322, inspiratory flow limitation determination 4324, apnea / hypopnea determination 4325, snore detection 4326, airway patency determination 4327, and therapy parameter determination 4329, such as one including a central vs. obstructive type determination as previously described.

#### 5.3.3.2.1 Phase determination

In one form of the present technology, a phase determination algorithm 4321 receives as an input a signal indicative of respiratory flow, $Qr$, and provides as an output a phase $\Phi$ of a current breathing cycle of a patient 1000.
In some forms, known as discrete phase determination, the phase output $\Phi$ is a discrete variable. One implementation of discrete phase determination provides a bi-valued phase output $\Phi$ with values of either inhalation or exhalation, for example represented as values of 0 and 0.5 revolutions respectively, upon detecting the start of spontaneous inhalation and exhalation respectively. RPT devices 4000 that "trigger" and "cycle" effectively perform discrete phase determination, since the trigger and cycle points are the instants at which the phase changes from exhalation to inhalation and from inhalation to exhalation, respectively. In one implementation of bi-valued phase determination, the phase output $\Phi$ is determined to have a discrete value of 0 (thereby "triggering" the RPT device 4000) when the respiratory flow rate $Qr$ has a value that exceeds a positive threshold, and a discrete value of 0.5 revolutions (thereby "cycling" the RPT device 4000) when a respiratory flow rate $Qr$ has a value that is more negative than a negative threshold.
Another implementation of discrete phase determination provides a tri-valued phase output $\Phi$ with a value of one of inhalation, mid-inspiratory pause, and exhalation.
In other forms, known as continuous phase determination, the phase output $\Phi$ is a continuous value, for example varying from 0 to 1 revolutions, or 0 to 27 radians. RPT devices 4000 that perform continuous phase determination may trigger and cycle when the continuous phase reaches 0 and 0.5 revolutions, respectively. In one implementation of continuous phase determination, a continuous value of phase $\Phi$ is determined using a fuzzy logic analysis of the respiratory flow rate $Qr$. A continuous value of phase determined in this implementation is often referred to as "fuzzy phase". In one implementation of a fuzzy phase determination algorithm 4321, the following rules are applied to the respiratory flow rate $Qr$:

1. If the respiratory flow rate is zero and increasing fast then the phase is 0 revolutions.

2. If the respiratory flow rate is large positive and steady then the phase is 0.25 revolutions.

3. If the respiratory flow rate is zero and falling fast, then the phase is 0.5 revolutions.

4. If the respiratory flow rate is large negative and steady then the phase is 0.75 revolutions.

5. If the respiratory flow rate is zero and steady and the 5-second low-pass filtered absolute value of the respiratory flow rate is large then the phase is 0.9 revolutions.

6. If the respiratory flow rate is positive and the phase is expiratory, then the phase is 0 revolutions.

7. If the respiratory flow rate is negative and the phase is inspiratory, then the phase is 0.5 revolutions.

8. If the 5-second low-pass filtered absolute value of the respiratory flow rate is large, the phase is increasing at a steady rate equal to the patient's respiratory rate, low-pass filtered with a time constant of 20 seconds.

The output of each rule may be represented as a vector whose phase is the result of the rule and whose magnitude is the fuzzy extent to which the rule is true. The fuzzy extent to which the respiratory flow rate is "large", "steady", etc. is determined with suitable membership functions. The results of the rules, represented as vectors, are then combined by some function such as taking the centroid. In such a combination, the rules may be equally weighted, or differently weighted.

In another implementation of continuous phase determination, the inhalation time $Ti$ and the exhalation time $Te$ are first estimated from the respiratory flow rate $Qr$. The phase $\Phi$ is then determined as the half the proportion of the inhalation time $Ti$ that has elapsed since the previous trigger instant, or 0.5 revolutions plus half the proportion of the exhalation time $Te$ that has elapsed since the previous cycle instant (whichever was more recent).

In some forms of the present technology, suitable for pressure support ventilation therapy (described below), the phase determination algorithm 4321 is configured to trigger even when the respiratory flow rate $Qr$ is insignificant, such as during an apnea. As a result, the RPT device 4000 delivers "backup breaths" in the absence of spontaneous respiratory effort from the patient 1000. For such forms, known as spontaneous / timed (S / T) modes, the phase determination algorithm 4321 may make use of the backup rate $Rb$ provided by the backup rate determination algorithm 4319.

A phase determination algorithm 4321 that uses "fuzzy phase" may implement S / T mode using the backup rate $Rb$ by including a "momentum" rule in the fuzzy phase rules. The effect of the momentum rule is to carry the continuous phase forward from exhalation to inhalation at the backup rate $Rb$ if there are no features of respiratory flow rate $Qr$ that would otherwise carry the continuous phase forward through the other rules. In one implementation, the more it is true that the measure of ventilation $Vent$ (described below) is well below a target value $Vtgt$ for ventilation (also described below), the more highly the momentum rule is weighted in the combination. However, as a result of the rapid increase in pressure support in response to mild to moderate hypoventilation (with respect to the target ventilation), the ventilation may be quite close to the target ventilation. It is desirable that the momentum rule is given a low weighting when the ventilation is close to target, to allow the patient to breathe at rates significantly lower than the respiratory rate at other times (when the patient is not in a central apnea) without being unnecessarily pushed to breathe at a higher rate by the ventilator. However, when the momentum rule is given a low weighting when ventilation is above a value which is below but close to the target ventilation, adequate ventilation may easily be achieved at a relatively high pressure support at a rate well below the backup rate. It would be desirable for the backup breaths to be delivered at a higher rate, because this would enable the target ventilation to be delivered at a lower pressure support. This is desirable for a number of reasons, a key one of which is to diminish mask leak.

To summarise, in a fuzzy phase determination algorithm 4321 that implements S / T mode, there is a dilemma in choosing the weighting for the momentum rule incorporating the backup rate $Rb$: if it is too high, the patient may feel "pushed along" by the backup rate. If it is too low, the pressure support may be excessive. Hence it is desirable to provide methods of implementing S / T mode which do not rely on the momentum rule described above.

A phase determination algorithm 4321 (either discrete, or continuous without a momentum rule) may implement S / T mode using the backup rate $Rb$ in a manner known as timed backup. Timed backup may be implemented as follows: the phase determination algorithm 4321 attempts to detect the start of inhalation due to spontaneous respiratory effort, for example by monitoring the respiratory flow rate $Qr$ as described above. If the start of inhalation due to spontaneous respiratory effort is not detected within a period of time after the last trigger instant whose duration is equal to the reciprocal of the backup rate $Rb$ (an interval known as the backup timing threshold), the phase determination algorithm 4321 sets the phase output $\Phi$ to a value of inhalation (thereby triggering the RPT device 4000). Once the RPT device 4000 is triggered, and a backup breath begins to be delivered, the phase determination algorithm 4321 attempts to detect the start of spontaneous exhalation, for example by monitoring the respiratory flow rate $Qr$, upon which the phase output $\Phi$ is set to a value of exhalation (thereby cycling the RPT device 4000).

If the backup rate $Rb$ is increased over time from the SBR to the STBR, as in a variable backup rate system described above, the backup timing threshold starts out longer and gradually becomes shorter. That is, the RPT device 4000 starts out less vigilant and gradually becomes more vigilant to lack of spontaneous respiratory effort as more backup breaths are delivered. Such an RPT device 4000 is less likely to make a patient feel "pushed along" if they would prefer to breathe at a lower than standard rate, while still delivering backup breaths when they are needed.

If the STBR in a variable backup rate system adapts to the patient's estimated spontaneous respiratory rate $Rs$, as in an adaptive variable backup rate system described above, the backup breaths will be delivered at a rate that adapts to the patient's own recent spontaneous respiratory efforts.

*5.3.3.2.2 Waveform determination*

In one form of the present technology, the therapy control module 4330 controls a pressure generator 4140 to provide a treatment pressure $Pt$ that varies as a function of phase $\Phi$ of a breathing cycle of a patient according to a waveform template $\Pi(\Phi)$.

In one form of the present technology, a waveform determination algorithm 4322 provides a waveform template $\Pi(\Phi)$ with values in the range [0, 1] on the domain of phase values $\Phi$ provided by the phase determination algorithm 4321 to be used by the therapy parameter determination algorithm 4329.

In one form, suitable for either discrete or continuously-valued phase, the waveform template $\Pi(\Phi)$ is a square-

wave template, having a value of 1 for values of phase up to and including 0.5 revolutions, and a value of 0 for values of phase above 0.5 revolutions. In one form, suitable for continuously-valued phase, the waveform template $\Pi(\Phi)$ comprises two smoothly curved portions, namely a smoothly curved (e.g., raised cosine) rise from 0 to 1 for values of phase up to 0.5 revolutions, and a smoothly curved (e.g., exponential) decay from 1 to 0 for values of phase above 0.5 revolutions. One example of such a "smooth and comfortable" waveform template is the "shark fin" waveform template, in which the rise is a raised cosine, and the smooth decay is quasi-exponential (so that the limit of $\Pi$ as $\Phi$ approaches one revolution is precisely zero).

In some forms of the present technology, the waveform determination algorithm 4322 selects a waveform template $\Pi(\Phi)$ from a library of waveform templates, dependent on a setting of the RPT device 4000. Each waveform template $\Pi(\Phi)$ in the library may be provided as a lookup table of values $\Pi$ against phase values $\Phi$. In other forms, the waveform determination algorithm 4322 computes a waveform template $\Pi(\Phi)$ "on the fly" using a predetermined functional form, possibly parametrised by one or more parameters (e.g., time constant of an exponentially curved portion). The parameters of the functional form may be predetermined or dependent on a current state of the patient 1000.

In some forms of the present technology, suitable for discrete bi-valued phase of either inhalation ($\Phi = 0$ revolutions) or exhalation ($\Phi = 0.5$ revolutions), the waveform determination algorithm 4322 computes a waveform template $\Pi$ "on the fly" as a function of both discrete phase $\Phi$ and time $t$ measured since the most recent trigger instant (transition from exhalation to inhalation). In one such form, the waveform determination algorithm 4322 computes the waveform template $\Pi(\Phi, t)$ in two portions (inspiratory and expiratory) as follows:

$$\Pi\left(\Phi,t\right) = \begin{cases} \Pi_i\left(t\right), & \Phi = 0 \\ \Pi_e\left(t - T_i\right), & \Phi = 0.5 \end{cases}$$

where $\Pi_i(t)$ and $\Pi_e(t)$ are inspiratory and expiratory portions of the waveform template $\Pi(\Phi, t)$, and *Ti* is the inhalation time. In one such form, the inspiratory portion $\Pi_i(t)$ of the waveform template is a smooth rise from 0 to 1 parametrised by a rise time, and the expiratory portion $\Pi_e(t)$ of the waveform template is a smooth fall from 1 to 0 parametrised by a fall time.

### 5.3.3.2.3 Determination of inspiratory flow limitation

In one form of the present technology, a processor executes one or more algorithms 4324 for the detection of inspiratory flow limitation (partial obstruction).

In one form the algorithm 4324 receives as an input a respiratory flow rate signal *Qr* and provides as an output a metric of the extent to which the inspiratory portion of the breath exhibits inspiratory flow limitation.

In one form of the present technology, the inspiratory portion of each breath is identified based on the phase $\Phi$ estimated at each instant. For example, the inspiratory portion of the breath is the values of respiratory flow for which the phase $\Phi$ is less than or equal to 0.5. A number of evenly spaced points (for example, sixty-five), representing points in time, are interpolated by an interpolator along the inspiratory flow-time curve for each breath. The curve described by the points is then scaled by a scaler to have unity length (duration/period) and unity area to remove the effects of changing respiratory rate and depth. The scaled breaths are then compared in a comparator with a pre-stored template representing a normal unobstructed breath. Breaths deviating by more than a specified threshold (typically 1 scaled unit) at any time during the inspiration from this template, such as those due to coughs, sighs, swallows and hiccups, as determined by a test element, are rejected. For non-rejected data, a moving average of the first such scaled point is calculated by central controller 4230 for the preceding several inspiratory events. This is repeated over the same inspiratory events for the second such point, and so on. Thus, for example, sixty five scaled data points are generated by central controller 4230, and represent a moving average of the preceding several inspiratory events, e.g., three events. The moving average of continuously updated values of the (e.g., sixty five) points are hereinafter called the "scaled flow", designated as *Qs(t)*. Alternatively, a single inspiratory event can be utilised rather than a moving average.

From the scaled flow, two shape factors relating to the determination of partial obstruction may be calculated.

Shape factor 1 is the ratio of the mean of the middle (e.g., thirty-two) scaled flow points to the mean overall (e.g., sixty-five) scaled flow points. Where this ratio is in excess of unity, the breath will be taken to be normal. Where the ratio is unity or less, the breath will be taken to be obstructed. A ratio of about 1.17 is taken as a threshold between partially obstructed and unobstructed breathing, and equates to a degree of obstruction that would permit maintenance of adequate oxygenation in a typical user.

Shape factor 2 is calculated as the RMS deviation from unit scaled flow, taken over the middle (e.g., thirty two) points. An RMS deviation of about 0.2 units is taken to be normal. An RMS deviation of zero is taken to be a totally flow-limited breath. The closer the RMS deviation to zero, the breath will be taken to be more flow limited.

Shape factors 1 and 2 may be used as alternatives, or in combination. In other forms of the present technology, the number of sampled points, breaths and middle points may differ from those described above. Furthermore, the threshold values can other than those described.

### 5.3.3.2.4 Determination of apneas and hypopneas

In one form of the present technology, a central controller 4230 executes one or more algorithms 4325 for the detection of apneas and/or hypopneas.

In one form, the one or more apnea / hypopnea detection algorithms 4325 receive as an input a respiratory flow rate $Qr$ and provide as an output a flag that indicates that an apnea or a hypopnea has been detected.

In one form, an apnea will be said to have been detected when a function of respiratory flow rate $Qr$ falls below a flow threshold for a predetermined period of time. The function may determine a peak flow, a relatively short-term mean flow, or a flow intermediate of relatively short-term mean and peak flow, for example an RMS flow. The flow threshold may be a relatively long-term measure of flow.

In one form, a hypopnea will be said to have been detected when a function of respiratory flow rate $Qr$ falls below a second flow threshold for a predetermined period of time. The function may determine a peak flow, a relatively short-term mean flow, or a flow intermediate of relatively short-term mean and peak flow, for example an RMS flow. The second flow threshold may be a relatively long-term measure of flow. The second flow threshold is greater than the flow threshold used to detect apneas.

In one form, such respiratory events may be characterized as central or obstructive based at least in part on the aforementioned finger sensor PPG based type detection.

### 5.3.3.2.5 Detection of snore

In one form of the present technology, a central controller 4230 executes one or more snore detection algorithms 4326 for the detection of snore.

In one form, the snore detection algorithm 4326 receives as an input a respiratory flow rate signal $Qr$ and provides as an output a metric of the extent to which snoring is present.

The snore detection algorithm 4326 may comprise a step of determining the intensity of the flow rate signal in the range of 30-300 Hz. The snore detection algorithm 4326 may further comprises a step of filtering the respiratory flow rate signal $Qr$ to reduce background noise, *e.g.,* the sound of airflow in the system from the blower 4142.

### 5.3.3.2.6 Determination of airway patency

In one form of the present technology, a central controller 4230 executes one or more algorithms 4327 for the determination of airway patency.

In one form, airway patency algorithm 4327 receives as an input a respiratory flow rate signal $Qr$, and determines the power of the signal in the frequency range of about 0.75Hz and about 3Hz. The presence of a peak in this frequency range is taken to indicate an open airway. The absence of a peak is taken to be an indication of a closed airway.

In one form, the frequency range within which the peak is sought is the frequency of a small forced oscillation in the treatment pressure $Pt$. In one implementation, the forced oscillation is of frequency 2 Hz with amplitude about 1 cmH$_2$O.

In one form, airway patency algorithm 4327 receives as an input a respiratory flow rate signal $Qr$, and determines the presence or absence of a cardiogenic signal. The absence of a cardiogenic signal is taken to be an indication of a closed airway.

### 5.3.3.2.7 Determination of therapy parameters

In some forms of the present technology, the central controller 4230 executes one or more therapy parameter determination algorithms 4329 for the determination of one or more therapy parameters using the values returned by one or more of the other algorithms in the therapy engine module 4320.

In one form of the present technology, the therapy parameter is an instantaneous treatment pressure $Pt$. In one implementation of this form, the therapy parameter determination algorithm 4329 determines the treatment pressure $Pt$ using the equation

$$Pt = A\Pi(\Phi) + P_0 \tag{1}$$

where:

- *A* is an amplitude,
- Φ is the current value of phase;
- Π(Φ) is the waveform template value (in the range 0 to 1) at the current value of phase, and
- $P_0$ is a base pressure.

If the waveform determination algorithm 4322 provides the waveform template Π(Φ) as a lookup table of values indexed by phase Φ, the therapy parameter determination algorithm 4329 applies equation (1) by locating the nearest lookup table entry to the current value Φ of phase returned by the phase determination algorithm 4321, or by interpolation between the two entries straddling the current value Φ of phase.

The values of the amplitude A and the base pressure $P_0$ may be set by the therapy parameter determination algorithm 4329 depending on the chosen pressure therapy mode in the manner described below.

### 5.3.3.3 Therapy control module

The therapy control module 4330 in accordance with one aspect of the present technology receives as inputs the therapy parameters from the therapy parameter determination algorithm 4329 of the therapy engine module 4320, and controls the pressure generator 4140 to deliver a flow of air in accordance with the therapy parameters.

In one form of the present technology, the therapy parameter is a treatment pressure *Pt,* and the therapy control module 4330 controls the pressure generator 4140 to deliver a flow of gas whose mask pressure *Pm* at the patient interface 3000 is equal to the treatment pressure *Pt.*

### 5.3.3.4 Detection of fault conditions

In one form of the present technology, a processor executes one or more methods 4340 for the detection of fault conditions. The fault conditions detected by the one or more methods may include at least one of the following:

- Power failure (no power, or insufficient power)
- Transducer fault detection
- Failure to detect the presence of a component
- Operating parameters outside recommended ranges (e.g., pressure, flow, temperature, $PaO_2$)
- Failure of a test alarm to generate a detectable alarm signal.

Upon detection of the fault condition, the corresponding algorithm signals the presence of the fault by one or more of the following:

- Initiation of an audible, visual &/or kinetic (e.g., vibrating) alarm
- Sending a message to an external device
- Logging of the incident

5.4 HUMIDIFIER

In one form of the present technology there is provided a humidifier 5000 *(e.g.,* as shown in Fig. 10) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

5.5 GLOSSARY

For the purposes of the present disclosure, in certain forms of the present technology, one or more of the following definitions may apply. In other forms of the present technology, alternative definitions may apply.

### 5.5.1 General

*Air:* In certain forms of the present technology, air may be taken to mean atmospheric air, and in other forms of the present technology air may be taken to mean some other combination of breathable gases, e.g., atmospheric air enriched with oxygen.

*Respiratory Pressure Therapy (RPT):* The delivery of a supply of air to the airways at a treatment pressure that is typically positive with respect to atmosphere.

*Continuous Positive Airway Pressure (CPAP) therapy:* Respiratory pressure therapy in which the treatment pressure is approximately constant through a breathing cycle of a patient. In some forms, the pressure at the entrance to the airways will be slightly higher during exhalation, and slightly lower during inhalation. In some forms, the pressure will vary between different breathing cycles of the patient, for example, being increased in response to detection of indications of partial upper airway obstruction, and decreased in the absence of indications of partial upper airway obstruction.

*Patient:* A person, whether or not they are suffering from a respiratory disease.

*Automatic Positive Airway Pressure (APAP) therapy:* CPAP therapy in which the treatment pressure is automatically adjustable, e.g., from breath to breath, between minimum and maximum limits, depending on the presence or absence of indications of SDB events.

### 5.5.2 Aspects of the breathing cycle

*Apnea:* According to some definitions, an apnea is said to have occurred when respiratory flow rate falls below a predetermined threshold for a duration, e.g., 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort.

*Breathing rate, or respiratory rate (Rs):* The rate of spontaneous respiration of a patient, usually measured in breaths per minute.

*Duty cycle:* The ratio of inhalation time, *Ti* to total breath duration, *Ttot.*

*Effort (breathing):* The work done by a spontaneously breathing person attempting to breathe.

*Expiratory portion of a breathing cycle:* The period from the start of expiratory flow to the start of inspiratory flow.

*Flow limitation:* The state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

*Hypopnea:* A reduction in flow, but not a cessation of flow. In one form, a hypopnea may be said to have occurred when there is a reduction in flow below a threshold for a duration. In one form in adults, the following either of the following may be regarded as being hypopneas:

(i) a 30% reduction in patient breathing for at least 10 seconds plus an associated 4% desaturation; or

(ii) a reduction in patient breathing (but less than 50%) for at least 10 seconds, with an associated desaturation of at least 3% or an arousal.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. *A patent* airway is open. Airway patency may be quantified, for example with a value of one (1) being patent, and a value of zero (0), being closed.

*Positive End-Expiratory Pressure (PEEP):* The pressure above atmosphere in the lungs that exists at the end of expiration.

*Peak flow rate (Qpeak):* The maximum value of flow during the inspiratory portion of the respiratory flow rate waveform.

*Respiratory flow / airflow rate, patient flow / airflow rate (Qr):* These synonymous terms may be understood to refer to the RPT device's estimate of respiratory airflow rate, as opposed to "true respiratory flow rate" or "true respiratory airflow rate", which is the actual respiratory flow rate experienced by the patient, usually expressed in litres per minute.

*Tidal volume (Vt):* The volume of air inhaled or exhaled during normal breathing, when extra effort is not applied.

*Inhalation Time (Ti):* The duration of the inspiratory portion of the respiratory flow rate waveform.

*Exhalation Time (Te):* The duration of the expiratory portion of the respiratory flow rate waveform.

*(total) Time, or breath duration (Ttot):* The total duration between the start of the inspiratory portion of one respiratory flow rate waveform and the start of the inspiratory portion of the following respiratory flow rate waveform.

*Upper airway obstruction (UAO):* includes both partial and total upper airway obstruction. This may be associated with a state of flow limitation, in which the flow rate increases only slightly or may even decrease as the pressure difference across the upper airway increases (Starling resistor behaviour).

*Ventilation (Vent):* A measure of the total amount of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.5.3 RPT device parameters

*Flow rate:* The instantaneous volume (or mass) of air delivered per unit time. While flow rate and ventilation have the same dimensions of volume or mass per unit time, flow rate is measured over a much shorter period of time. Flow may be nominally positive for the inspiratory portion of a breathing cycle of a patient, and hence negative for the expiratory portion of the breathing cycle of a patient. In some cases, a reference to flow rate will be a reference to a scalar quantity, namely a quantity having magnitude only. In other cases, a reference to flow rate will be a reference to a vector quantity, namely a quantity having both magnitude and direction. Flow rate will be given the symbol Q. 'Flow rate' is sometimes shortened to simply 'flow'. Total flow rate, $Qt$, is the flow of air leaving the RPT device. Vent flow rate, $Qv$, is the flow of air leaving a vent to allow washout of exhaled gases. Leak flow rate, $Ql$, is the flow rate of unintentional leak from a patient interface system. Respiratory flow rate, $Qr$, is the flow of air that is received into the patient's respiratory system.

*Leak:* The word leak will be taken to be an unintended flow of air. In one example, leak may occur as the result of an incomplete seal between a mask and a patient's face. In another example leak may occur in a swivel elbow to the ambient.

*Pressure:* Force per unit area. Pressure may be measured in a range of units, including $cmH_2O$, $g\text{-}f/cm^2$, hectopascal. 1 $cmH_2O$ is equal to 1 $g\text{-}f/cm^2$ and is approximately 0.98 hectopascal. In this specification, unless otherwise stated, pressure is given in units of $cmH_2O$. The pressure in the patient interface (mask pressure) is given the symbol $Pm$, while the treatment pressure, which represents a target value to be achieved by the mask pressure $Pm$ at the current instant of time, is given the symbol $Pt$.

### 5.5.4 Terms for ventilators

*Adaptive Servo-Ventilator (ASV):* A servo-ventilator that has a changeable rather than a fixed target ventilation. The changeable target ventilation may be learned from some characteristic of the patient, for example, a respiratory characteristic of the patient.

*Backup rate:* A parameter of a ventilator that establishes the respiratory rate (typically in number of breaths per minute) that the ventilator will deliver to the patient, if not triggered by spontaneous respiratory effort.

*Cycled:* The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP):* a base pressure, to which a pressure varying within the breath is added to produce the desired mask pressure which the ventilator will attempt to achieve at a given time.

*End expiratory pressure (EEP):* Desired mask pressure which the ventilator will attempt to achieve at the end of the expiratory portion of the breath. If the pressure waveform template $\Pi(\Phi)$ is zero-valued at the end of expiration, *i.e.,* $\Pi(\Phi) = 0$ when $\Phi = 1$, the EEP is equal to the EPAP.

*IPAP:* desired mask pressure which the ventilator will attempt to achieve during the inspiratory portion of the breath.

*Pressure support:* A number that is indicative of the increase in pressure during ventilator inspiration over that during ventilator expiration, and generally means the difference in pressure between the maximum value during inspiration and the base pressure *(e.g., PS = IPAP - EPAP).* In some contexts pressure support means the difference which the ventilator aims to achieve, rather than what it actually achieves.

*Servo-ventilator:* A ventilator that measures patient ventilation, has a target ventilation, and which adjusts the level of pressure support to bring the patient ventilation towards the target ventilation.

*Servo-assistance:* Pressure support minus minimum pressure support.

*Spontaneous / Timed (S / T):* A mode of a ventilator or other device that attempts to detect the initiation of a breath of a spontaneously breathing patient. If however, the device is unable to detect a breath within a predetermined period of time, the device will automatically initiate delivery of the breath.

*Swing:* Equivalent term to pressure support.

*Triggered:* When a ventilator delivers a breath of air to a spontaneously breathing patient, it is said to be triggered to do so at the initiation of the inspiratory portion of the breathing cycle by the patient's efforts.

*Typical recent ventilation:* The typical recent ventilation $Vtyp$ is the value around which recent measures of ventilation over some predetermined timescale tend to cluster, that is, a measure of the central tendency of the measures of ventilation over recent history.

*Ventilator:* A mechanical device that provides pressure support to a patient to perform some or all of the work of breathing.

### 5.5.5 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

## 5.6 OTHER REMARKS

A portion of the disclosure of this patent document contains material which is subject to copyright protection. The copyright owner has no objection to the facsimile reproduction by anyone of the patent document or the patent disclosure, as it appears in the Patent and Trademark Office patent file or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being preferably used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

Moreover, in interpreting the disclosure, all terms should be interpreted in the broadest reasonable manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular embodiments, it is to be understood that these embodiments are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative embodiments and that other arrangements may be devised without departing from the technology. For example, although any aspects of the aforementioned methodologies are generally described for implementation with a PPG sensor and a respiratory signal derived with such a sensor, other sensing signals or sensors may be implemented. Thus, other sensors, such as any described in this specification, may be implemented for generation of such a respiratory signal (e.g.., respiratory effort signal), such as a flow rate sensor, pressure sensor, non-contact motion sensor (e.g., radio frequency motion sensor (e.g., radar) or sonar sensor), chest band sensor etc. for generating any of the aforementioned indicators and/or may be used with any aspects of the aforementioned classification/evaluation techniques.

REFERENCES

[1] A. v. Benjafield et al., "Estimation of the global prevalence and burden of obstructive sleep apnoea: a literature-based analysis," The Lancet Respiratory Medicine, vol. 7, no. 8, pp. 687-698, Aug. 2019, doi: 10.1016/52213-2600(19)30198-5.

[2] N. M. Punjabi, "The epidemiology of adult obstructive sleep apnea," Proceedings of the American Thoracic Society, vol. 5, no. 2. pp. 136-143, Feb. 2008. doi: 10.1513/pats.200709-155MG.

[3] F.-C. Yen, K. Behbehani, S. Member, E. A. Lucas, J. R. Burk, and J. R. Axe, "A Nonnvasive Technique for Detecting Obstructive and Central Sleep Apnea," IEEE transactions on biomedical engineering, vol. 44, no. 12, pp. 1262-1268, 1997, doi: 10.1109/10.649998.

[4] J. A. Dempsey, S. C. Veasey, B. J. Morgan, and C. P. O'donnell, "Pathophysiology of Sleep Apnea," Physiological reviews, vol. 90, no. 1, pp. 47-112, 2010, doi: 10.1152/physrev.00043.2008.

[5] S. M. Caples, A. S. Gami, and V. K. Somers, "Obstructive Sleep Apnea," Annals of internal medicine, vol. 142, no. 3, pp. 187-197, 2005, doi: 10.7326/0003-4819-142-3-200502010-00010.

[6] R. G. Smallwood, M. v Vitiello, E. C. Giblin, and P. N. Prinz, "Sleep Apnea: Relationship to Age, Sex, and Alzheimer's Dementia," Sleep, vol. 6, no. 1, pp. 16-22, 1983, doi: 10.1093/sleep/6.1.16.

[7] D. A. Pevernagie et al., "On the rise and fall of the apnea-hypopnea index: A historical review and critical appraisal," Journal of Sleep Research, vol. 29, no. 4. Blackwell Publishing Ltd, Aug. 01, 2020. doi: 10.1111/jsr.13066.

[8] D. J. Eckert, A. S. Jordan, P. Merchia, and A. Malhotra, "Central sleep apnea: Pathophysiology and treatment," Chest, vol. 131, no. 2, pp. 595-607, 2007, doi: 10.1378/chest.06.2287.

[9] S. Javaheri and J. A. Dempsey, "Central sleep apnea," Comprehensive Physiology, vol. 3, no. 1, pp. 141-163, 2013, doi: 10.1002/cphy.c110057.

[10] C. Sullivan, F. Issa, M. Berthon-Jones, and L. Eves, "Reversal of obstructive sleep apnoea by continuous positive airway pressure applied through the nares.," Lancet, vol. 1, no. 8225, pp. 862-862, 1981, doi: 10.1016/s0140-6736(81)92140-1.

[11] W. J. Randerath et al., "Non-CPAP therapies in obstructive sleep apnoea," European Respiratory Journal, vol. 37, no. 5, p. 1000, May 2011, doi: 10.1183/09031936.00099710.

[12] L. Rosenthal et al., "CPAP therapy in patients with mild OSA: implementation and treatment outcome," Sleep medicine, vol. 1, no. 3, pp. 215-220, 2000, doi: 10.1016/s1389-9457(00)00012-5.

[13] R. N. Aurora et al., "The treatment of central sleep apnea syndromes in adults: Practice parameters with an evidence-based literature review and meta-analyses," Sleep, vol. 35, no. 1. pp. 17-40, 2012. doi: 10.5665/sleep.1580.

[14] K. E. Bloch, "Polysomnography: a systematic review," Technology and Health Care, vol. 5, no. 4, pp. 285-305, 1997, doi: 10.3233/THC-1997-5403.

[15] C. A. Kushida et al., "Practice Parameters for the Indications for PSG-AASM Practice Parameters SLEEP Practice Parameters for the Indications for Polysomnography and Related Procedures: An Update for 2005," Sleep, vol. 28, no. 4, pp. 499-521, 2005, doi: 10.1093/sleep/28.4.499.

[16] J. V. Rundo and R. Downey, "Polysomnography," in Handbook of Clinical Neurology, vol. 160, Elsevier B.V., 2019, pp. 381-392. doi: 10.1016/B978-0-444-64032-1.00025-4.

[17] I. M. Rosen et al., "Clinical use of a home sleep apnea test: An American academy of sleep medicine position statement," Journal of Clinical Sleep Medicine, vol. 13, no. 10, pp. 1205-1207, 2017, doi: 10.5664/jcsm.6774.

[18] American Academy of Sleep Medicine Task Force, "Sleep-related breathing disorders in adults: recommendations for syndrome definition and measurement techniques in clinical research. The Report of an American Academy of Sleep Medicine Task Force.," Sleep, vol. 22, no. 5, pp. 667-689, 1999, doi: 10.1093/sleep/22.5.667.

[19] O. C. Ioachimescu et al., "Performance of peripheral arterial tonometry-based testing for the diagnosis of obstructive sleep apnea in a large sleep clinic cohort," Journal of Clinical Sleep Medicine, vol. 6, no. 10, pp. 1663-1674, Oct. 2020, doi: 10.5664/jcsm.8620.

[20] F. Massie, D. M. de Almeida, P. Dreesen, I. Thijs, J. Vranken, and S. Klerkx, "An evaluation of the Night Owl home sleep apnea testing system," Journal of Clinical Sleep Medicine, vol. 14, no. 10, pp. 1791-1796, Oct. 2018, doi: 10.5664/jcsm.7398.

[21] G. Pillar et al., "Detecting central sleep apnea in adult patients using WatchPAT-a multicenter validation study," Sleep and Breathing, vol. 24, no. 1, pp. 387-398, Mar. 2020, doi: 10.1007/s11325-019-01904-5.

[22] R. Lazazzera et al., "Detection and Classification of Sleep Apnea and Hypopnea Using PPG and SpO2 Signals," IEEE Transactions on Biomedical Engineering, vol. 68, no. 5, pp. 1496-1506, May 2021, doi: 10.1109/TBME.2020.3028041.

[23] J. Allen, "Photoplethysmography and its application in clinical physiological measurement," Physiological Measurement, vol. 28, no. 3. Mar. 01, 2007. doi: 10.1088/0967-3334/28/3/R01.

[24] A. A. Alian and K. H. Shelley, "Photoplethysmography," Best Practice and Research: Clinical Anaesthesiology, vol. 28, no. 4. Bailliere Tindall Ltd, pp. 395-406, 2014. doi: 10.1016/j.bpa.2014.08.006.

[25] A. Malhotra et al., "Performance of an automated polysomnography scoring system versus computer-assisted manual scoring," Sleep, vol. 36, no. 4, pp. 573-582, Apr. 2013, doi: 10.5665/sleep.2548.

[26] R. B. Berry et al., "Rules for scoring respiratory events in sleep: Update of the 2007 AASM manual for the scoring of sleep and associated events," Journal of Clinical Sleep Medicine, vol. 8, no. 5, pp. 597-619, 2012, doi: 10.5664/jcsm.2172.

**Claims**

1. A processor-readable medium, having stored thereon processor-executable instructions which, when executed by one or more processors, cause the one or more processors to distinguish sleep disordered breathing events from a signal generated by a photoplethysmography (PPG) sensor, the processor-executable instructions comprising:

   instructions to receive data representing the signal from the PPG sensor;
   instructions to derive a PPG-based respiratory signal from the data representing the signal from the PPG sensor;
   instructions to compute first level features from the PPG-based respiratory signal comprising instructions to compute values from a plurality of windows of the PPG-based respiratory signal;

instructions to compute second level features from the first level features, wherein the instructions to compute the second level features from the first level features comprise:

instructions to derive a peripheral arterial tonometry (PAT) signal from the data representing the signal from the PPG sensor; and

instructions to detect one or more respiratory events based on the PAT signal,

instructions to select a subset of values for each of the first level features wherein the subset of values are associated with a subset of windows, of the plurality of windows, that correspond with occurrence of a detected respiratory event of the one or more respiratory events; and

instructions to compute, from the selected subset of values, one or more values of the second level features;

instructions to evaluate the first level features and the second level features; and

instructions to generate, based on the evaluation, an output indication that identifies a sleep disordered breathing type.

2. The processor-readable medium of claim 1, wherein the output indication that identifies the sleep disordered breathing type characterizes a plurality of events of a sleep session.

3. The processor-readable medium of any one of claims 1 to 2, wherein the output indication that identifies a sleep disordered breathing type characterizes the one or more respiratory events.

4. The processor-readable medium of any one of claims 1 to 3, wherein:

(a) the signal from the PPG sensor is a signal from a finger sensor that is the PPG sensor;

(b) the output indication identifies a central sleep apnea type;

(c) the output indication identifies a central sleep apnea type wherein the central sleep apnea type comprises a mixed central and obstructive sleep apnea type; and/or

(d) the output indication identifies an obstructive sleep apnea type.

5. The processor-readable medium of claim 1, wherein the computed values comprise, for each of the plurality of windows, any one or more of a range, an interquartile range, a variance, and a number of local maxima.

6. The processor-readable medium of claim 5, wherein:

(a) the plurality of windows comprises windows of a first uniform size moving along the PPG-based respiratory signal; or

(b) the plurality of windows comprises windows of a first uniform size moving along the PPG-based respiratory signal, and, wherein the plurality of windows further comprises windows of a second uniform size moving along the PPG-based respiratory signal, the second uniform size being shorter than the first uniform size.

7. The processor-readable medium of claim 1, wherein the one or more computed values of the second level features comprise any one or more of: a minimum value, an average of lowest three values, and one or more percentile values.

8. The processor-readable medium of any one of claims 1 to 7, wherein:

(a) the instructions to derive a PPG-based respiratory signal from the data representing the signal from the PPG sensor comprises:

instructions to filter baseline modulations of the data representing the signal from the PPG sensor to generate a filtered signal, and

instructions to generate an envelope signal from peaks of the filtered signal;

(b) the instructions to derive a PPG-based respiratory signal from the data representing the signal from the PPG sensor comprises:

instructions to filter baseline modulations of the data representing the signal from the PPG sensor to generate a filtered signal, and

instructions to generate an envelope signal from peaks of the filtered signal,

wherein the instructions to derive a PPG-based respiratory signal from the data representing the signal from the PPG sensor further comprise instructions to normalize the envelope signal by subtracting a moving average window of the envelope signal and dividing the envelope signal by a moving interquartile range window of the envelope signal; and/or

(c) the evaluation is in a classifier or the evaluation is in a classifier that comprises an ensemble of trees classifier.

9. The processor-readable medium of any one of claims 1 to 8, wherein:

(a) the processor-executable instructions further comprise instructions to detect an event of vasoconstriction from the data representing the signal from the PPG sensor, and wherein the output indication is based on the detected event of vasoconstriction; or
(b) the processor-executable instructions further comprise instructions to detect an event of vasoconstriction from the data representing the signal from the PPG sensor, and wherein the output indication is based on the detected event of vasoconstriction, wherein the instructions to detect an event of vasoconstriction comprise instructions to:

filter the PPG signal data representing the signal from the PPG sensor to produce a filtered PPG signal; and process the filtered PPG signal to detect a trough attributable to a vasoconstriction.

10. The processor-readable medium of claim 9, wherein the processor-executable instructions further comprise instructions to omit, from the evaluation, one or more values of one or both of the first level features and second level features that are associated with the detected event of vasoconstriction.

11. The processor-readable medium of any one of claims 1 to 10, wherein:

(a) the processor-executable instructions further comprise instructions to detect an event of arrythmia from the data representing the signal from the PPG sensor, and wherein the output indication is based on the detected event of arrythmia; or
(b) the processor-executable instructions further comprise instructions to detect an event of arrythmia from the data representing the signal from the PPG sensor, and wherein the output indication is based on the detected event of arrythmia, wherein the instructions to detect an event of arrythmia comprise instructions to detect one or more irregular peak-to-peak interval zones in the PPG signal that are associated with arrythmia.

12. The processor-readable medium of claim 11, wherein:

(a) the processor-executable instructions further comprise instructions to omit, from the evaluation, data corresponding to at least one respiratory event, of the one or more respiratory events, that is associated with the detected one or more irregular peak-to-peak interval zones; or
(b) the processor-executable instructions further comprise instructions to omit, from the evaluation, data corresponding to at least one respiratory event, of the one or more respiratory events, that is associated with the detected one or more irregular peak-to-peak interval zones,
wherein the instructions to detect the one or more irregular peak-to-peak interval zones comprise instructions to:

compute an absolute value of a difference of two neighboring peak-to-peak intervals; and
compute an average value of absolute values of differences of neighboring peak-to-peak intervals of the PPG signal; and
compare the absolute value and the average value.

13. The processor-readable medium of any one of claims 1 to 12, wherein:

(a) the processor-executable instructions further comprise instructions to generate a signal for controlling operation of a respiratory therapy apparatus based on the output indication,;
(b) the processor-executable instructions further comprise instructions to generate a signal for controlling operation of a respiratory therapy apparatus based on the output indication, wherein the controlling operation comprises controlling a pressure or flow therapy of a blower of the respiratory therapy apparatus; and/or
(c) the instructions to receive the data representing the signal from the PPG sensor comprises instructions for receiving the data at a server.

14. A processing device comprising: one or more processors; and a processor-readable medium of any one of claims 1 to 13.

15. The processing device of claim 14, wherein:

(a) the processing device is a respiratory therapy apparatus; or
(b) the processing device is a respiratory therapy apparatus, and the processing device is configured to generate a pressure therapy or a flow therapy.

**Patentansprüche**

1. Prozessorlesbares Medium, das darauf gespeicherte prozessorausführbare Anweisungen aufweist, die, wenn sie von einem oder mehreren Prozessoren ausgeführt werden, den einen oder mehrere Prozessoren veranlassen, Schlaf-Atmungsstörungsereignisse von einem Signal zu unterscheiden, das von einem Photoplethysmographie-, (PPG)-Sensor generiert wird, wobei die prozessorausführbaren Anweisungen umfassen:

Anweisungen, um Daten zu empfangen, die das Signal vom PPG-Sensor darstellen;
Anweisungen, um ein PPG-basiertes Atemsignal aus den Daten abzuleiten, die das Signal vom PPG-Sensor darstellen;
Anweisungen, um Merkmale erster Stufe aus dem PPG-basierten Atemsignal zu berechnen, die Anweisungen umfassen, um Werte aus einer Vielzahl von Fenstern des PPG-basierten Atemsignals zu berechnen;
Anweisungen, um Merkmale zweiter Stufe aus den Merkmalen erster Stufe zu berechnen, wobei die Anweisungen, um Merkmale zweiter Stufe aus den Merkmalen erster Stufe zu berechnen, umfassen:

Anweisungen, um ein peripheres arterielles Tonometrie-Signal (PAT-Signal) aus den Daten abzuleiten, die das Signal vom PPG-Sensor darstellen; und
Anweisungen, um ein oder mehrere Atemereignisse basierend auf dem PAT-Signal zu erkennen,
Anweisungen, um eine Teilmenge von Werten für jedes der Merkmale erster Stufe auszuwählen, wobei die Teilmenge von Werten einer Teilmenge von Fenstern aus der Vielzahl von Fenstern zugeordnet ist, die dem Auftreten eines erkannten Atemereignisses des einen oder mehrerer Atemereignisse entsprechen; und
Anweisungen, um einen oder mehrere Werte der Merkmale zweiter Stufe aus der ausgewählten Teilmenge von Werten zu berechnen;

Anweisungen, um die Merkmale erster und zweiter Stufe zu bewerten; und
Anweisungen, um basierend auf der Bewertung eine Ausgabeangabe zu generieren, die einen Schlaf-Atmungsstörungstyp identifiziert.

2. Prozessorlesbares Medium nach Anspruch 1, wobei die Ausgabeangabe, die den Schlaf-Atmungsstörungstyp identifiziert, eine Vielzahl von Ereignissen einer Schlafsitzung charakterisiert.

3. Prozessorlesbares Medium nach einem der Ansprüche 1 bis 2, wobei die Ausgabeangabe, die einen Schlaf-Atmungsstörungstyp identifiziert, das eine oder mehrere Atemereignisse charakterisiert.

4. Prozessorlesbares Medium nach einem der Ansprüche 1 bis 3, wobei:

(a) das Signal vom PPG-Sensor ein Signal von einem Fingersensor ist, welcher der PPG-Sensor ist;
b) die Ausgabeangabe einen zentralen Schlafapnoe-Typ identifiziert;
(c) die Ausgabeangabe einen zentralen Schlafapnoe-Typ identifiziert, wobei der zentrale Schlafapnoe-Typ einen gemischten zentralen und obstruktiven Schlafapnoe-Typ umfasst; und/oder
(d) die Ausgabeangabe einen obstruktiven Schlafapnoe-Typ identifiziert.

5. Prozessorlesbares Medium nach Anspruch 1, wobei die berechneten Werte für jedes der Vielzahl von Fenstern jeweils einen oder mehrere von einem Bereich, einem Interquartilbereich, einer Varianz und einer Anzahl an lokalen Maxima umfassen.

6. Prozessorlesbare Medium nach Anspruch 5, wobei:

(a) die Vielzahl von Fenstern Fenster einer ersten einheitlichen Größe umfasst, die sich entlang des PPG-basierten Atemsignals bewegen; oder

b) die Vielzahl von Fenstern Fenster einer ersten einheitlichen Größe umfasst, die sich entlang des PPG-basierten Atemsignals bewegen, und wobei die Vielzahl von Fenstern weiter Fenster einer zweiten einheitlichen Größe umfasst, die sich entlang des PPG-basierten Atemsignals bewegen, wobei die zweite einheitliche Größe kleiner ist als die erste einheitliche Größe.

7. Prozessorlesbares Medium nach Anspruch 1, wobei der eine oder mehrere berechnete Werte der Merkmale zweiter Stufe einen oder mehr umfassen von: einem Minimalwert, einem Durchschnitt der drei niedrigsten Werte und einem oder mehreren Perzentilwerten.

8. Prozessorlesbares Medium nach einem der Ansprüche 1 bis 7, wobei:

(a) die Anweisungen, um ein PPG-basiertes Atemsignal aus den Daten abzuleiten, die das Signal vom PPG-Sensor darstellen, umfassen:

Anweisungen, um Basislinienmodulationen der Daten zu filtern, die das Signal des PPG-Sensors darstellen, um ein gefiltertes Signal zu generieren, und
Anweisungen, um ein Hüllkurvensignals aus Spitzen des gefilterten Signals zu generieren;

b) die Anweisungen, um ein PPG-basiertes Atemsignal aus den Daten abzuleiten, die das Signal vom PPG-Sensor darstellen, umfassen:

Anweisungen, um Basislinienmodulationen der Daten zu filtern, die das Signal des PPG-Sensors darstellen, um ein gefiltertes Signal zu generieren, und
Anweisungen, um ein Hüllkurvensignals aus Spitzen des gefilterten Signals zu generieren,
wobei die Anweisungen, um ein PPG-basiertes Atemsignal aus den Daten, die das Signal des PPG-Sensors darstellen, abzuleiten, weiter Anweisungen umfassen, um das Hüllkurvensignal durch Subtrahieren eines gleitenden Mittelwertfensters von dem Hüllkurvensignal und Dividieren des Hüllkurvensignals durch ein gleitendes Interquartilsbereichsfenster des Hüllkurvensignals zu normalisieren; und/oder

(c) die Bewertung in einem Klassifikator erfolgt oder die Bewertung in einem Klassifikator erfolgt, der einen Klassifikator von Baumanordnungen umfasst.

9. Prozessorlesbares Medium nach einem der Ansprüche 1 bis 8, wobei:

(a) die vom Prozessor ausführbaren Anweisungen weiter Anweisungen umfassen, um ein Vasokonstriktionsereignis aus den Daten zu erkennen, die das Signal von dem PPG-Sensor darstellen, und wobei die Ausgabeangabe auf dem erkannten Vasokonstriktionsereignis basiert; oder

(b) die vom Prozessor ausführbaren Anweisungen weiter Anweisungen umfassen, um ein Vasokonstriktionsereignis aus den Daten zu erkennen, die das Signal von dem PPG-Sensor darstellen, und wobei die Ausgabeangabe auf dem erkannten Vasokonstriktionsereignis basiert; wobei die Anweisungen, um ein Vasokonstriktionsereignis zu erkennen Anweisungen umfassen, um:

die PPG-Signaldaten, die das Signal von dem PPG-Sensor darstellen, zu filtern, um ein gefiltertes PPG-Signal zu erzeugen; und
das gefilterte PPG-Signal zu verarbeiten, um einen durch eine Vasokonstriktion bedingten Tiefpunkt zu erkennen.

10. Prozessorlesbares Medium nach Anspruch 9, wobei die vom Prozessor ausführbaren Anweisungen weiter Anweisungen umfassen, um einen oder mehrere Werte von einem oder beiden der Merkmale erster Stufe und der Merkmale zweiter Stufe, die mit dem erkannten Vasokonstriktionsereignis zugeordnet werden, aus der Bewertung wegzulassen.

11. Prozessorlesbares Medium nach einem der Ansprüche 1 bis 10, wobei:

(a) die vom Prozessor ausführbaren Anweisungen weiter Anweisungen umfassen, um ein Arrhythmieereignis aus den Daten zu erkennen, die das Signal von dem PPG-Sensor darstellen, und wobei die Ausgabeangabe auf dem erkannten Arrhythmieereignis basiert; oder

(b) die vom Prozessor ausführbaren Anweisungen weiter Anweisungen umfassen, um ein Arrhythmieereignis aus den Daten zu erkennen, die das Signal von dem PPG-Sensor darstellen, und wobei die Ausgabeangabe auf dem erkannten Arrhythmieereignis basiert, wobei die Anweisungen zum Erkennen eines Arrhythmieereignisses Anweisungen zum Erkennen einer oder mehrerer unregelmäßiger Peak-to-Peak-Intervallzonen im PPG-Signal umfassen, die Arrhythmie zugeordnet werden.

12. Prozessorlesbares Medium nach Anspruch 11, wobei:

(a) die vom Prozessor ausführbaren Anweisungen weiter Anweisungen umfassen, um Daten aus der Bewertung wegzulassen, die mindestens einem Atemereignis des einen oder mehrerer Atemereignisse entsprechen, das der erkannten einen oder mehreren unregelmäßigen Peak-to-Peak-Intervallzonen zugeordnet ist; oder
b) die vom Prozessor ausführbaren Anweisungen weiter Anweisungen umfassen, um Daten aus der Bewertung wegzulassen, die mindestens einem Atemereignis des einen oder mehrerer Atemereignisse entsprechen, das der erkannten einen oder mehreren unregelmäßigen Peak-to-Peak-Intervallzonen zugeordnet ist,
wobei die Anweisungen, um die eine oder mehrere unregelmäßige Peak-to-Peak-Intervallzonen Anweisungen zu erkennen, Anweisungen umfassen um:

den Absolutwert einer Differenz zweier benachbarter Peak-to-Peak-Intervalle zu berechnen; und
einen Mittelwert von Absolutwerten von Differenzen benachbarter Peak-to-Peak-Intervalle des PPG-Signals zu berechnen; und
den Absolutwert und den Mittelwert zu vergleichen.

13. Prozessorlesbares Medium nach einem der Ansprüche 1 bis 12, wobei:

(a) die vom Prozessor ausführbaren Anweisungen weiter Anweisungen umfassen, um ein Signal zur Steuerung eines Betriebs einer Atemtherapieeinrichtung basierend auf der Ausgabeangabe zu generieren;
b) die vom Prozessor ausführbaren Anweisungen weiter Anweisungen umfassen, um ein Signal zur Steuerung eines Betriebs einer Atemtherapieeinrichtung basierend auf der Ausgabeangabe zu generieren, wobei die Steuerungsoperation eine Steuerung einer Druck- oder Flusstherapie eines Gebläses der Atemtherapieeinrichtung umfasst; und/oder
(c) die Anweisungen, um die Daten zu empfangen, die das Signal vom PPG-Sensor darstellen, Anweisungen zum Empfangen der Daten auf einem Server umfassen.

14. Verarbeitungsvorrichtung, umfassend: einen oder mehrere Prozessoren; und ein prozessorlesbares Medium nach einem der Ansprüche 1 bis 13.

15. Verarbeitungsvorrichtung nach Anspruch 14, wobei:

(a) die Verarbeitungsvorrichtung eine Atemtherapieeinrichtung ist; oder
b) die Verarbeitungsvorrichtung eine Atemtherapieeinrichtung ist und die Verarbeitungsvorrichtung konfiguriert ist, um eine Drucktherapie oder eine Flusstherapie zu generieren.

**Revendications**

1. Support lisible par processeur, sur lequel sont stockées des instructions exécutables par processeur qui, lorsqu'elles sont exécutées par un ou plusieurs processeurs, amènent l'un ou les plusieurs processeurs à distinguer des événements respiratoires anormaux du sommeil d'un signal généré par un capteur de photopléthysmographie (PPG), les instructions exécutables par processeur comprenant :

des instructions pour recevoir des données représentant le signal provenant du capteur PPG ;
des instructions pour dériver un signal respiratoire basé sur le PPG à partir des données représentant le signal provenant du capteur PPG ;
des instructions pour calculer des caractéristiques de premier niveau à partir du signal respiratoire basé sur le PPG comprenant des instructions pour calculer des valeurs à partir d'une pluralité de fenêtres du signal respiratoire basé sur le PPG ;
des instructions pour calculer des caractéristiques de second niveau à partir des caractéristiques de premier niveau, dans lequel les instructions pour calculer les caractéristiques de second niveau à partir des caractéristiques de premier niveau comprennent :

des instructions pour dériver un signal de tonométrie artérielle périphérique (PAT) à partir des données représentant le signal provenant du capteur PPG ; et
des instructions pour détecter un ou plusieurs événements respiratoires sur la base du signal PAT,
des instructions pour sélectionner un sous-ensemble de valeurs pour chacune des caractéristiques de premier niveau, dans lequel le sous-ensemble de valeurs est associé à un sous-ensemble de fenêtres, parmi la pluralité de fenêtres, qui correspondent à l'occurrence d'un événement respiratoire détecté de l'un ou des plusieurs événements respiratoires ; et
des instructions pour calculer, à partir du sous-ensemble de valeurs sélectionné, une ou plusieurs valeurs des caractéristiques de second niveau ;

des instructions pour évaluer les fonctionnalités de premier niveau et les fonctionnalités de second niveau ; et
des instructions pour générer, sur la base de l'évaluation, une indication de sortie qui identifie un type respiratoire anormal du sommeil.

**2.** Support lisible par processeur selon la revendication 1, dans lequel l'indication de sortie qui identifie le type respiratoire anormal du sommeil caractérise une pluralité d'événements d'une session de sommeil.

**3.** Support lisible par processeur selon l'une quelconque des revendications 1 à 2, dans lequel l'indication de sortie qui identifie un type respiratoire anormal du sommeil caractérise l'un ou les plusieurs événements respiratoires.

**4.** Support lisible par processeur selon l'une quelconque des revendications 1 à 3, dans lequel :

(a) le signal provenant du capteur PPG est un signal provenant d'un capteur de doigt qui est le capteur PPG ;
(b) l'indication de sortie identifie un type d'apnée du sommeil centrale ;
(c) l'indication de sortie identifie un type d'apnée du sommeil centrale dans lequel le type d'apnée du sommeil centrale comprend un type d'apnée du sommeil centrale mixte et obstructive ; et/ou
(d) l'indication de sortie identifie un type d'apnée du sommeil obstructive.

**5.** Support lisible par processeur selon la revendication 1, dans lequel les valeurs calculées comprennent, pour chacune des pluralités de fenêtres, un ou plusieurs éléments parmi une plage, une plage interquartile, une variance et un nombre de maxima locaux.

**6.** Support lisible par processeur selon la revendication 5, dans lequel :

(a) la pluralité de fenêtres comprend des fenêtres d'une première taille uniforme se déplaçant le long du signal respiratoire basé sur le PPG ; ou
(b) la pluralité de fenêtres comprend des fenêtres d'une première taille uniforme se déplaçant le long du signal respiratoire basé sur le PPG, et dans lequel la pluralité de fenêtres comprend en outre des fenêtres d'une seconde taille uniforme se déplaçant le long du signal respiratoire basé sur le PPG, la seconde taille uniforme étant plus courte que la première taille uniforme.

**7.** Support lisible par processeur selon la revendication 1, dans lequel l'une ou les plusieurs valeurs calculées des caractéristiques de second niveau comprennent une ou plusieurs parmi : une valeur minimale, une moyenne des trois valeurs les plus basses et une ou plusieurs valeurs de percentile.

**8.** Support lisible par processeur selon l'une quelconque des revendications 1 à 7, dans lequel :

(a) les instructions pour dériver un signal respiratoire basé sur le PPG à partir des données représentant le signal provenant du capteur PPG comprennent :

des instructions pour filtrer des modulations de référence des données représentant le signal provenant du capteur PPG pour générer un signal filtré, et
des instructions pour générer un signal d'enveloppe à partir de pics du signal filtré ;

(b) les instructions pour dériver un signal respiratoire basé sur le PPG à partir des données représentant le signal provenant du capteur PPG comprennent :

des instructions pour filtrer des modulations de référence des données représentant le signal provenant du capteur PPG pour générer un signal filtré, et

des instructions pour générer un signal d'enveloppe à partir de pics du signal filtré,

dans lequel les instructions pour dériver un signal respiratoire basé sur le PPG à partir des données représentant le signal du capteur PPG comprennent en outre des instructions pour normaliser le signal d'enveloppe en soustrayant une fenêtre de moyenne mobile du signal d'enveloppe et en divisant le signal d'enveloppe par une fenêtre de plage interquartile mobile du signal d'enveloppe ; et/ou

(c) l'évaluation est dans un classificateur ou l'évaluation est dans un classificateur qui comprend un ensemble de classificateurs d'arbres.

9.   Support lisible par processeur selon l'une quelconque des revendications 1 à 8, dans lequel :

(a) les instructions exécutables par processeur comprennent en outre des instructions pour détecter un événement de vasoconstriction à partir des données représentant le signal provenant du capteur PPG, et dans lequel l'indication de sortie est basée sur l'événement de vasoconstriction détecté ; ou

(b) les instructions exécutables par processeur comprennent en outre des instructions pour détecter un événement de vasoconstriction à partir des données représentant le signal provenant du capteur PPG, et dans lequel l'indication de sortie est basée sur l'événement de vasoconstriction détecté, dans lequel les instructions pour détecter un événement de vasoconstriction comprennent des instructions pour :

filtrer les données de signal PPG représentant le signal provenant du capteur PPG pour produire un signal PPG filtré ; et

traiter le signal PPG filtré pour détecter un creux attribuable à une vasoconstriction.

10.   Support lisible par processeur selon la revendication 9, dans lequel les instructions exécutables par processeur comprennent en outre des instructions pour omettre de l'évaluation une ou plusieurs valeurs d'une ou des deux caractéristiques de premier niveau et des caractéristiques de second niveau qui sont associées à l'événement détecté de vasoconstriction.

11.   Support lisible par processeur selon l'une quelconque des revendications 1 à 10, dans lequel :

(a) les instructions exécutables par processeur comprennent en outre des instructions pour détecter un événement d'arythmie à partir des données représentant le signal provenant du capteur PPG, et dans lequel l'indication de sortie est basée sur l'événement d'arythmie détecté ; ou

(b) les instructions exécutables par processeur comprennent en outre des instructions pour détecter un événement d'arythmie à partir des données représentant le signal provenant du capteur PPG, et dans lequel l'indication de sortie est basée sur l'événement d'arythmie détecté, dans lequel les instructions pour détecter un événement d'arythmie comprennent des instructions pour détecter une ou plusieurs zones d'intervalle crête à crête irrégulières dans le signal PPG qui sont associées à l'arythmie.

12.   Support lisible par processeur selon la revendication 11, dans lequel :

(a) les instructions exécutables par processeur comprennent en outre des instructions pour omettre de l'évaluation des données correspondant à l'au moins un événement respiratoire, de l'un ou des plusieurs événements respiratoires, qui est associé à l'une ou aux plusieurs zones d'intervalle crête à crête irrégulières détectées ; ou

(b) les instructions exécutables par processeur comprennent en outre des instructions pour omettre de l'évaluation des données correspondant à l'au moins un événement respiratoire, de l'un ou des plusieurs événements respiratoires, qui est associé à l'une ou aux plusieurs zones d'intervalle crête à crête irrégulières détectées,

dans lequel les instructions pour détecter l'une ou les plusieurs zones d'intervalle crête à crête irrégulières comprennent des instructions pour :

calculer une valeur absolue d'une différence entre deux intervalles crête à crête voisins ; et

calculer une valeur moyenne de valeurs absolues de différences entre des intervalles crête à crête voisins du signal PPG ; et

comparer la valeur absolue et la valeur moyenne.

13. Support lisible par processeur selon l'une quelconque des revendications 1 à 12, dans lequel :

(a) les instructions exécutables par processeur comprennent en outre des instructions pour générer un signal pour commander le fonctionnement d'un appareil de thérapie respiratoire sur la base de l'indication de sortie ;
(b) les instructions exécutables par processeur comprennent en outre des instructions pour générer un signal pour commander le fonctionnement d'un appareil de thérapie respiratoire sur la base de l'indication de sortie, dans lequel le fonctionnement de commande comprend la commande d'une thérapie par pression ou par débit d'un ventilateur de l'appareil de thérapie respiratoire ; et/ou
(c) les instructions pour recevoir les données représentant le signal provenant du capteur PPG comprennent des instructions pour recevoir les données au niveau d'un serveur.

14. Dispositif de traitement comprenant : un ou plusieurs processeurs ; et un support lisible par processeur selon l'une quelconque des revendications 1 à 13.

15. Dispositif de traitement selon la revendication 14, dans lequel :

(a) le dispositif de traitement est un appareil de thérapie respiratoire ; ou
(b) le dispositif de traitement est un appareil de thérapie respiratoire, et le dispositif de traitement est configuré pour générer une thérapie par pression ou une thérapie par flux.

FIG. 1

# FIG. 2

Comparison of a typical central and obstructive respiratory event. Both events are accompanied by cessation of airflow, but a central event is characterized by lack of respiratory effort which is reflected in a flat region of the respiratory effort belt signal. PSG = polysomnography.

**FIG. 3**

Example segment of PPG signal with the envelope reflecting respiratory effort modulations. PPG = photoplethysmography.

**FIG. 4**

Comparison of respiratory effort signal extracted from the finger PPG with the respiratory effort channel of the PSG. Zones highlighted in pink indicate flat respiratory effort zones. PPG = photoplethysmography, PSG = polysomnography.

**Expert Annotations**

central event
obstructive event

**PAT HSAT Events Labeled By Expert Annotations**

central event
obstructive event
removed event

Time (seconds)

## FIG. 5

The procedure used to label PAT HSAT events. PAT HSAT event 1 is labeled as central because it has a uniquely overlapping central Expert annotation. PAT HSAT event 2 is labeled as obstructive because it has an Expert obstructive annotation preceding by not more than 10 seconds. PAT HSAT event 3 is removed because it cannot be labeled using the Expert annotations. This event will not be used for training. PAT HSAT event 4 has two overlapping annotations, but since at least one of them is central, it is labeled as central. PAT HSAT event 5 is labeled as obstructive because it has a single overlapping obstructive annotation. PAT HSAT = home sleep apnea testing based on peripheral arterial tonometry.

Raw PPG signal

PPGDR

Time (seconds)

## FIG. 6

PPG signal and PPGDR of two vasoconstriction-affected zones (visualized by grey areas). The PPGDR extracted from these zones is of lower quality, apparent from the comparatively more erratic swings in the PPGDR signal, and is therefore rejected. PPG = photoplethysmography, PPGDR = PPG-derived respiratory effort.

**PPG signal**

**Respiratory effort signal extracted from the PPG**

**Respiratory effort channel of the PSG**

Time (seconds)

# FIG. 7

Example of an artifacted PPG signal (top), PPGDR extracted from the PPG signal (middle), and the corresponding respiratory effort channel of the PSG (bottom). The PPGDR extracted from this artifacted zone does not reflect the true respiratory modulations. PPG = photoplethysmography, PPGDR = PPG-derived respiratory effort, PSG = polysomnography.

**FIG. 8**

ROC curves for cAHI cutoffs of 5, 10, and 15 of the PPG-based method. cAHI = central apnea-hypopnea index, AUC = area under the curve, ROC =receiver operating characteristic.

**FIG. 9**

(Left) Scatterplot of Expert Analysis cAHI vs PPG-based method's predicted cAHI. (Right) Scatterplot of Expert Analysis cAHI vs Local Analysis cAHI. cAHI = central apnea-hypopnea index, P: P-value, PPG = photoplethysmography, r: Pearson's correlation coefficient, $R^2$: R-squared, $y = a + b*x$: the equation of the least-squares fitted line.

EP 4 472 498 B1

## FIG. 10

PPV, NPV, and the sum of their values for the PPG-based method for cAHI cutoffs in the range of 0 to 15. cAHI = central apnea-hypopnea index, PPV = Positive Predictive Value, NPV = Negative Predictive Value.

EP 4 472 498 B1

TABLE I
DEMOGRAPHIC AND CLINICAL CHARACTERISTICS OF PATIENTS IN DATASET

|  | Mean | STD | Min | Max |
|---|---|---|---|---|
| Age (years) | 53.9 | 13.7 | 21.0 | 84.0 |
| AHI (events/hr) | 31.1 | 24.8 | 0.0 | 117.3 |
| cAHI (event/hr) | 3.2 | 7.5 | 0.0 | 45.5 |
| BMI (kg m⁻²) | 29.9 | 5.9 | 18.2 | 53.8 |

AHI = apnea-hypopnea index, cAHI = central apnea-hypopnnea index, BMI = body mass index, Max = maximum, Min = minimum, STD = standard deviation.

## FIG. 11

TABLE II
PERFORMANCE ENDPOINTS

| AHIc cutoff | | cAHI ≥ 5 | cAHI ≥ 10 | cAHI ≥ 15 |
|---|---|---|---|---|
| Sensitivity (%) | PPG – EXPERT | 78.1 [63.8, 92.4] | 81.0 [64.2, 97.8] | 72.7 [46.4, 99.0] |
| | LOCAL – EXPERT | 71.9 [56.3, 87.5] | 57.1 [35.9, 78.3] | 72.7 [46.4, 99.0] |
| Specificity (%) | PPG – EXPERT | 95.3 [92.5, 98.1] | 99.1 [97.9, 100.0] | 97.8 [95.9, 99.7] |
| | LOCAL – EXPERT | 95.3 [92.5, 98.1] | 99.1 [97.9, 100.0] | 98.3 [96.6, 100] |
| PPV (%) | PPG – EXPERT | 71.4 [56.4, 86.4] | 89.5 [75.7, 100.0] | 61.5 [35.0, 88.0] |
| | LOCAL – EXPERT | 69.7 [54.0, 85.4] | 85.7 [67.4, 100.0] | 66.7 [40.0, 93.4] |
| NPV (%) | PPG – EXPERT | 96.7 [94.3, 99.1] | 98.2 [96.5, 99.9] | 98.7 [97.2, 100.0] |
| | LOCAL – EXPERT | 95.8 [93.1, 98.5] | 96.1 [93.6, 98.6] | 98.7 [97.2, 100.0] |
| Accuracy (%) | PPG – EXPERT | 93.1 [89.9, 96.3] | 97.6 [95.7, 99.5] | 96.7 [94.5, 98.9] |
| | LOCAL – EXPERT | 92.2 [88.8, 95.6] | 95.5 [92.9, 98.1] | 97.1 [95.0, 99.2] |
| Cohen's Kappa | PPG – EXPERT | 0.71 [0.57, 0.84] | 0.84 [0.71, 0.97] | 0.65 [0.41, 0.89] |
| | LOCAL – EXPERT | 0.66 [0.52, 0.81] | 0.66 [0.47, 0.86] | 0.68 [0.45, 0.91] |
| ROC AUC | PPG – EXPERT | 0.94 | 0.98 | 0.98 |
| | LOCAL – EXPERT | NA | NA | NA |

95% confidence interval estimates for the performance endpoints are shown in brackets. PPG – EXPERT refers to the PPG-based method's predictions compared to Expert Analysis. LOCAL – EXPERT refers to the Local Analysis compared to the Expert Analysis. cAHI = central apnea-hypopnea index. AUC = area under curve, NPV = negative predictive value, PPV = positive predictive value, ROC = receiver operating characteristic, PPG = photoplethysmography.

FIG. 12

TABLE III
CONFUSION MATRIX

**Predicted Class**

| | | cAHI < 5 | | 5 ≤ cAHI < 10 | | 10 ≤ cAHI < 15 | | cAHI ≥ 15 | |
|---|---|---|---|---|---|---|---|---|---|
| | | PPG – EXPERT | LOCAL – EXPERT | PPG – EXPERT | LOCAL – EXPERT | PPG – EXPERT | LOCAL – EXPERT | PPG – EXPERT | LOCAL – EXPERT |
| True Class | cAHI < 5 | 203 | 203 | 9 | 8 | 1 | 1 | 0 | 1 |
| | 5 ≤ cAHI < 10 | 5 | 5 | 5 | 6 | 0 | 0 | 1 | 0 |
| | 10 ≤ cAHI < 15 | 2 | 3 | 1 | 3 | 3 | 1 | 4 | 3 |
| | cAHI ≥ 15 | 0 | 1 | 1 | 2 | 2 | 0 | 8 | 8 |

PPG – EXPERT refers to the PPG-based method's predictions compared to Expert Analysis. LOCAL – EXPERT refers to the Local Analysis compared to the Expert Analysis. cAHI = central apnea-hypopnea index, PPG = photoplethysmography.

## FIG. 13

EP 4 472 498 B1

13000

13010 — receiving data representing the signal
from the PPG sensor

13040 — deriving a PPG-based respiratory signal from
the data representing the signal from the
PPG sensor

13050 — computing first level features from
the PPG-based respiratory signal

13060 — computing second level features
from the first level features

13070 — evaluating the first level features and/or
the second level features such as in a
classifier

13080 — generating, based on the evaluation such as of the
classifier, an output indication that identifies a sleep
disordered breathing type

13090 — generating an output with, or based on, the
generated indicator and/or evaluation

FIG. 14A

14000

14010 — receiving data representing the signal from the PPG sensor

14020 — deriving a peripheral arterial tonometry (PAT) signal from the data representing the signal from the PPG sensor

14030 — detecting one or more respiratory events based on the PAT signal

14040 — deriving a PPG-based respiratory signal (e.g., respiratory effort signal) from the data representing the signal from the PPG sensor

14050 — computing first level features from the PPG-based respiratory signal

14060 — computing second level features from the first level features

14070 — evaluating the first level features and the second level features such as in a classifier

14080 — generating, based on the evaluation such as of the classifier, an output indication that identifies a sleep disordered breathing type such as for each of the one or more detected respiratory events

14090 — generating an output with, or based on, the generated indicator and/or evaluation

**FIG. 14B**

15000 —

15010 —

| receiving data representing the<br>signal from the PPG sensor |

15025 —

| detecting an event of vasoconstriction<br>and/or an event of arrythmia from the<br>data representing the signal from the<br>PPG sensor |

15070 —

| evaluating the data representing the<br>signal from the PPG sensor to generate,<br>based on the evaluation and the detected<br>event of vasoconstriction and/or the<br>detected event of arrythmia, an output<br>indication that identifies a sleep<br>disordered breathing event |

15090 —

| generating an output with, or based<br>on, the generated indicator and/or<br>evaluation |

**FIG. 14C**

FIG. 15A

FIG. 15B

FIG. 15C

EP 4 472 498 B1

FIG. 16

4015

4000

4018

4112

4015

4220

Superior

Posterior

4012

Anterior

Inferior

4202

4142

4100, 4020

4200

4010

4016

4210

4014

FIG. 17A

4110 — 4112 Air inlet filter ← Supplementary O₂

Pneumatic block

4120 — 4122 Inlet muffler

4270 — Transducer(s)

4180

4140 — | 4142 Blower | 4144 Motor |

4020

4120 — 4124 Outlet muffler

Upstream

4270 — Transducer(s)

↕

Downstream

4160 — Anti-spillback valve

5000 — Humidifier

4110 — 4114 Air outlet filter

4180

4170 — Air circuit / delivery tube ← Supplementary O₂

4180

4270 — Transducer(s)

4180

**FIG. 17B**

3000 — Patient Interface ← Supplementary O₂

FIG. 17C

FIG. 17D

4500

4520

Y Apnea / hypopnea index > threshold for a time?

N

4530 4560

Obstruction Index > threshold ? N Decrease treatment pressure

Y

4540 4550

Airway patent? N Increase treatment pressure

Y

FIG. 17E

5000

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63306413
- US 2021275090 A1
- US 2020015737 A1
- WO 2021260192 A1
- EP 2021067532 W
- AU 2010000894 W
- WO 2011006199 A

### Non-patent literature cited in the description

- **A. V. BENJAFIELD et al.** Estimation of the global prevalence and burden of obstructive sleep apnoea: a literature-based analysis. *he Lancet Respiratory Medicine*, August 2019, vol. 7 (8), 687-698
- **N. M. PUNJABI**. The epidemiology of adult obstructive sleep apnea. *Proceedings of the American Thoracic Society*, February 2008, vol. 5 (2), 136-143
- **F.-C. YEN** ; **K. BEHBEHANI** ; **S. MEMBER** ; **E. A. LUCAS** ; **J. R. BURK** ; **J. R. AXE**. A Nonnvasive Technique for Detecting Obstructive and Central Sleep Apnea. *IEEE transactions on biomedical engineering*, 1997, vol. 44 (12), 1262-1268
- **J. A. DEMPSEY** ; **S. C. VEASEY** ; **B. J. MORGAN** ; **C. P. O'DONNELL**. Pathophysiology of Sleep Apnea. *Physiological reviews*, 2010, vol. 90 (1), 47-112
- **S. M. CAPLES** ; **A. S. GAMI** ; **V. K. SOMERS**. Obstructive Sleep Apnea. *Annals of internal medicine*, 2005, vol. 142 (3), 187-197
- **R. G. SMALLWOOD** ; **M. V VITIELLO** ; **E. C. GIBLIN** ; **P. N. PRINZ**. Sleep Apnea: Relationship to Age, Sex, and Alzheimer's Dementia. *Sleep,*, 1983, vol. 6 (1), 16-22
- On the rise and fall of the apnea-hypopnea index: A historical review and critical appraisal. **D. A. PEVERNAGIE et al.** Journal of Sleep Research. Blackwell Publishing Ltd, 01 August 2020, vol. 29
- **D. J. ECKERT** ; **A. S. JORDAN** ; **P. MERCHIA** ; **A. MALHOTRA**. Central sleep apnea: Pathophysiology and treatment. *Chest*, 2007, vol. 131 (2), 595-607
- **S. JAVAHERI** ; **J. A. DEMPSEY**. Central sleep apnea. *Comprehensive Physiology*, 2013, vol. 3 (1), 141-163
- **C. SULLIVAN** ; **F. ISSA** ; **M. BERTHON-JONES** ; **L. EVES**. Reversal of obstructive sleep apnoea by continuous positive airway pressure applied through the nares.. *Lancet*, 1981, vol. 1 (8225), 862-862
- **W. J. RANDERATH et al.** Non-CPAP therapies in obstructive sleep apnoea. *European Respiratory Journal*, May 2011, vol. 37 (5), 1000
- **L. ROSENTHAL et al.** CPAP therapy in patients with mild OSA: implementation and treatment outcome. *Sleep medicine*, 2000, vol. 1 (3), 215-220
- **R. N. AURORA et al.** The treatment of central sleep apnea syndromes in adults: Practice parameters with an evidence-based literature review and meta-analyses. *Sleep*, 2012, vol. 35 (1), 17-40
- **K. E. BLOCH**. Polysomnography: a systematic review. *Technology and Health Care*, 1997, vol. 5 (4), 285-305
- **C. A. KUSHIDA et al.** Practice Parameters for the Indications for PSG-AASM Practice Parameters SLEEP Practice Parameters for the Indications for Polysomnography and Related Procedures: An Update for 2005. *Sleep*, 2005, vol. 28 (4), 499-521
- Polysomnography. **J. V. RUNDO** ; **R. DOWNEY**. Handbook of Clinical Neurology. Elsevier B.V., 2019, vol. 160, 381-392
- **I. M. ROSEN et al.** Clinical use of a home sleep apnea test: An American academy of sleep medicine position statement. *Journal of Clinical Sleep Medicine*, 2017, vol. 13 (10), 1205-1207
- **AMERICAN ACADEMY OF SLEEP MEDICINE TASK FORCE**. Sleep-related breathing disorders in adults: recommendations for syndrome definition and measurement techniques in clinical research. The Report of an American Academy of Sleep Medicine Task Force.. *Sleep*, 1999, vol. 22 (5), 667-689
- **O. C. IOACHIMESCU et al.** Performance of peripheral arterial tonometry-based testing for the diagnosis of obstructive sleep apnea in a large sleep clinic cohort. *Journal of Clinical Sleep Medicine*, October 2020, vol. 6 (10), 1663-1674

- **F. MASSIE** ; **D. M. DE ALMEIDA** ; **P. DREESEN** ; **I. THIJS** ; **J. VRANKEN** ; **S. KLERKX**. An evaluation of the Night Owl home sleep apnea testing system. *Journal of Clinical Sleep Medicine*, October 2018, vol. 14 (10), 1791-1796
- **G. PILLAR et al.** Detecting central sleep apnea in adult patients using WatchPAT-a multicenter validation study. *Sleep and Breathing,*, March 2020, vol. 24 (1), 387-398
- **R. LAZAZZERA et al.** Detection and Classification of Sleep Apnea and Hypopnea Using PPG and SpO2 Signals. *IEEE Transactions on Biomedical Engineering*, May 2021, vol. 68 (5), 1496-1506
- **J. ALLEN**. Photoplethysmography and its application in clinical physiological measurement. *Physiological Measurement*, 01 March 2007, vol. 28 (3)
- Photoplethysmography. **A. A. ALIAN** ; **K. H. SHELLEY**. Best Practice and Research: Clinical Anaesthesiology. Bailliere Tindall Ltd, 2014, vol. 28, 395-406
- **A. MALHOTRA et al.** Performance of an automated polysomnography scoring system versus computer-assisted manual scoring. *Sleep*, April 2013, vol. 36 (4), 573-582
- **R. B. BERRY et al.** Rules for scoring respiratory events in sleep: Update of the 2007 AASM manual for the scoring of sleep and associated events. *Journal of Clinical Sleep Medicine*, 2012, vol. 8 (5), 597-619